# EUROPEAN PATENT APPLICATION

(11) **EP 4 361 155 A1**
(43) Date of publication of application: **01.05.2024**
(21) Application number: 22827441.1
(22) Date of filing: 15.06.2022
(51) Int. Cl.: C07D 491/052, C07D 213/80, C07D 213/803, A61K 31/436, A61K 31/44, A61P 29/00, A61P 27/02, G01N 30/02

(54) **METHOD FOR PREPARING PRANOPROFEN AND COMPOSITION CONTAINING PRANOPROFEN AND IMPURITY**

(30) Priority: 25.06.2021 CN 202110710363; 25.06.2021 CN 202110710374; 25.06.2021 CN 202110711219; 25.06.2021 CN 202110715556; 25.06.2021 CN 202110715909; 25.06.2021 CN 202110716136
(71) Applicant: Shenyang Xingqi Pharmaceutical Co., Ltd., Shenyang, Liaoning 110167 (CN)
(72) Inventor: LIU, Jidong, Shenyang, Liaoning 110163 (CN); YANG, Qiang, Shenyang, Liaoning 110163 (CN); LI, Wenzhao, Shenyang, Liaoning 110163 (CN); SUN, Yang, Shenyang, Liaoning 110163 (CN); JI, Xueyu, Shenyang, Liaoning 110163 (CN)
(74) Representative: Casalonga
(86) International application number: PCT/CN2022/098881
(87) International publication number: WO 2022/267945

(57) **Abstract**

The present invention belongs to the field of medicine and relates to a method for preparing pranoprofen. The present invention also relates to a composition containing pranoprofen and impurities, a quality control method, an intermediate compound for synthesizing pranoprofen and a preparation method thereof. The method of the present invention overcomes the deficiencies of the prior art, shortens the synthetic route, lowers the requirements of process conditions, simplifies the process operation, improves labor safety, is more suitable for industrial production, is more environmentally friendly and has higher yield.

## Description

### Technical Field

The present invention belongs to the field of medicine, and relates to a method for preparing pranoprofen. The present invention also relates to a composition containing pranoprofen and impurities, a quality control method, an intermediate compound for synthesizing pranoprofen and a preparation method thereof. The intermediate compound is selected from the group consisting of a compound represented by Formula I or pharmaceutically acceptable salt or ester thereof, a compound represented by Formula II or pharmaceutically acceptable salt or ester thereof, a compound represented by Formula III or pharmaceutically acceptable salt or ester thereof, and a compound represented by Formula IV or pharmaceutically acceptable salt or ester thereof.

### Background Art

Pranoprofen is a propionic acid-type non-steroidal anti-inflammatory analgesic drug developed and listed by Yoshitomiyakuhin Corporation in 1981; and later, it was developed by Senju Pharmaceutical Co., Ltd. to form eye drops and listed with trade name Pranopulin in Japan in 1988, which is used for symptomatic treatment of outer eye and anterior eye inflammation (blepharitis, conjunctivitis, keratitis, scleritis, superficial scleritis, iridocyclitis, postoperative inflammation). Pranoprofen belongs to propionic acid-type non-steroidal anti-inflammatory drugs, and has chemical name: (2*RS*)-2-(10-hydrogen-9-oxa-1-aza-anthracen-6-yl)propionic acid, and CAS registration number: 52549-17-4. Its effect is stronger than that of aspirin, indomethacin, and ibuprofen. Its action mechanism is to inhibit the activity of cyclooxygenase, block the synthesis of eicosatetraene acid derivatives, and reduce the synthesis of prostaglandins, thereby blocking the function of inflammatory mediators to exert medicinal effect.

The structural Formula of pranoprofen is represented by Formula 0 below:

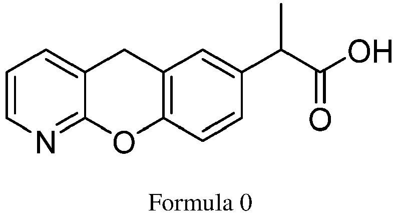

There are many reports about the synthesis of pranoprofen, and the main difference thereof lies in that different methods are used to establish its side chain, which are specifically listed as follows:
Route 1: It is based on the idea of rearrangement. There is a document (Bull. Chem. Soc. Jpn. 1987, 60, 4015-4018) that reports a method for preparing 2-aryl propionic acid by rearrangement of 2-hydroxy-propylketal and sulfonyl chloride. By referring to Japanese patents (JP4288080, JP4288081), Jin Qingrong et al. reported in "Synthetic Research Improvement of Pranoprofen" (Fine Chemical Intermediates, 2009, 39 (3), 37-39) that 2-chloronicotinic acid and phenol were used as raw materials, underwent nucleophilic substitution and PPA cyclization reaction to obtain SH-[1]-benzopyran[2,3-b]pyridin-5-one, and then underwent potassium borohydride reduction and acidic hydrolysis to obtain SH-[1]-benzopyran[2,3-b]pyridine, and then underwent acylation reaction with 2-chloropropionyl chloride to obtain the key intermediate 6-(2-chloropropionyl)-10H-9-oxa-1-azanthene. It reacted with sodium methoxide to get hydroxy ketal, then underwent sulfonylation of hydroxy using sulfonyl chloride, rearrangement and hydrolysis to get pranoprofen.

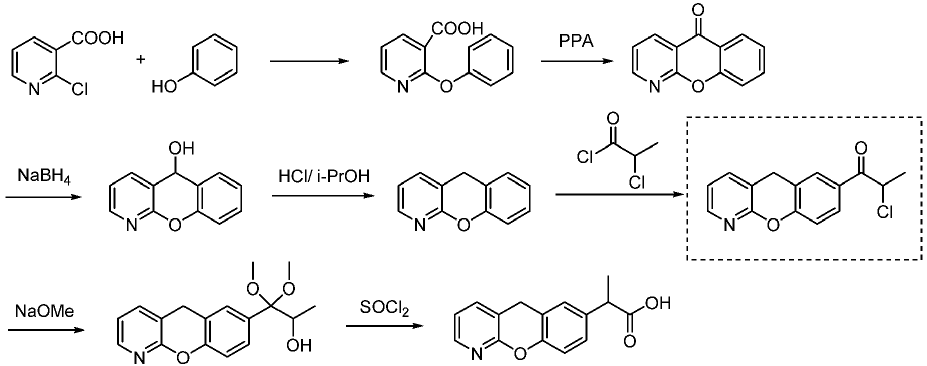

The disadvantage of Route 1 is that polyphosphoric acid (PPA) is used in the production process, and a large amount of phosphorus-containing wastewater would be produced in the post-treatment process, which is not conducive to environmental protection; 2-chloropropionyl chloride and sulfuryl chloride are also used in the process, and the odor is highly irritating, which is not conducive to the labor protection of production personnel; the rearrangement reaction is placed at the end, making it difficult to purify related substances and inconvenient for quality control of finished product.

Route 2: It is based on the idea of using side chain "2+1", using cyanide as carbon source to introduce carboxyl, and using cyanide to extend carbon chain. In the route, 2-chloronicotinic acid and p-ethylphenol are used as raw materials, undergoes nucleophilic substitution and Friedel-Crafts ring closure to obtain 7-ethyl-5H-[1]-benzopyrano[2,3-b]pyridin-5-one, undergoes halogenation reaction and reduction reaction to obtain α-halo-5H-[1]-benzopyran[2,3-b]pyridine-7-ethane, and then undergoes carbon chain extension using cyanide to obtain α-methyl-5H-[1]-benzopyran[2,3-b]pyridine-7-acetonitrile, and finally undergoes cyano hydrolysis to obtain pranoprofen.

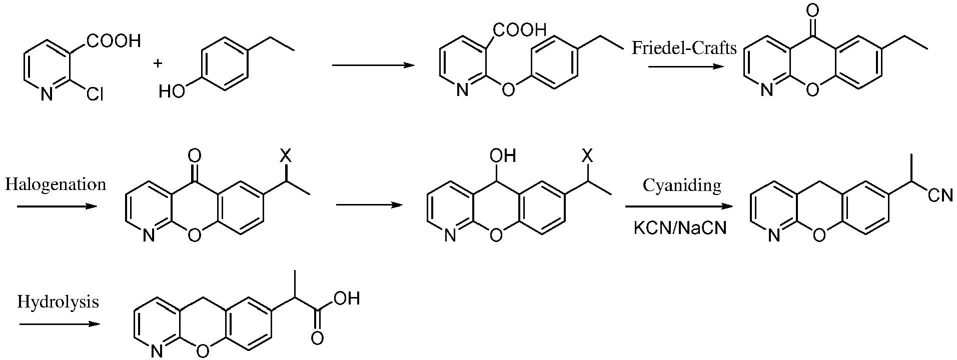

The disadvantage of Route 2 is that the highly toxic reagent cyanide is used in the production, which increases safety risks and is not conducive to labor protection.

Route 3: It is based on the idea of using side chain "2+1", using CO₂ as carbon source to introduce carboxyl, and using CO₂ to extend carbon chain. In the route, 2-chloronicotinic acid and p-ethylphenol are used as raw materials, undergoes nucleophilic substitution and Friedel-Crafts ring closure to obtain 7-ethyl-5H-[1]-benzopyrano[2,3-b]pyridin-5-one, undergoes halogenation reaction and reduction reaction to obtain α-halo-5H-[1]-benzopyran[2,3-b]pyridine-7-ethane, and this halogenated hydrocarbon reacts with magnesium to form Grignard reagent, then is fed with carbon dioxide, and undergoes hydrolysis under acidic conditions to obtain pranoprofen.

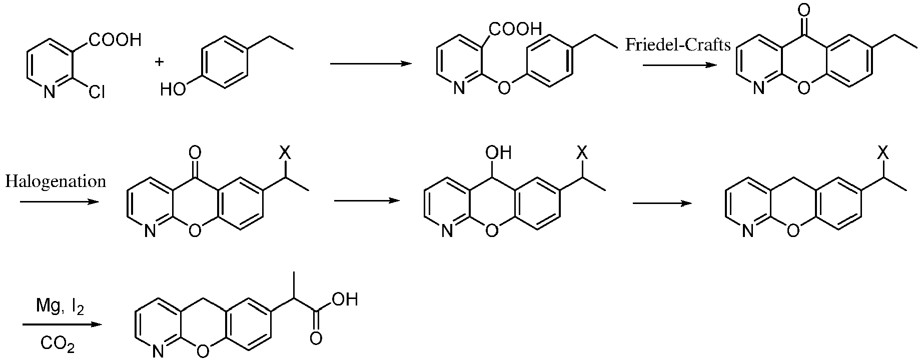

The disadvantage of Route 3 is that the reaction requires high conditions, that are, Grignard reaction is required, and cryogenic conditions at -80°C are required, the conditions are relatively harsh, and it is not conducive to industrial production.

Route 4: It is based on the idea of using side chain "2+1", using iodomethane as carbon source to introduce methyl. By using 2-chloronicotinic acid and p-hydroxyphenylacetonitrile or methyl 4-hydroxyphenylacetate as starting materials, 7-R-5H-[1]-benzopyrano [2,3-b]pyridin-5-one (R=COOMe, CN) is obtained through substitution reaction and Friedel-Crafts ring closure reaction. Then, reduction with sodium borohydride and hydrolysis with isopropanol hydrochloride are performed to obtain 7-R-SH-[1]-benzopyrano[2,3-b]pyridine (R=COOMe, CN). By using iodomethane, a methyl is introduced at the α position, and then hydrolysis of cyano or ester group is performed to obtain pranoprofen.

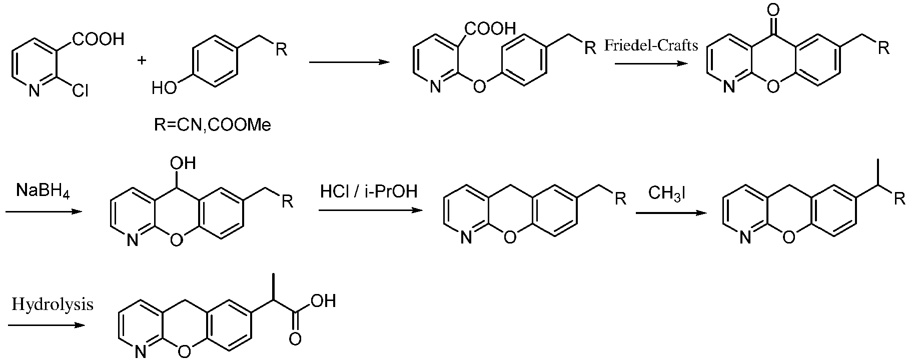

The disadvantage of Route 4 is that when iodomethane is used to introduce methyl, the reaction selectivity is poor, and the purification of dimethylated impurities is difficult, which is not conducive to product quality. And since iodomethane is a highly toxic reagent, it is not conducive to production and the health of researchers.

Route 5: It is based on using side chain "1+1+1" to build the three-carbons side chain. In the route, 2-chloronicotinic acid and phenol are used as raw materials, undergoes nucleophilic substitution and phosphorus oxychloride-catalyzed Friedel-Crafts reaction for ring closure to obtain SH-[1]-benzopyrano[2,3-b]pyridin-5-one, and then undergoes reduction with Na-Hg/ethanol and hydrolysis with isopropanol hydrochloride to construct the tricyclic parent SH-[1]-benzopyrano[2,3-b]pyridine. Then, the first carbon is introduced by chloromethylation, the second carbon is introduced by cyanation, and finally the third carbon is introduced by iodomethane to obtain methyl α-methyl-5H-[1]-benzopyrano[2,3-b]pyridine-7-acetate, which is finally hydrolyzed to obtain pranoprofen.

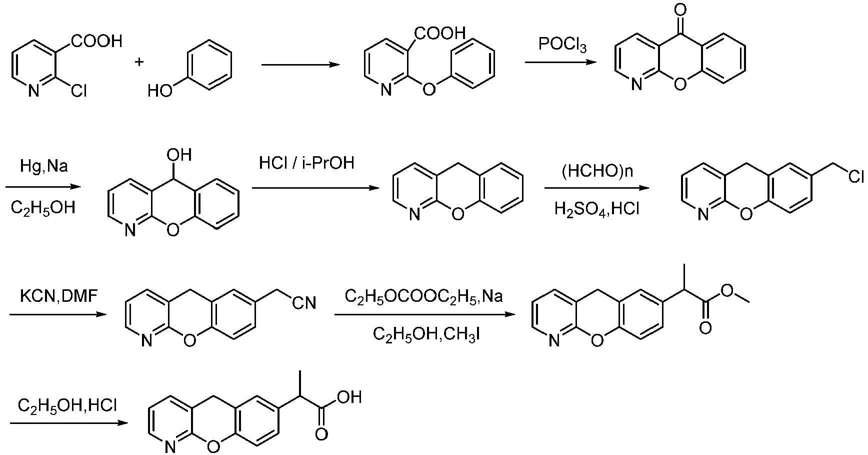

The disadvantage of Route 5 is that the reaction steps are lengthy, which has effects on labor, energy consumption and yield. In addition, highly toxic reagents such as phosphorus oxychloride, cyanide, and iodomethane are used in the production, which increases safety risks and is not conducive to the health of production personnel and researchers; and the introduction of iodomethane also easily produces dimethylated impurities, which are difficult to be separated and removed, thereby affecting product quality.

In addition, with regard to stability and impurities, the Japanese Pharmacopoeia stipulates that the total impurities should not exceed 1.0%, and single impurity should not exceed 0.5%, but there is no clear regulation on the structure of impurities. Pranoprofen is not currently included in the Chinese Pharmacopoeia, so there is no relevant regulation.

Generally speaking, there are a lot of defects of synthesizing pranoprofen in the prior art, which mainly concentrate on the following aspects: lengthy synthetic route and lower total yield; for example, Route 1 has a total yield of 23.7%, Route 3 has a total yield of 29.2%, Route 5 has a total yield of 3.7% (Route 2 and Route 4 utilize many existing technologies, but their total yields are not cleared reported); the use of highly toxic chemical reagents that is not conducive to ensure personnel safety; harsh reaction conditions that are not conducive to industrial production, etc. In terms of impurities and impurity content, there is also room for improvement. For example, the product will degrade during the placement process, and impurities will gradually increase. In addition, if the impurity content of bulk drug is high, it will affect the quality of eye drop product. Therefore, it is necessary to improve the purity of bulk drug and reduce the types and quantities of impurities.

At present, it is necessary to innovate a synthesis process of pranoprofen and develop a process technology that is more suitable for commercial production.

### Contents of the present invention

The inventors have obtained a new method for preparing pranoprofen through in-depth research and creative work. The inventors surprisingly found that the method overcomes the shortcomings of the prior art, shortens the synthesis route, lowers the requirements for process conditions, simplifies the process operation, improves labor safety, is more suitable for industrial production, is more environmentally friendly, and has a higher yield.

Therefore, the following invention is provided:
(I)
   One aspect of the present invention relates to a compound represented by Formula I or pharmaceutically acceptable salt or ester thereof, 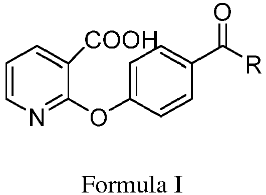 wherein, R is C₂-C₁₀ straight chain or branched chain alkyl, preferably, R is C₂-C₆ straight chain or branched chain alkyl, more preferably, R is C₂-C₄ straight chain or branched chain alkyl, such as ethyl, propyl, isopropyl or n-butyl.

Another aspect of the present invention relates to a method for preparing a compound represented by Formula I, comprising a step of reacting a compound represented by Formula A with a compound represented by Formula B to generate the compound represented by Formula I: 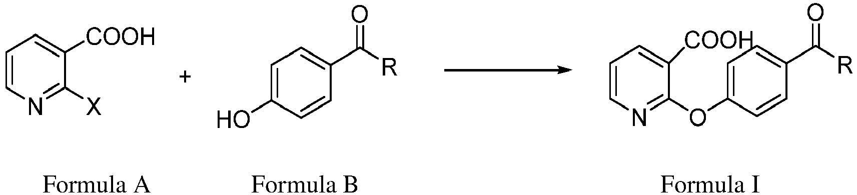 wherein,
X is halogen, such as fluorine, chlorine, bromine or iodine, preferably chlorine or bromine;
R is C₂-C₁₀ straight chain or branched chain alkyl, preferably, R is C₂-C₆ straight chain or branched chain alkyl, more preferably, R is C₂-C₄ straight chain or branched chain alkyl, such as ethyl, propyl, isopropyl or n-butyl.

The structural formulas of Formula A compound and Formula B compound are as follows respectively: 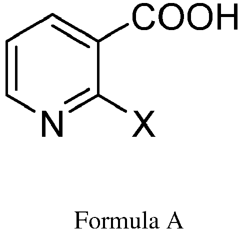 wherein,
X is halogen such as fluorine, chlorine, bromine or iodine, preferably chlorine or bromine. 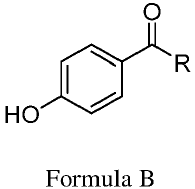 wherein, R is C₂-C₁₀ straight chain or branched chain alkyl, preferably, R is C₂-C₆ straight chain or branched chain alkyl, more preferably, R is C₂-C₄ straight chain or branched chain alkyl, such as ethyl, propyl, isopropyl or n-butyl.

In some embodiments of the present invention, in the method for preparing the compound represented by Formula I, the compound represented by Formula A (2-halogenated nicotinic acid) is selected from 2-chloronicotinic acid, 2-bromonicotinic acid and 2-iodonicotinic acid; preferably 2-chloronicotinic acid or 2-bromonicotinic acid.

In some embodiments of the present invention, in the method for preparing the compound represented by Formula I, the compound represented by Formula B (4-hydroxyphenyl ketone compound) is selected from 4-hydroxypropiophenone, 4-hydroxybutyrophenone and 4-hydroxyphenyl pentyl ketone.

In some embodiments of the present invention, in the method for preparing the compound represented by Formula I, the reaction system temperature is 60°C to 150°C, preferably 80°C to 120°C, more preferably 95°C to 120°C, 95°C to 110°C, 105°C to 120°C, 105°C to 115°C, 110°C to 120°C, 95°C to 105°C, 105°C to 110°C, 95°C, 100°C, 105°C, 110°C, 115°C or 120°C.

In some embodiments of the present invention, in the method for preparing the compound represented by Formula I, the reaction time is at least 1 hour, at least 2 hours, at least 3 hours, at least 4 hours, at least 5 hours, 1 to 10 hours, 2 to 8 hours, 3 to 7 hours, 4 to 6 hours, 1 hour, 2 hours, 3 hours, 4 hours, 5 hours, 6 hours, 7 hours, 8 hours, 9 hours or 10 hours.

In some embodiments of the present invention, in the method for preparing the compound represented by Formula I, the compound represented by Formula A and the compound represented by Formula B react in an organic solvent as a reaction solvent, preferably, the organic solvent is N,N-dimethylformamide or dimethylsulfoxide; more preferably, the organic solvent is N,N-dimethylformamide.

In some embodiments of the present invention, in the method for preparing the compound represented by Formula I, the compound represented by Formula A and the compound represented by Formula B react under the action of a catalyst, preferably, the catalyst is cuprous iodide.

In some embodiments of the present invention, in the method for preparing the compound represented by Formula I, the reaction system further comprises an acid-binding agent, such as potassium carbonate, sodium carbonate, potassium bicarbonate or sodium bicarbonate.

In some embodiments of the present invention, in the method for preparing the compound represented by Formula I, according to the calculation of molar ratio, the compound represented by Formula A: the compound represented by Formula B: the catalyst: the acid-binding agent is 1:(1 to 5):(0.05 to 1):(1 to 4), preferably 1:(1 to 3):(0.05 to 0.1):(1 to 2).

In some embodiments of the present invention, the method for preparing the compound represented by Formula I further comprises a step of isolating the compound represented by Formula I from the reaction system, preferably, comprises the following steps:
the reaction system is cooled (e.g., to 50°C or lower), and added with water, acidified to pH 3-4, the precipitated solid is collected, added with water to disperse, adjusted to have pH 7-7.5, the solid is filtered out, and the filtrate is acidified to pH 3-4, the precipitated solid is collected, washed with water and dried to obtain the compound represented by Formula I.

Without being limited by theory, the compound represented by Formula I may not be isolated, and directly and continuously undergo the reaction to prepare the compound represented by Formula II, but it is not a preferred scheme, because if there is no separation and purification process, the unreacted starting material of the compound represented by Formula I will also participate in the second step reaction (the reaction for preparing the compound represented by Formula II), which will consume additional IBD in the second step, and will also produce new impurities, reducing the purity of the product and increasing the difficulty of post-processing and purification.

In some embodiments of the present invention, the method for preparing the compound represented by Formula I comprises the following steps:
2-halogenated nicotinic acid (compound represented by Formula A), cuprous iodide and reaction solvent are added into a reaction vessel, controlled at a temperature of 60°C to 150°C, added with an acid-binding agent and 4-hydroxyphenyl ketone compound (compound represented by Formula B), controlled at a temperature of 60°C to 150°C, and reacted for 2-8 hours;
after cooling down, water is added, acidification is carried out to pH 3-4, the precipitated solid is collected, added with water to disperse, adjusted to pH 7-7.5 with sodium bicarbonate, the solid is filter out, the filtrate is acidified to pH 3-4, the precipitated solid is collected, washed with water and dried to obtain an intermediate 2-[4-(1-oxoalkyl)phenoxy]-3-pyridinecarboxylic acid (compound represented by Formula I).

In some embodiments of the present invention, in the method for preparing the compound represented by Formula I, the compound represented by Formula I has a yield greater than or equal to 50%, greater than or equal to 60%, greater than or equal to 70%, greater than or equal to 75%, 50% to 80%, 50% to 75%, 50% to 70%, 50% to 60%, 60% to 80%, 60% to 75%, 60% to 70%, 70% to 80%, 70% to 75%, 75% to 80%, or 75% to 85%.

Another aspect of the present invention relates to a use of the compound represented by Formula I or pharmaceutically acceptable salt or ester thereof in the manufacture of pranoprofen or a pharmaceutically acceptable salt or ester of pranoprofen. Preferably, the compound represented by Formula I is prepared by the method for preparing the compound represented by Formula I according to any one of items of the present invention.

The compound represented by Formula I also has other uses, for example, it can be used as an intermediate to synthesize other several compounds, such as 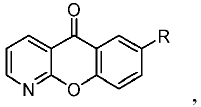 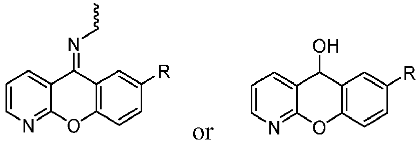 etc., wherein R is independently C₁-C₁₀ straight chain or branched chain alkyl, preferably, R is C₁-C₆ straight chain or branched chain alkyl, more preferably, R is C₁-C₄ straight chain or branched chain alkyl, such as ethyl, propyl, isopropyl or n-butyl.

(II)
One aspect of the present invention relates to a compound represented by Formula II, or a pharmaceutically acceptable salt or ester thereof, 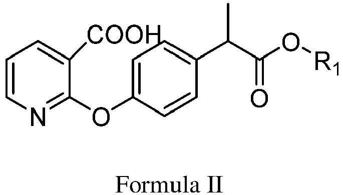 wherein, R₁ is C₁-C₁₀ straight chain or branched chain alkyl, preferably, Ri is C₁-C₅ straight chain or branched chain alkyl, more preferably, R₁ is C₁-C₃ straight chain or branched chain alkyl, such as methyl, ethyl, propyl or isopropyl.

Another aspect of the present invention relates to a method for preparing a compound represented by Formula II, comprising a step of reacting a compound represented by Formula I with a compound represented by Formula C and a compound represented by Formula D to generate a compound represented by Formula II: 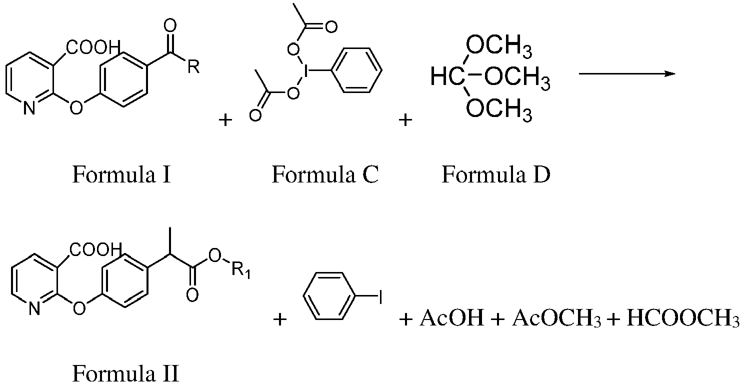 wherein,
R is C₂-C₁₀ straight chain or branched chain alkyl, preferably, R is C₂-C₆ straight chain or branched chain alkyl, more preferably, R is C₂-C₄ straight chain or branched chain alkyl, such as ethyl, propyl, isopropyl or n-butyl;
Ri is C₁-C₁₀ straight chain or branched chain alkyl, preferably, Ri is C₁-C₅ straight chain or branched chain alkyl, more preferably, R₁ is C₁-C₃ straight chain or branched chain alkyl, such as methyl, ethyl, propyl or isopropyl;
preferably, R has one more carbon atom than R₁.

In some embodiments of the present invention, in the method for preparing the compound represented by Formula II, the compound represented by Formula I is preferably 2-[4-(1-oxopropyl)phenoxy]-3-pyridinecarboxylic acid, 2-[4-(1-oxobutyl)phenoxy] - 3-pyridinecarboxylic acid or 2-[4-(1-oxopentyl)phenoxy]-3-pyridinecarboxylic acid, more preferably 2-[4-(1-oxopropyl)phenoxy]-3-pyridinecarboxylic acid. Preferably, the compound represented by Formula I is prepared by the method for preparing the compound represented by Formula I according to any one of items of the present invention.

In some embodiments of the present invention, the method for preparing the compound represented by Formula II further comprises the following steps:
the compound represented by Formula I is prepared by the method for preparing the compound represented by Formula I according to any one of items of the present invention.

In some embodiments of the present invention, in the method for preparing the compound represented by Formula II, the reaction system temperature is -5°C to 30°C, preferably -5°C to 20°C, more preferably - 5°C to 15°C, -5°C to 10°C, -5°C to 5°C, 5°C to 20°C, 5°C to 15°C, 5°C to 10°C, 10°C to 20°C, 10°C to 15°C, 15°C to 20°C, -5°C, 5°C, 10°C, 15°C or 20°C.

In some embodiments of the present invention, in the method for preparing the compound represented by Formula II, the reaction time is at least 0.2 hours, at least 0.4 hours, at least 0.6 hours, at least 0.8 hours, at least 0.9 hours, at least 1 hour, 0.2 to 2 hours, 0.4 to 1.6 hours, 0.6 to 1.4 hours, 0.8 to 1.2 hours, 0.2 hours, 0.4 hours, 0.6 hours, 0.8 hours, 1 hour, 1.2 hours, 1.4 hours, 1.6 hours, 1.8 hours or 2 hours.

In some embodiments of the present invention, in the method for preparing the compound represented by Formula II, the compound represented by Formula I reacts with the compound represented by Formula C in an organic solvent as a reaction solvent, preferably, the organic solvent is trimethyl orthoformate. Without being limited by theory, trimethyl orthoformate is not only a reaction solvent, but also a substrate, participating in the reaction.

In some embodiments of the present invention, in the method for preparing the compound represented by Formula II, the compound represented by Formula I reacts with the compound represented by Formula C under the action of a catalyst, preferably, the catalyst is an acid, preferably, the acid is one or more selected from the group consisting of concentrated sulfuric acid, hydrochloric acid, phosphoric acid and acetic acid, preferably concentrated sulfuric acid.

In some embodiments of the present invention, in the method for preparing the compound represented by Formula II, according to the calculation of molar ratio, the compound represented by Formula I: iodobenzene diacetate: acid is 1: (1-3): (1-4), preferably 1:(1-2):(1-3).

In some embodiments of the present invention, the method for preparing the compound represented by Formula II further comprises a step of isolating the compound represented by Formula II from the reaction system, preferably, comprises the following steps:
an alkaline solution (e.g., sodium bicarbonate aqueous solution) is added for neutralization, extraction is carried out with an organic solvent (preferably dichloromethane), the aqueous phase is retained, acidified to pH 3-4, the precipitated solid is collected, washed with water and dried to obtain the compound represented by Formula II.

Without being limited by theory, if the compound represented by Formula II is not isolated, but directly and continuously undergoes the following reaction to prepare the compound represented by Formula III, excess trimethyl orthoformate may participate in the next step reaction, reducing the yield and generating impurities.

In some embodiments of the present invention, the method for preparing the compound represented by Formula II comprises the following steps:
the compound represented by Formula I and triethyl orthoformate are added into a reaction vessel, slowly added with the acid solution, and the reaction temperature is controlled at -5°C to 30°C;
iodobenzene diacetate is added slowly, and the reaction temperature is controlled at -5°C to 30°C;
the acid solution is added again slowly, and the reaction temperature is controlled at -5°C to 30°C;
the reaction is carried out by keeping the temperature for 0.5 to 2 hours, a sodium bicarbonate aqueous solution is added for neutralization, extraction is performed with dichloromethane, then the water phase is retained, acidified to pH 3-4, the precipitated solid is collected, washed with water and dried to obtain an intermediate 2-[4-(2-alkoxy-1-methyl-2-oxoethyl)phenoxy]-3-pyridinecarboxylic acid (compound represented by Formula II).

Another aspect of the present invention relates to a use of the compound represented by Formula II or pharmaceutically acceptable salt or ester thereof in the manufacture of pranoprofen or a pharmaceutically acceptable salt or ester of pranoprofen. Preferably, the compound represented by Formula II is prepared by the method for preparing the compound represented by Formula II according to any one of items of the present invention.

The compound represented by Formula II also has other uses, for example, it can be used as an intermediate to synthesize other several compounds, such as 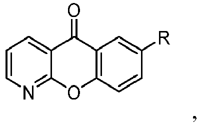 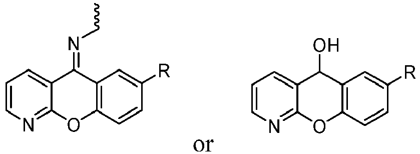 etc., wherein R is independently C₁-C₁₀ straight chain or branched chain alkyl, preferably, R is C₁-C₆ straight chain or branched chain alkyl, more preferably, R is C₁-C₄ straight chain or branched chain alkyl, such as ethyl, propyl, isopropyl or n-butyl.

(III)
One aspect of the present invention relates to a compound represented by Formula III, or a pharmaceutically acceptable salt or ester thereof, 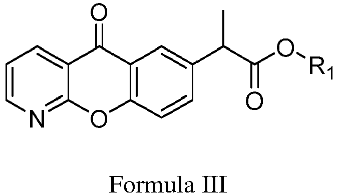 wherein, Ri is C₁-C₁₀ straight chain or branched chain alkyl, preferably, Ri is C₁-C₅ straight chain or branched chain alkyl, more preferably, R₁ is C₁-C₃ straight chain or branched chain alkyl, such as methyl, ethyl, propyl or isopropyl.

Another aspect of the present invention relates to a method for preparing a compound represented by Formula III, comprising a step of preparing a compound represented by Formula III from a compound represented by Formula II: 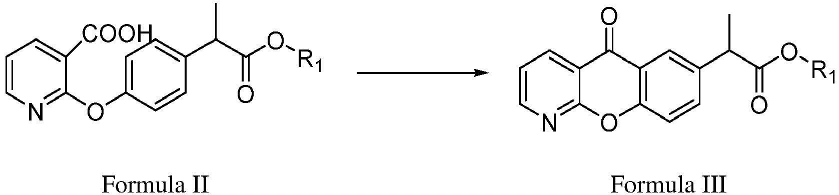 wherein, Ri is C₁-C₁₀ straight chain or branched chain alkyl, preferably, Ri is C₁-C₅ straight chain or branched chain alkyl, more preferably, R₁ is C₁-C₃ straight chain or branched chain alkyl, such as methyl, ethyl, propyl or isopropyl.

The above reaction scheme contains two reactions, as shown below; the product in the middle square brackets is not separated; preferably, it is directly fed for subsequent reaction.

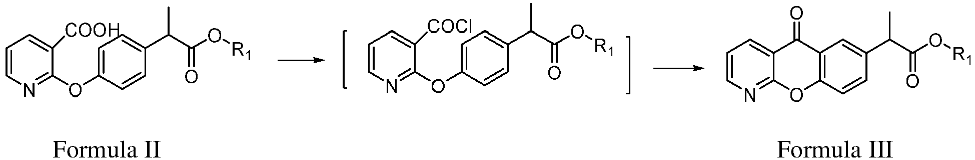

In some embodiments of the present invention, in the method for preparing the compound represented by Formula III, the compound represented by Formula II is preferably 2-[4-(2-methoxy-1-methyl-2-oxoethyl)phenoxy]-3-pyridinecarboxylic acid, 2-[4-(2-ethoxy-1-methyl-2-oxoethyl)phenoxy]-3-pyridinecarboxylic acid or 2-[4-(2-propoxy-1-methyl-2-oxoethyl)phenoxy]-3-pyridinecarboxylic acid; more preferably 2-[4-(2-methoxy-1-methyl-2-oxoethyl)phenoxy]-3-pyridinecarboxylic acid. Preferably, the compound represented by Formula II is prepared by the method for preparing the compound represented by Formula II according to any one of items of the present invention.

In some embodiments of the present invention, the method for preparing the compound represented by Formula III further comprises the following step:
the compound represented by Formula II is prepared by the method for preparing the compound represented by Formula II according to any one of items of the present invention.

In some embodiments of the present invention, the method for preparing the compound represented by Formula III comprises the following steps:
(1) reacting the compound represented by Formula II with an acylating agent to obtain a reaction product,
(2) generating the compound represented by Formula III from the reaction product in step (1) under the action of a Lewis acid;
   preferably, further comprises the following step:
(3) isolate the compound represented by Formula III; preferably, cooling the reaction product obtained in step (2) to below 20°C (e.g., adding cold water to quench), adjusting pH, and removing the solvent under reduced pressure to obtain the compound represented by Formula III.

In some embodiments of the present invention, in the method for preparing the compound represented by Formula III, the reaction temperature in step (1) is 10°C to 30°C, preferably 15°C to 25°C, 15°C to 20°C or 20°C to 25°C.

In some embodiments of the present invention, in the method for preparing the compound represented by Formula III, the reaction time in step (1) is at least 0.2 hours, at least 0.4 hours, at least 0.6 hours, at least 0.8 hours, at least 0.9 hours, at least 1 hour, 0.2-2 hours, 0.4-1.6 hours, 0.6-1.4 hours, 0.8-1.2 hours, 0.2 hours, 0.4 hours, 0.6 hours, 0.8 hours, 1 hour, 1.2 hours, 1.4 hours, 1.6 hours, 1.8 hours or 2 hours.

In some embodiments of the present invention, in the method for preparing the compound represented by Formula III, step (1) further comprises the following step:
cooling the obtained reaction product to -10°C to 0°C.

In some embodiments of the present invention, in the method for preparing the compound represented by Formula III, the reaction temperature in step (2) is 10°C to 30°C, preferably 15°C to 25°C, 15°C to 20°C or 20°C to 25°C.

In some embodiments of the present invention, in the method for preparing the compound represented by Formula III, the reaction time in step (2) is at least 0.2 hours, at least 0.4 hours, at least 0.6 hours, at least 0.8 hours, at least 0.9 hours, at least 1 hour, 0.2 to 2 hours, 0.4 to 1.6 hours, 0.6 to 1.4 hours, 0.8 to 1.2 hours, 1 to 1.5 hours, 0.2 hours, 0.4 hours, 0.6 hours, 0.8 hours, 1 hour, 1.2 hours, 1.4 hours, 1.5 hours, 1.6 hours, 1.8 hours or 2 hours.

In some embodiments of the present invention, in the method for preparing the compound represented by Formula III, the acylating agent is one or more selected from oxalyl chloride and thionyl chloride.

In some embodiments of the present invention, in the method for preparing the compound represented by Formula III, the Lewis acid is a catalyst; preferably, the Lewis acid is one or more selected from anhydrous aluminum trichloride and titanium tetrachloride, preferably anhydrous aluminum trichloride.

In some embodiments of the present invention, in the method for preparing the compound represented by Formula III, the reaction is carried out in an organic solvent as a reaction solvent, preferably, the organic solvent is dichloromethane and/or *N,N*-dimethylformamide. Without being limited by theory, dichloromethane is the reaction solvent, and the amount of N,N-dimethylformamide is only 0.1 equivalent, which is more suitable as a catalyst.

In some embodiments of the present invention, in the method for preparing the compound represented by Formula III, the molar ratio of the compound represented by Formula II to the acylating agent is (1:1) to (1:5), preferably 1:(1.5-3), particularly preferably 1:2.5.

In some embodiments of the present invention, in the method for preparing the compound represented by Formula III, the molar ratio of the compound represented by Formula II to oxalyl chloride is (1:1) to (1:5), preferably 1:(1.5-3), particularly preferably 1:2.5.

In some embodiments of the present invention, in the method for preparing the compound represented by Formula III, step (3) comprises the following operations:
cold water is added to the reaction product of step (2) to quench the reaction, the organic phase is separated, adjusted to pH 7-8 (e.g., with sodium carbonate solution), and then the organic phase is separated; the organic phase is concentrated under reduced pressure until the distillate is no longer evaporated, methanol or a mixed solvent of methanol and dichloromethane is added for beating for 3 to 8 hours (preferably 5 to 6 hours), after filtration, the filter residue is rinsed with methanol and dried to obtain the compound represented by Formula III. In some embodiments of the present invention, the volume ratio of methanol to dichloromethane is 1:(0-0.4), preferably 1:(0.1-0.2).

Without being limited by theory, the present inventors have found that if the compound represented by Formula III is not isolated, it is not possible to prepare the compound represented by Formula IV by direct and continuous reaction, because there is a large amount of aluminum trichloride in the system when the compound represented by Formula III is prepared. During the reaction of step 4, it will react with methanol, affecting the yield. In addition, when aluminum trichloride is mixed with potassium borohydride/sodium borohydride, it may further reduce the methyl ester of the compound represented by Formula III to generate an alcohol, rather than the compound represented by Formula IV.

In some embodiments of the present invention, the method for preparing the compound represented by Formula III comprises the following steps:
the compound represented by Formula II, dichloromethane and N,N-dimethylformamide are added into a first reaction vessel, mixed and stirred, the acylating reagent is added at a temperature kept at 15°C to 25°C, the reaction is carried out by keeping the temperature for 0.5 to 2 hours, and cooled down to -10°C to 0°C for standby;
the Lewis acid and dichloromethane are added into a second reaction vessel, stirred evenly, the solution in the first reaction vessel is added at a temperature kept at 15°C to 25°C, the reaction is carried out by keeping the temperature for 1 to 1.5 hours, and cold water is slowly added at a temperature kept below 20°C for quenching;
the organic phase is retained, adjusted with sodium carbonate solution to pH 7-8, subjected to liquid separation, and the organic phase is concentrated under reduced pressure;
when the distillate is no longer evaporated, a mixed solvent of methanol and dichloromethane is added for beating for 5-6 hours, after filtration, the solid is rinsed with methanol, and dried to obtain an intermediate 2-(10-oxo-9-oxa-1-aza-anthracen-6-yl)propionate compound (compound of Formula III).

The inventors have also surprisingly found that specific impurities were contained in the obtained product, including the impurities generated by passing the by-products of the compound represented by Formula II and the compound represented by Formula II, which are named as Formula III compound-IMP-A, Formula III compound-IMP-B, Formula III compound-IMP-C, etc., and their structures are as follows: 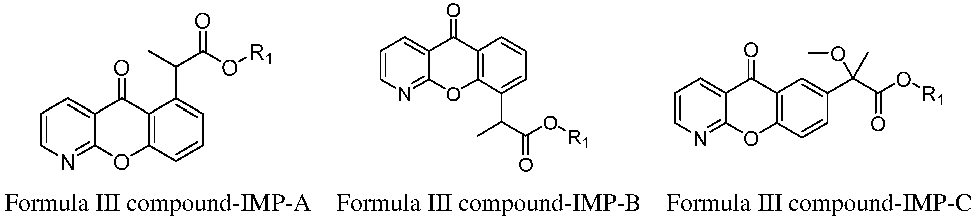 wherein, Ri is C₁-C₁₀ straight chain or branched chain alkyl, preferably, Ri is C₁-C₅ straight chain or branched chain alkyl, more preferably, R₁ is C₁-C₃ straight chain or branched chain alkyl, such as methyl, ethyl, propyl or isopropyl.

The inventor speculates that if these three impurities are passed in the subsequent reaction, they may be derivatized into impurities as follows, which are similar to the structure of pranoprofen:

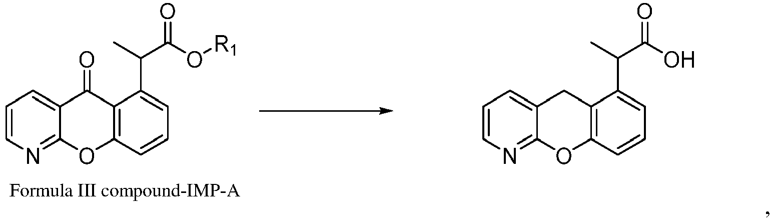

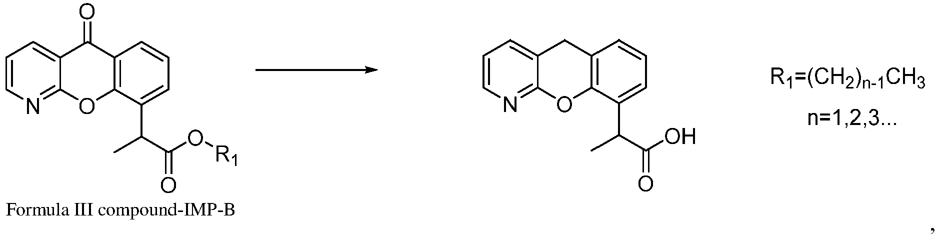

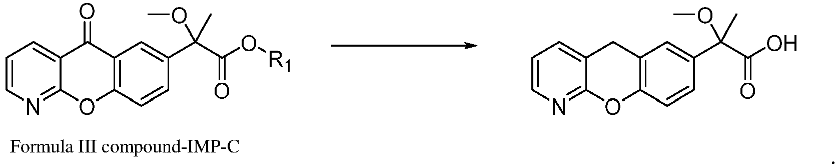

Without being limited by theory, since the structures of these derivatized impurities are very similar to that of pranoprofen, it is speculated that it is difficult to separate and remove them from the finished product. Therefore, these impurities should be removed by refining in advance, and the limits of these three impurities should be controlled. For example, it is required that each impurity should not exceed 0.1% of the total mass of the intermediate product (the maximum amount of impurities produced by the conversion of these three impurities should not exceed 0.1%), and the quality of the finished product can be controlled in the upstream step; so the finished product does not need to be additionally tested for derivative impurities.

In some embodiments of the present invention, the following step is used to remove these impurities by refining:
after the solvent dichloromethane is distilled off, methanol or methanol/dichloromethane mixed solvent is used for pulp washing (that is, beating). By using an optimized solvent ratio, the impurities can be controlled at desired levels.

The inventor has carried out verification with multiple batches of experimental data, and when these three impurities are controlled at below 0.1%, the derivatized impurities of these three impurities are not detected in the finished product pranoprofen.

Another aspect of the present invention relates to a use of the compound represented by Formula III or pharmaceutically acceptable salt or ester thereof in the manufacture of pranoprofen or a pharmaceutically acceptable salt or ester of pranoprofen. Preferably, the compound represented by Formula III is prepared by the method for preparing the compound represented by Formula III according to any one of items of the present invention.

The compound represented by Formula III also has other uses, for example, it can be used as an intermediate to synthesize other several compounds, such as 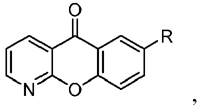 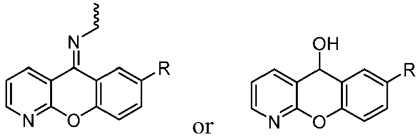 etc., wherein R is independently C₁-C₁₀ straight chain or branched chain alkyl, preferably, R is C₁-C₆ straight chain or branched chain alkyl, more preferably, R is C₁-C₄ straight chain or branched chain alkyl, such as ethyl, propyl, isopropyl or n-butyl.

(IV)
One aspect of the present invention relates to a compound represented by Formula IV, or a pharmaceutically acceptable salt or ester thereof, 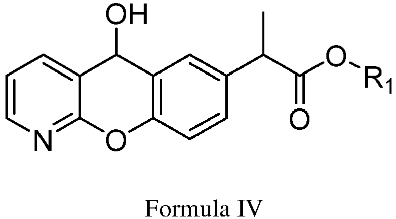 wherein, Ri is C₁-C₁₀ straight chain or branched chain alkyl, preferably, Ri is C₁-C₅ straight chain or branched chain alkyl, more preferably, R₁ is C₁-C₃ straight chain or branched chain alkyl, such as methyl, ethyl, propyl or isopropyl.

Another aspect of the present invention relates to a method for preparing a compound represented by Formula IV, comprising a step of preparing a compound represented by Formula IV from a compound represented by Formula III: 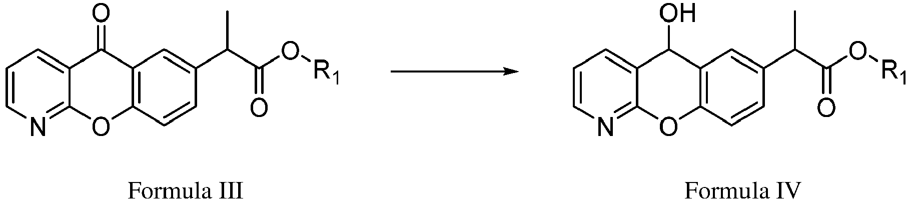 wherein, Ri is C₁-C₁₀ straight chain or branched chain alkyl, preferably, Ri is C₁-C₅ straight chain or branched chain alkyl, more preferably, R₁ is C₁-C₃ straight chain or branched chain alkyl, such as methyl, ethyl, propyl or isopropyl.

In some embodiments of the present invention, in the method for preparing the compound represented by Formula IV, the compound represented by Formula III is preferably methyl 2-(10-oxo-9-oxa-1-azaanthracen-6-yl)propionate, ethyl 2-(10-oxo-9-oxa-1-azaanthren-6-yl)propionate or propyl 2-(10-oxo-9-oxa-1-azaanthracen-6-yl) propionate; more preferably methyl 2-(10-oxo-9-oxa-1-azaanthracen-6-yl)propionate. Preferably, the compound represented by Formula III is prepared by the method for preparing the compound represented by Formula III according to any one of items of the present invention.

In some embodiments of the present invention, the method for preparing the compound represented by Formula IV further comprises the following step:
the compound represented by Formula III is prepared by the method for preparing the compound represented by Formula III according to any one of items of the present invention.

In some embodiments of the present invention, in the method for preparing the compound represented by Formula IV, the compound represented by Formula IV is prepared by reacting the compound represented by Formula III with a reducing agent. Preferably, the reducing agent is borohydride, more preferably sodium borohydride.

In some embodiments of the present invention, in the method for preparing the compound represented by Formula IV, the reaction temperature is 20°C to 40°C, preferably 30°C to 40°C, 30°C to 35°C, or 35°C to 40°C.

In some embodiments of the present invention, in the method for preparing the compound represented by Formula IV, the reaction time is at least 1 hour, at least 2 hours, at least 3 hours, at least 4 hours, at least 5 hours, at least 6 hours, 1 to 100 hours, 2 to 50 hours, 3 to 40 hours, 4 to 30 hours, 5 to 25 hours, 6 to 20 hours, 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours or 20 hours.

In some embodiments of the present invention, in the method for preparing the compound represented by Formula IV, the reaction is carried out in an organic solvent as a reaction solvent, preferably, the organic solvent is dichloromethane and methanol (mixed solvent). Without being bound by theory, dichloromethane is the solvent, and methanol is both the solvent and the substrate to provide the source of hydrogen. The molar ratio of dichloromethane to methanol is 1:(2.11-3.16), preferably 1:2.11. In some embodiments of the present invention, the mass (g) of the compound represented by Formula III: the volume (mL) of the mixed solvent is 1:(5-15), preferably 1:(8-10).

In some embodiments of the present invention, in the method for preparing the compound represented by Formula IV, the molar ratio of the compound represented by Formula III to the reducing agent is (1:0.8) to (1:1.2), preferably 1:0.9-1.1.

In some embodiments of the present invention, the method for preparing the compound represented by Formula IV further comprises a step of isolating the compound represented by Formula IV from the reaction system; preferably, comprises the following steps:
the reaction product is cooled to -10°C to 10°C, and adjusted to pH 5.5-6.5; preferably, a diluted acid solution (e.g., 30% acetic acid aqueous solution) is slowly added to pH 5.5-6.5 (not limited by theory, the function of acid is to quench the excess reducing agent in the reaction, and the acid can be either diluted inorganic acid or diluted organic acid); the diluted acid can be an inorganic acid or organic acid with a concentration less than or equal to 30%;
agitation is carried out at -10°C to 10°C for 5 to 30 minutes (preferably 15 minutes), purified water is added, the organic phase is collected and washed with saturated sodium bicarbonate and purified water in sequence;
the washed organic phase is concentrated to dryness under reduced pressure at 40°C to obtain a yellow-green oil;
a crystallization solvent is added and stirred at 20°C to 40°C for 5 to 30 minutes (preferably 15 minutes); and
purified water is slowly added dropwise, the solid is collected, washed with water, and dried in vacuum to obtain the compound represented by Formula IV.

In some embodiments of the present invention, in the method for preparing the compound represented by Formula IV, preferably, the crystallization solvent can be selected from the group consisting of methanol aqueous solution (preferably, methanol:water is 1:2), acetone aqueous solution (preferably, acetone:water is 1:2), a mixture of methanol, acetone and water (preferably, methanol:acetone:water is 1:1.5:5), preferably a mixture of methanol, acetone and water (preferably, methanol:acetone:water is 1:1.5:5). Preferably, the mass (g) of the compound represented by Formula III: the volume (mL) of the crystallization solvent is 1:5.

In some embodiments of the present invention, the method for preparing the compound represented by Formula IV comprises the following steps:
the compound represented by Formula III, dichloromethane and methanol are added into a reaction vessel, and stirred to dissolve at a temperature of 15°C to 30°C;
borohydride is slowly added, reacted by keeping temperature at 20°C to 40°C for 6 to 20 hours, cooled down to -10°C to 10°C, slowly added dropwise with 30% acetic acid aqueous solution to pH 5.5-6.5, controlled at temperature of -10°C to 10°C and stirred for 5-30 minutes (preferably 15 minutes), and added with purified water, the organic phase is collected, washed with saturated sodium bicarbonate, and washed again with purified water;
the organic phase is concentrated to dryness under reduced pressure at 40°C to obtain a yellow-green oil;
a mixed solution of methanol/acetone is added and stirred at 20°C to 40°C to disperse for 15 minutes, purified water is slowed added dropwise to crystallize for 1 to 5 hours (preferably 2 hours), the solid is collected, washed with water and dried in vacuum to obtain an intermediate 2-(10-hydroxy-9-oxa-1-azaanthracen-6-yl)propionate compound (compounds of Formula IV).

Without being bound by theory, the function of acetic acid is to quench excess sodium borohydride, and the pH range is controlled to ensure complete quenching.

Another aspect of the present invention relates to a use of the compound represented by Formula IV or pharmaceutically acceptable salt or ester thereof in the manufacture of pranoprofen or a pharmaceutically acceptable salt or ester of pranoprofen. Preferably, the compound represented by Formula IV is prepared by the method for preparing the compound represented by Formula IV according to any one of items of the present invention.

The compound represented by Formula IV also has other uses, for example, it can be used as an intermediate to synthesize other several compounds, such as 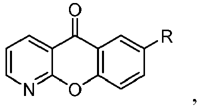 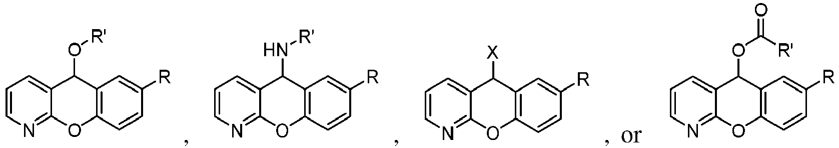 etc., wherein R and R' are each independently C₁-C₁₀ straight chain or branched chain alkyl, preferably, R is C₁-C₆ straight chain or branched chain alkyl, more preferably, R is C₁-C₄ straight chain or branched chain alkyl, such as ethyl, propyl, isopropyl or n-butyl; X is halogen, such as fluorine, chlorine, bromine or iodine.

(V)
Another aspect of the present invention relates to a method for preparing pranoprofen (compound represented by Formula 0) or a pharmaceutically acceptable salt or ester of pranoprofen, comprising a step of preparing:
the compound represented by Formula I or pharmaceutically acceptable salt or ester thereof,
the compound represented by Formula II or pharmaceutically acceptable salt or ester thereof,
the compound represented by Formula III or pharmaceutically acceptable salt or ester thereof, and/or
the compound represented by Formula IV or pharmaceutically acceptable salt or ester thereof;
preferably, the compound represented by Formula I is prepared by the method for preparing the compound represented by Formula I according to any one of items of the present invention;
preferably, the compound represented by Formula II is prepared by the method for preparing the compound represented by Formula III according to any one of items of the present invention;
preferably, the compound represented by Formula III is prepared by the method for preparing the compound represented by Formula III according to any one of items of the present invention;
preferably, the compound represented by Formula IV is prepared by the method for preparing the compound represented by Formula IV according to any one of items of the present invention.

In some embodiments of the present invention, in the method for preparing pranoprofen (compound represented by Formula 0), the synthetic route is shown as follows:

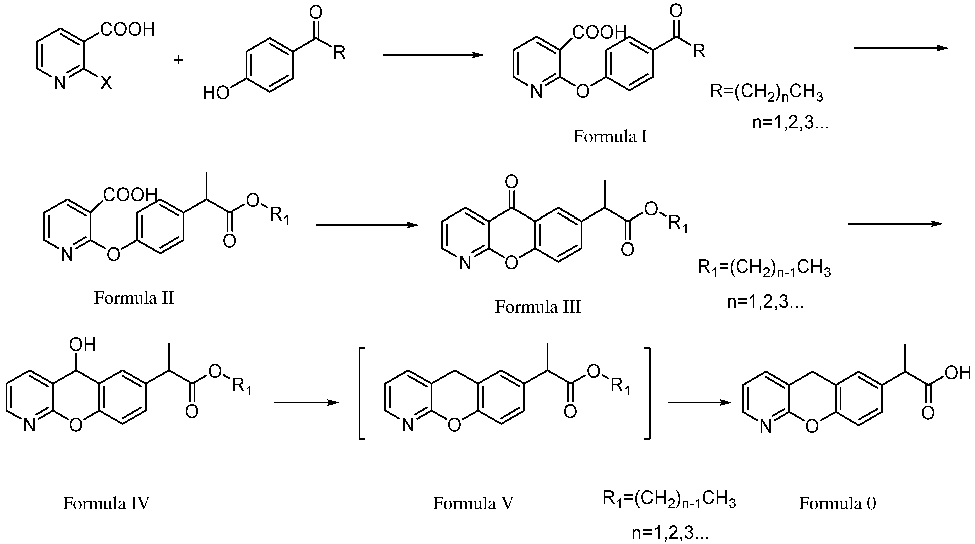

In some embodiments of the present invention, the method for preparing pranoprofen (compound represented by Formula 0) comprises the following steps:
(1) by using 2-halogenated nicotinic acid (compound represented by Formula A) and 4-hydroxyphenyl ketone compound (compound represented by Formula B) as starting materials, an intermediate 2-[4-(1-oxoalkyl)phenoxy]-3-pyridinecarboxylic acid (compound represented by Formula I) is prepared by Ullmann condensation reaction in the presence of cuprous iodide;
(2) under the action of acid and iodobenzene diacetate, an intermediate 2-[4-(2-methoxy-1-methyl-2-oxoalkyl)phenoxy]-3-pyridinecarboxylic acid (compound represented by Formula II) is prepared by rearrangement reaction in trimethyl orthoformate;
(3) after being halogenated to form acid chloride, an intermediate 2-(10-oxo-9-oxa-1-azaanthracen-6-yl)propionate compound (compound represented by Formula III) is prepared by intramolecular ring closure under the action of a Lewis acid;
(4) after being reduced with borohydride, an intermediate 2-(10-hydroxy-9-oxa-1-azaanthracen-6-yl)propionate compound (compound represented by Formula IV) is prepared;
(5) finally, pranoprofen (compound represented by Formula 0) is prepared by "one-pot method" through reduction with isopropanol hydrogen chloride, hydrolysis with a base and post-treatment acidification.

The structural formulas of Formula A compound and Formula B compound are as follows: 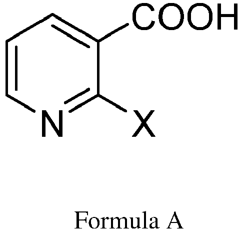 wherein,
X is halogen, such as fluorine, chlorine, bromine or iodine, preferably chlorine or bromine. 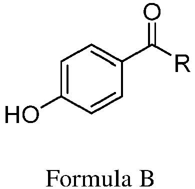 wherein, R is C₂-C₁₀ straight chain or branched chain alkyl, preferably, R is C₂-C₆ straight chain or branched chain alkyl, more preferably, R is C₂-C₄ straight chain or branched chain alkyl, such as ethyl, propyl, isopropyl or n-butyl.

Another aspect of the present invention relates to a method for preparing pranoprofen (compound represented by Formula 0) or a pharmaceutically acceptable salt or ester of pranoprofen, comprising a step of preparing pranoprofen from the compound represented by Formula IV: 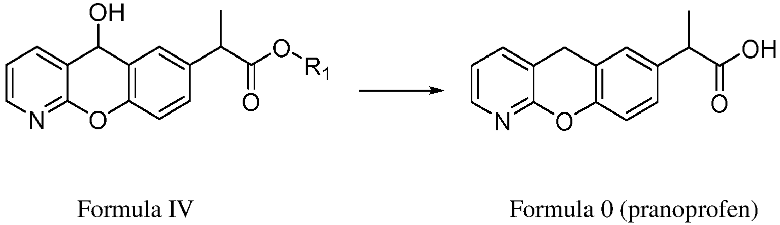 wherein, Ri is C₁-C₁₀ straight chain or branched chain alkyl, preferably, Ri is C₁-C₅ straight chain or branched chain alkyl, more preferably, R₁ is C₁-C₃ straight chain or branched chain alkyl, such as methyl, ethyl, propyl or isopropyl.

In some embodiments of the present invention, the method for preparing pranoprofen (compound represented by Formula 0) comprises the following steps:
(1) first preparing a compound represented by Formula V from the compound represented by Formula IV (reduction reaction);
(2) preparing pranoprofen (compound represented by Formula 0) from the compound represented by Formula V (hydrolysis reaction); 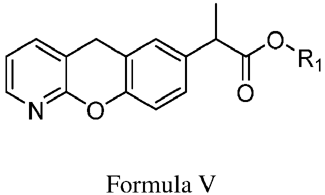
wherein, Ri is C₁-C₁₀ straight chain or branched chain alkyl, preferably, Ri is C₁-C₅ straight chain or branched chain alkyl, more preferably, R₁ is C₁-C₃ straight chain or branched chain alkyl, such as methyl, ethyl, propyl or isopropyl.

In some embodiments of the present invention, in the method for preparing pranoprofen (compound represented by Formula 0), the compound represented by Formula IV is preferably methyl 2-(10-hydroxyl-9-oxa-1-azaanthracen-6-yl)propionate, ethyl 2-(10-hydroxy-9-oxa-1-azaanthracen-6-yl)propionate or propyl 2-(10-hydroxy-9-oxa-1-azaanthracen-6-yl)propionate; more preferably methyl 2-(10-hydroxy-9-oxa-1-azaanthracen-6-yl)propionate.

In some embodiments of the present invention, in the method for preparing pranoprofen (compound represented by Formula 0), in step (1), the compound represented by Formula IV is reacted with a reducing agent to prepare the compound represented by Formula V.

In some embodiments of the present invention, in the method for preparing pranoprofen (compound represented by Formula 0), in step (1), the reducing agent is one or more selected from isopropanol hydrogen chloride and isopropanol hydrochloride.

In some embodiments of the present invention, in the method for preparing pranoprofen (compound represented by Formula 0), in step (1), the molar ratio of the compound represented by Formula IV to the reducing agent is 1:(1.5-3.5), preferably 1:(2.5-3.5), such as 1:2.5, 1:3 or 1:3.5.

In some embodiments of the present invention, in the method for preparing pranoprofen (compound represented by Formula 0), in step (1), the reaction is carried out in an organic solvent as a reaction solvent, preferably, the organic solvent is isopropanol.

In some embodiments of the present invention, in the method for preparing pranoprofen (compound represented by Formula 0), in step (1), the reaction temperature is 50°C to 90°C, preferably 60°C to 80°C, 60°C to 70°C, 70°C to 80°C, or 65°C to 75°C.

In some embodiments of the present invention, in the method for preparing pranoprofen (compound represented by Formula 0), in step (1), the reaction time is at least 0.5 hours, at least 1 hour, at least 1.5 hours, at least 2 hours, 1 to 10 hours, 2 to 8 hours, 2 to 6 hours, 2 to 4 hours, 2 to 3 hours, 0.5 hours, 1 hour, 1.5 hours, 2 hours, 3 hours, 4 hours, 5 hours, 6 hours, 7 hours, 8 hours, 9 hours or 10 hours.

In some embodiments of the present invention, in the method for preparing pranoprofen (compound represented by Formula 0), in step (2), the compound represented by Formula V is reacted with a base (hydrolysis reaction) to prepare pranoprofen (compound represented by Formula 0).

In some embodiments of the present invention, in the method for preparing pranoprofen (compound represented by Formula 0), in step (2), the base is one or more selected from the group consisting of sodium hydroxide, potassium hydroxide, lithium hydroxide, sodium carbonate and potassium carbonate, preferably sodium hydroxide and/or potassium hydroxide.

In some embodiments of the present invention, in the method for preparing pranoprofen (compound represented by Formula 0), in step (2), the reaction temperature is 40°C to 75°C, preferably 40°C to 55°C, 40°C to 50°C, 45°C to 55°C, 40°C to 45°C, 45°C to 50°C, or 50°C to 55°C.

In some embodiments of the present invention, in the method for preparing pranoprofen (compound represented by Formula 0), in step (2), the reaction time is at least 0.5 hours, at least 1 hour, at least 1.5 hours, 0.5 to 10 hours, 1 to 8 hours, 1.5 to 6 hours, 1.5 to 4 hours, 1.5 to 3 hours, 1.5 to 2.5 hours, 0.5 hours, 1 hour, 1.5 hours, 2 hours, 2.5 hours, 3 hours, 4 hours, 5 hours, 6 hours, 7 hours, 8 hours, 9 hours or 10 hours.

In some embodiments of the present invention, the method for preparing pranoprofen (compound represented by Formula 0) further comprises a step of isolating pranoprofen (compound represented by Formula 0) from the reaction system; preferably, comprises the following step (3):
adding purified water to the reaction system, and then extracting the aqueous phase with ethyl acetate;
collecting the aqueous phase and adjusting pH to 2-7 with an acid (preferably, adjusting with 20% diluted acid solution), and gradually precipitating a solid;
filtering, rinsing the filter cake successively with purified water and methanol, and drying in vacuum to obtain crude pranoprofen (compound represented by Formula 0); and
recrystallizing from methanol (preferably, recrystallizing under refluxing with 8 to 12 volumes of methanol, such as 10 volumes of methanol) to obtain refined pranoprofen (compound represented by Formula 0).

In some embodiments of the present invention, in the method for preparing pranoprofen (compound represented by Formula 0), in step (3), the pH is preferably 5-7, more preferably 5.5-6.5, especially preferably 5.8-6.2.

In some embodiments of the present invention, in the method for preparing pranoprofen (compound represented by Formula 0), in step (3), the acid is one or more selected from the group consisting of acetic acid, phosphoric acid, hydrochloric acid and sulfuric acid, preferably acetic acid and/or hydrochloric acid.

In some embodiments of the present invention, the method for preparing pranoprofen (compound represented by Formula 0) does not comprise a step of isolating the compound represented by Formula V.

In some embodiments of the present invention, in the method for preparing pranoprofen (compound represented by Formula 0), the compound represented by Formula IV is preferably methyl 2-(10-hydroxyl-9-oxa-1-azaanthen-6-yl)propionate, ethyl 2-(10-hydroxy-9-oxa-1-azaanthracen-6-yl)propionate or propyl 2-(10-hydroxy-9-oxa-1-azaanthren-6-yl)propionate; more preferably methyl 2-(10-hydroxy-9-oxa-1-azaanthracen-6-yl)propionate.

In some embodiments of the present invention, in the method for preparing pranoprofen (compound represented by Formula 0), the compound represented by Formula IV is prepared by the method for preparing compound represented by Formula IV according to any one of items of the present invention.

In some embodiments of the present invention, the method for preparing pranoprofen (compound represented by Formula 0) further comprises the following step:
the compound represented by Formula IV is prepared by the method for preparing the compound represented by Formula IV according to any one of items of the present invention.

In some embodiments of the present invention, in the method for preparing pranoprofen (compound represented by Formula 0), the molar ratio of the compound represented by Formula IV to the reducing agent is (1:1) to (1:5), preferably (1:1) to (1:3) or (1:1.5) to (1:3.5), more preferably (1:2) to (1:3) or (1:2) to (1:3.5 ), such as 1:2, 1:2.5 or 1:3.

In some embodiments of the present invention, the method for preparing pranoprofen (compound represented by Formula 0) comprises the following steps:
the compound represented by Formula IV, the reducing agent and isopropanol are added into a reaction vessel, stirred and heated to 50°C to 90°C, reacted by keeping the temperature for 2 to 4 hours, concentrated under reduced pressure at 55°C to dryness, added with methanol (methanol is used as the solvent) after being cooled to room temperature, then added with an alkaline water (or added with an alkaline water, then added with methanol after being cooled to room temperature), and reacted by keeping at temperature of 40°C to 75°C for 2 hours;
purified water is added to the reaction solution, then the aqueous phase is extracted with ethyl acetate, the aqueous phase is collected and adjusted to pH 2-7 with 20% diluted acid solution, and a solid is gradually precipitated;
after being filtered, the filter cake is rinsed with purified water and methanol successively;
the crude product is obtained by vacuum drying, and refined by methanol recrystallization to obtain the finished product of pranoprofen (compound represented by Formula 0).

In some embodiments of the present invention, the method for preparing pranoprofen (compound represented by Formula 0) comprises the process steps of Preparation Examples 1a, 2c, 3c, 4b, and 5a.

Another aspect of the present invention relates to a method for preparing a compound represented by Formula V or a pharmaceutically acceptable salt or ester of the compound represented by Formula V, comprising a step of preparing the compound represented by Formula V from the compound represented by Formula IV: 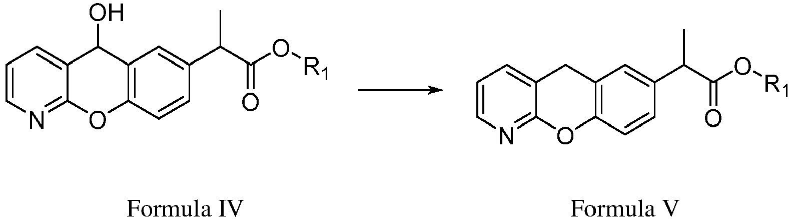 wherein, Ri is C₁-C₁₀ straight chain or branched chain alkyl, preferably, Ri is C₁-C₅ straight chain or branched chain alkyl, more preferably, R₁ is C₁-C₃ straight chain or branched chain alkyl, such as methyl, ethyl, propyl or isopropyl.

In some embodiments of the present invention, in the method for preparing the compound represented by Formula V, the compound represented by Formula V is prepared by reacting the compound represented by Formula IV with a reducing agent.

In some embodiments of the present invention, in the method for preparing the compound represented by Formula V, the reducing agent is one or more selected from the group consisting of isopropanol hydrogen chloride and isopropanol hydrochloride.

In some embodiments of the present invention, in the method for preparing the compound represented by Formula V, the molar ratio of the compound represented by Formula IV to the reducing agent is 1:(1.5-3.5), preferably 1:(2.5-3.5), for example 1:2.5, 1:3 or 1:3.5.

In some embodiments of the present invention, in the method for preparing the compound represented by Formula V, the reaction is carried out in an organic solvent as a reaction solvent, preferably, the organic solvent is isopropanol.

In some embodiments of the present invention, in the method for preparing the compound represented by Formula V, the reaction temperature is 50°C to 90°C, preferably 60°C to 80°C, 60°C to 70°C, 70°C to 80°C, or 65°C to 75°C.

In some embodiments of the present invention, in the method for preparing the compound represented by Formula V, the reaction time is at least 0.5 hours, at least 1 hour, at least 1.5 hours, at least 2 hours, 1 to 10 hours, 2 to 8 hours, 2 to 6 hours, 2 to 4 hours, 2 to 3 hours, 0.5 hours, 1 hour, 1.5 hours, 2 hours, 3 hours, 4 hours, 5 hours, 6 hours, 7 hours, 8 hours, 9 hours or 10 hours.

Another aspect of the present invention relates to a use of the compound represented by formula V or pharmaceutically acceptable salt or ester thereof in the manufacture of pranoprofen or a pharmaceutically acceptable salt or ester of pranoprofen. Preferably, the compound represented by Formula V is prepared by the method for preparing the compound represented by Formula V according to any one of items of the present invention.

Some intermediate compounds involved in the present invention are shown in Table A below.

**Table A: Some intermediate compounds involved in the present invention**

| | General structural formula | Compound name | Compound structure formula | Compound No. |
|---|---|---|---|---|
| Compound represented by Formula I | 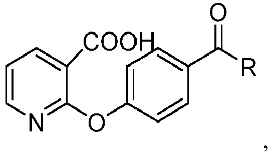 R is C₁-C₁₀ straight chain or branched chain alkyl, preferably, R is C₂-C₆ straight chain or branched chain alkyl, more preferably, R is C₂-C₄ straight chain or branched chain alkyl, such as ethyl, propyl, isopropyl or n-butyl. | 2-[4-(1-Oxopropyl)phenoxy]-3-pyridinecarboxylic acid | 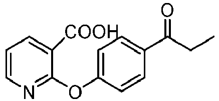 | I-A |
| | | 2-[4-(1-Oxobutyl)phenoxy]-3-pyridinecarboxylic acid | 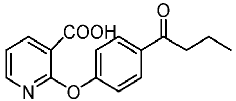 | I-B |
| | | 2-[4-(1-Oxopentyl)phenoxy]-3-pyridinecarboxylic acid | 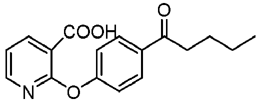 | I-C |
| Compound represented by Formula II | 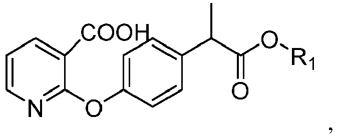 wherein, R₁ is C₁-C₁₀ straight chain or branched chain alkyl, preferably, R₁ is C₁-C₅ straight chain or branched chain alkyl, more preferably, R₁ is C₁-C₃ straight chain or branched chain alkyl, such as methyl, ethyl, propyl or isopropyl. | 2-[4-(2-Methoxy-1-methyl-2-oxoethyl) phenoxy]-3-pyridinecarboxylic acid | 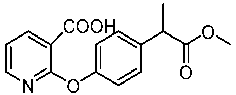 | II-A |
| | | 2-[4-(2-Ethoxy-1-methyl-2-oxoethyl) phenoxy]-3-pyridinecarboxylic acid | 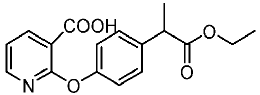 | II-B |
| | | 2-[4-(2-Propoxy-1-methyl-2-oxoethyl) phenoxy]-3-pyridinecarboxylic acid | 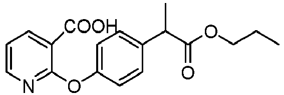 | II-C |
| Compound represented by Formula III | 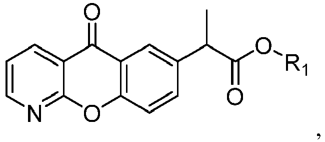 wherein, R₁ is C₁-C₁₀ straight chain or branched chain alkyl, preferably, Ri is C₁-C₅ straight chain or | Methyl 2-(10-oxo-9-oxa-1-azaanthracen-6-yl)propionate | 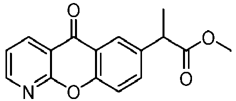 | III-A |
| | | Ethyl 2-(10-oxo-9-oxa-1-azaanthracen-6-yl)propionate | 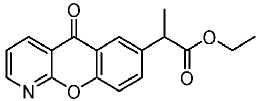 | III-B |
| | branched chain alkyl, more preferably, R₁ is C₁-C₃ straight chain or branched chain alkyl, such as methyl, ethyl, propyl or isopropyl. | Propyl 2-(10-oxo-9-oxa-1-azaanthracen-6-yl)propionate | 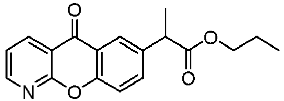 | III-C |
| Compound represented by Formula IV | 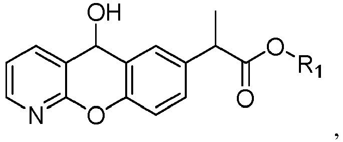 Wherein, R₁ is C₁-C₁₀ straight chain or branched chain alkyl, preferably, R₁ is C₁-C₅ straight chain or branched chain alkyl, more preferably, Ri is C₁-C₃ straight chain or branched chain alkyl, such as methyl, ethyl, propyl or isopropyl. | Methyl 2-(10-hydroxy-9-oxa-1-azaanthracen-6-yl)propionate | 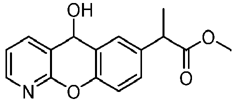 | IV-A |
| | | Ethyl 2-(10-hydroxy-9-oxa-1-azaanthracen-6-yl)propionate | 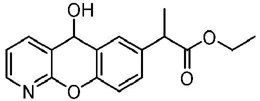 | IV-B |
| | | Propyl 2-(10-hydroxy-9-oxa-1-azaanthracen-6-yl)propionate | 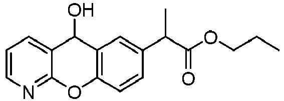 | IV-C |

(VI)
A composition comprising pranoprofen and an impurity, wherein, calculated by mass percentage, the impurity in the composition has a content of less than or equal to 0.5%, less than or equal to 0.4%, less than or equal to 0.3%, less than or equal to 0.25%, less than or equal to 0.24%, less than or equal to 0.23%, less than or equal to 0.22%, less than or equal to 0.21%, less than or equal to 0.20%, less than or equal to 0.19%, less than or equal to 0.18%, less than or equal to 0.17 %, less than or equal to 0.16%, less than or equal to 0.15%, less than or equal to 0.14%, less than or equal to 0.13%, less than or equal to 0.12%, less than or equal to 0.11%, less than or equal to 0.10%, less than or equal to 0.09%, less than or equal to 0.08%, less than or equal to 0.07%, less than or equal to 0.06%, less than or equal to 0.05%, less than or equal to 0.04%, less than or equal to 0.03%, less than or equal to 0.02%, less than or equal to 0.01%, or less than or equal to 0.005%.

In one or more embodiments of the present invention, in the composition, calculated by mass percentage, the pranoprofen in the composition has a content (purity) of greater than or equal to 99%, greater than or equal to 99.1%, greater than or equal to 99.2%, greater than or equal to 99.3%, greater than or equal to 99.4%, greater than or equal to 99.5%, greater than or equal to 99.6%, greater than or equal to 99.7%, greater than or equal to 99.8%, greater than or equal to 99.9%, greater than or equal to 99.91%, greater than or equal to 99.92%, greater than or equal to 99.93%, greater than or equal to 99.94%, greater than or equal to 99.95%, greater than or equal to 99.96%, greater than or equal to 99.97%, greater than or equal to 99.98%, or greater than or equal to 99.99%.

In one or more embodiments of the present invention, in the composition, the impurity has content of greater than zero.

In one or more embodiments of the present invention, the composition consists of pranoprofen and impurities.

In one or more embodiments of the present invention, in the composition, the impurity is total of impurities.

In one or more embodiments of the present invention, in the composition, the impurity is the maximum single impurity.

In one or more embodiments of the present invention, in the composition, the impurity comprises any one, any two, any three or any four selected from the group consisting of impurity A, impurity C, impurity D, and impurity L.

In one or more embodiments of the present invention, in the composition, the impurity consists of any one, any two, any three or any four selected from the group consisting of impurity A, impurity C, impurity D, and impurity L.

In one or more embodiments of the present invention, in the composition, the impurity is impurity A, impurity C, impurity D or impurity L.

In one or more embodiments of the present invention, in the composition, the impurity is impurity A.

In one or more embodiments of the present invention, in the composition, the impurity is impurity C.

In one or more embodiments of the present invention, in the composition, the impurity is impurity D.

In one or more embodiments of the present invention, in the composition, the impurity is impurity L.

In one or more embodiments of the present invention, in the composition, the impurities are impurity A and impurity C.

In one or more embodiments of the present invention, in the composition, the impurity consists of impurity A and impurity D.

In one or more embodiments of the present invention, in the composition, the impurity consists of impurity A and impurity L.

In one or more embodiments of the present invention, in the composition, the impurity consists of impurity C and impurity D.

In one or more embodiments of the present invention, in the composition, the impurity consists of impurity C and impurity L.

In one or more embodiments of the present invention, in the composition, the impurity consists of impurity D and impurity L.

In one or more embodiments of the present invention, in the composition, the impurity consists of impurity A, impurity C and impurity D.

In one or more embodiments of the present invention, in the composition, the impurity consists of impurity A, impurity C and impurity L.

In one or more embodiments of the present invention, in the composition, the impurity consists of impurity A, impurity D and impurity L.

In one or more embodiments of the present invention, in the composition, the impurity consists of impurity C, impurity D and impurity L.

In one or more embodiments of the present invention, in the composition, the impurity consists of impurity A, impurity C, impurity D and impurity L.

In one or more embodiments of the present invention, in the composition, the impurity comprise impurity A, impurity C, impurity D and impurity L.

In one or more embodiments of the present invention, in the composition, the total impurity content is less than or equal to 0.2%, less than or equal to 0.15%, less than or equal to 0.12%, less than or equal to 0.1%, less than or equal to 0.09%, less than or equal to 0.08%, less than or equal to 0.07%, less than or equal to 0.06%, less than or equal to 0.05%, less than or equal to 0.04%, less than or equal to 0.03%, less than or equal to 0.02%, or less than or equal to 0.01%.

In one or more embodiments of the present invention, in the composition, the content of impurity A is less than or equal to 0.2%, less than or equal to 0.15%, less than or equal to 0.12%, less than or equal to 0.1%, less than or equal to 0.09%, less than or equal to 0.08%, less than or equal to 0.07%, less than or equal to 0.06%, less than or equal to 0.05%, less than or equal to 0.04%, less than or equal to 0.03%, less than or equal to 0.02%, or less than or equal to 0.01%.

In one or more embodiments of the present invention, in the composition, the content of impurity C is less than or equal to 0.2%, less than or equal to 0.15%, less than or equal to 0.12%, less than or equal to 0.1%, less than or equal to 0.09%, less than or equal to 0.08%, less than or equal to 0.07%, less than or equal to 0.06%, less than or equal to 0.05%, less than or equal to 0.04%, less than or equal to 0.03%, less than or equal to 0.02%, less than or equal to 0.01%, or less than or equal to 0.005%.

In one or more embodiments of the present invention, in the composition, the content of impurity D is less than or equal to 0.2%, less than or equal to 0.15%, less than or equal to 0.12%, less than or equal to 0.1%, less than or equal to 0.09%, less than or equal to 0.08%, less than or equal to 0.07%, less than or equal to 0.06%, less than or equal to 0.05%, less than or equal to 0.04%, less than or equal to 0.03%, less than or equal to 0.02%, less than or equal to 0.01 %, or less than or equal to 0.005%.

In one or more embodiments of the present invention, in the composition, the content of impurity L is less than or equal to 0.2%, less than or equal to 0.15%, less than or equal to 0.12%, less than or equal to 0.1%, less than or equal to 0.09%, less than or equal to 0.08%, less than or equal to 0.07%, less than or equal to 0.06%, less than or equal to 0.05%, less than or equal to 0.04%, less than or equal to 0.03%, less than or equal to 0.02%, less than or equal to 0.01%, or less than or equal to 0.005%.

In one or more embodiments of the present invention, in the composition, after the accelerated experiment for 1 month, 2 months or 3 months, compared with 0 month, the change (increase or decrease) of the content of total impurities, maximum single impurity, impurity A, impurity C, impurity D and/or impurity L does not exceed 50%, 40%, 30%, 20%, 10% or 5%.

In one or more embodiments of the present invention, in the composition, after the accelerated experiment for 6 months, compared with 0 month, the change (increase or decrease) of the content of total impurities, maximum single impurity, impurity A, impurity C, impurity D and/or impurity L does not exceed 300%, 250%, 200%, 150%, 130%, 120%, 110%, 100%, 90%, 80%, 70%, 60%, 50%, 40%, 30%, 20%, 10%, or 5%.

In one or more embodiments of the present invention, in the composition, after the accelerated experiment for 1 month, 2 months or 3 months, the content of the total impurities, maximum single impurity, impurity A, impurity C, impurity D and/or impurity L is less than or equal to 0.2%, less than or equal to 0.15%, less than or equal to 0.12%, less than or equal to 0.1%, less than or equal to 0.09%, less than or equal to 0.08%, less than or equal to 0.07%, 0.06% or less, 0.05% or less, 0.04% or less, 0.03% or less, 0.02% or less, or 0.01% or less.

In one or more embodiments of the present invention, in the composition, after the accelerated experimentation for 6 months, the content of total impurities, maximum single impurity, impurity A, impurity C, impurity D and/or impurity L is less than or equal to 0.5%, less than or equal to 0.45%, less than or equal to 0.4%, less than or equal to 0.35%, less than or equal to 0.3%, less than or equal to 0.25%, less than or equal to 0.2%, less than or equal to 0.15%, less than or equal to 0.12%, less than or equal to 0.1%, less than or equal to 0.09%, less than or equal to 0.08%, less than or equal to 0.07%, less than or equal to 0.06%, less than or equal to 0.05%, less than or equal to 0.04%, less than or equal to 0.03%, less than or equal to 0.02%, or less than or equal to 0.01%.

The structures of impurity A, impurity C, impurity D, and impurity L are shown in Table B below.

**Table B: Some impurity compounds involved in the present invention**

| - | Impurity A | Impurity C | Impurity D | Impurity L |
|---|---|---|---|---|
| Structural formula | 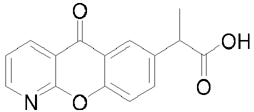 | 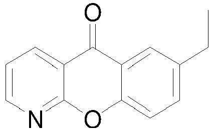 | 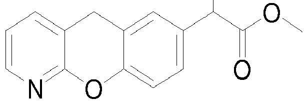 | 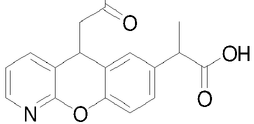 |
| Chinese name of impurity compound | 2-(10- 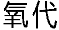 -9- 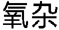 -1- 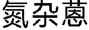 -6 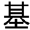 )- 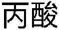 | 6- 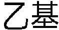 -10- 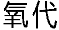 -9- 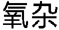 -1- 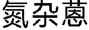 | 2-(10H-9- 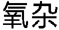 -1- 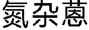 -6- 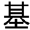 ) 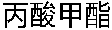 | 2-[10-(2- 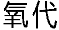 - 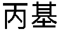 )-10H- 9- 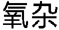 - 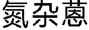 -6- 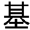 ]- 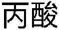 |
| English name of impurity compound | 2-(10-Oxo-9-oxa-1-azaanthrac en-6-yl)propanoic acid | 6-Ethyl-9-oxa-1-aza-anthrace n-10-one | 2-(10H-9-Oxa-1-aza-anthrace n-6-yl)-propionic acid methyl ester | 2-[10-(2-Oxo-propyl)-10H-9-oxa-1-aza-anthracen-6-yl]-pro pionic acid |

Another aspect of the present invention relates to a pharmaceutical composition, which comprises the compound according to any one of items described in the present invention above, and one or more pharmaceutically acceptable excipients.

In the present invention, unless otherwise specified, the composition is a pranoprofen product, which contains a dominant amount of the pranoprofen compound and one or more impurities. Those skilled in the art can understand that the existence or generation of impurities is unavoidable during the synthesis, purification and/or storage of the compound.

In the present invention, unless otherwise specified, the pranoprofen refers to a pure pranoprofen.

In the present invention, unless otherwise specified, the accelerated experiment is carried out under the conditions of 40°C±2°C and RH75%±5% in accordance with the regulations in the "Technical Guidelines for Stability Research of Chemical Drugs"; preferably, an average value of 3 samples measured in parallel is taken.

In the present invention, unless otherwise specified, the content of each impurity or total impurities is measured by high performance liquid chromatography. Preferably, the chromatographic conditions comprise: using octadecylsilane-bonded silica gel as filler (5µm, 250mm×4.6mm); using 0.05mol/L sodium perchlorate aqueous solution-methanol (90:10, adding perchloric acid to adjust pH to 3.2) as mobile phase A, methanol as mobile phase B, following the gradient elution in Table 10, flow rate of 1.0ml/min, column temperature of 40°C, detection wavelength of 275nm. The elution program is also shown in Table 10.

In the present invention, unless otherwise specified, the content of pranoprofen, impurities, total impurities, maximum single impurity, impurity A, impurity C, impurity D or impurity L refers to the content calculated according to mass percentage in the composition.

Another aspect of the present invention relates to a method for quality inspection or quality control of a pranoprofen product, comprising a step of detecting the content of (pure) pranoprofen or the content of impurities therein; preferably, the pranoprofen product is the composition or pharmaceutical preparation according any one of items of the present invention. Preferably, the method is a high performance liquid chromatography. Preferably, the chromatographic conditions comprise: using octadecylsilane-bonded silica gel as filler (5µm, 250mm×4.6mm); using 0.05mol/L sodium perchlorate aqueous solution-methanol (90:10, adding perchloric acid to adjust pH to 3.2) as mobile phase A, methanol as mobile phase B, following the gradient elution in Table 10, flow rate of 1.0ml/min, column temperature of 40°C, detection wavelength of 275nm. The elution program is also shown in Table 10.

Another aspect of the present invention relates to a use of any one, any two, any three or any four selected from the group consisting of impurity A, impurity C, impurity D and impurity L in the quality inspection or quality control of a pranoprofen product; preferably, the pranoprofen product is the composition or pharmaceutical preparation described in any one of items of the foregoing present inventions.

Another aspect of the present invention relates to a use of the following impurities or combinations of impurities in the quality inspection or quality control of a pranoprofen product; preferably, the pranoprofen product is the composition or pharmaceutical preparation described in any one of items of the foregoing present inventions:
Impurity L,
Impurity L and Impurity A,
Impurity L and Impurity C,
Impurity L and Impurity D,
Impurity L, Impurity A and Impurity C,
Impurity L, Impurity A and Impurity D,
Impurity L, Impurity C and Impurity D,
   or
Impurity L, Impurity A, Impurity C and Impurity D.

In one or more embodiments of the present invention, in the method for preparing the compound represented by Formula I, the compound represented by Formula II, the compound represented by Formula III, the compound represented by Formula IV or pranoprofen, the preparation method does not use highly toxic chemical reagents such as potassium cyanide, iodomethane. Therefore, the preparation method of the present invention reduces production hazards and risks in worker operation.

In one or more embodiments of the present invention, in the method for preparing the compound represented by Formula I, the compound represented by Formula II, the compound represented by Formula III, the compound represented by Formula IV or pranoprofen, the preparation method does not use phosphorus-containing chemical reagents such as polyphosphoric acid. Therefore, the preparation method of the present invention reduces environmental pollution and is more friendly to the environment.

In the present invention, the term "C₁₋₁₀ alkyl" refers to a straight-chain or branched-chain alkyl having 1 to 10 carbon atoms, such as methyl, ethyl, propyl, isopropyl, n-butyl, sec-butyl, tert-butyl, pentoxy, 2-pentoxy, isopentoxy, neo-pentoxy, hexyloxy, 2-hexyloxy, 3-hexyloxy, etc.; C₂₋₁₀ alkyl, C₁₋₆ alkyl, C₂₋₆ alkyl, C₁₋₃ alkyl or C₂₋₄ alkyl, and the like, preferably, C₁₋₆ alkyl, C₂₋₆ alkyl, C₁₋₃ alkyl or C₂₋₄ alkyl.

### Beneficial effects of the present invention

The present invention achieves any one or more of the following technical effects (1) to (11):
(1) the raw materials used in the present invention are easier to get;
(2) it is beneficial to continuously remove impurities in the process and improve product quality;
(3) it avoids the use of highly toxic chemicals such as potassium cyanide and iodomethane, reducing production hazards and worker operational risks;
(4) it avoids the use of phosphorus-containing chemicals such as polyphosphoric acid, reducing environmental pollution and being more environmentally friendly;
(5) the reaction steps are short and the process route is simplified;
(6) the reaction conditions are mild;
(7) it is easy to operate;
(8) it is more suitable for industrial production;
(9) the yield of the compound represented by Formula 0, Formula I, Formula II, Formula III and/or Formula IV prepared by the present invention is relatively high;
(10) the purity of the compound represented by Formula 0, Formula I, Formula II, Formula III and/or Formula IV prepared by the present invention is relatively high;
(11) it achieves a good balance between yield and purity for the compound represented by Formula 0, Formula I, Formula II, Formula III and/or Formula IV prepared by the present invention.

### Brief Description of the Drawings

Figure 1 shows the high-resolution mass spectrum of the sample.
Figure 2A shows the infrared spectrum of the standard product.
Figure 2B shows the infrared spectrum of the sample.
Figure 3A shows the UV spectrum of the neutral standard product.
Figure 3B shows the UV spectrum of the acidic standard product.
Figure 3C shows the UV spectrum of the basic standard product.
Figure 3D shows the UV spectrum of the neutral sample.
Figure 3E shows the UV spectrum of the acidic sample.
Figure 3F shows the UV spectrum of the alkaline sample.

### Specific Models for Carrying Out the present invention

Embodiments of the present invention will be described in detail below in conjunction with examples, but those skilled in the art will understand that the following examples are only for illustrating the present invention, and should not be considered as limiting the scope of the present invention. For those without giving specific conditions in the examples, they were carried out according to the conventional conditions or conditions recommended by the manufacturer. For those reagents or instruments without giving manufacturers, they were all commercially available conventional products.

### Preparation Examples 1a to 1i: Preparation of 2-[4-(1-oxopropyl)phenoxy]-3-pyridinecarboxylic acid (Compound I-A) or 2-[4-(1-oxobutyl)phenoxy]-3-pyridinecarboxylic acid Compound I-B)

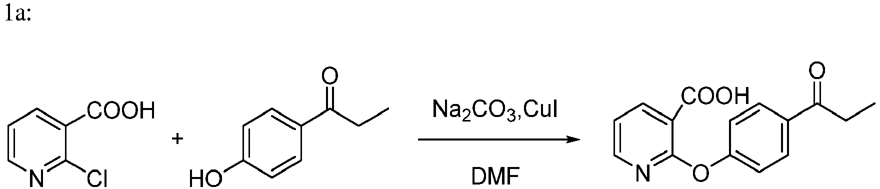

2-Chloronicotinic acid (20.0g, 1equiv.), *N,N*-dimethylformamide (20ml), cuprous iodide (2.4g, 0.1equiv.) were added into a 250mL reaction bottle, stirred and mixed, and heated to 75°C to 80°C, sodium carbonate (26.9g, 2equiv.) and 4-hydroxypropiophenone (38.1g, 2equiv.) were added in batches, after the addition was completed, the temperature was raised to 110°C to 115°C, and the reaction was carried out by keeping the temperature for 5h. The reaction liquid was cooled to 50°C, added with 100g of water, and stirred for 15min. After being acidified to pH 3-4, it was filtered, and the filter cake obtained was transferred into a 1L beaker, added with 200g of water, stirred evenly, adjusted to pH 7-7.5 with sodium bicarbonate and filtered, the filter cake was washed with appropriate amount of water, the filtrate was retained, and transferred to a clean 1L beaker. The obtained filtrate was acidified to pH 3-4 to precipitate a product. After being filtered, the obtained filter cake was air-dried at 60°C to obtain 29.5g of Compound **I-A** with a yield of 85.6%, and a purity of 97.53%.

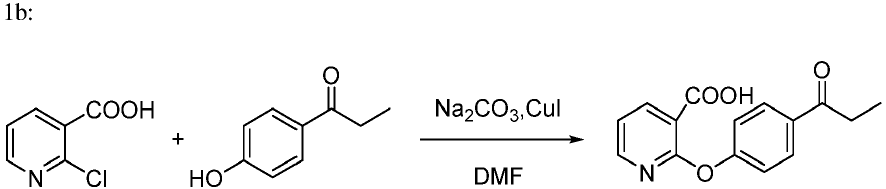

2-Chloronicotinic acid (20.0g, 1equiv.), *N,N*-dimethylformamide (20ml), cuprous iodide (1.2g, 0.05equiv.) were added into a 250ml reaction bottle, stirred and mixed, and heated to 75°C to 80°C, and sodium carbonate (26.9g, 2equiv.) and 4-hydroxypropiophenone (38.1g, 2equiv.) were added in batches, after the addition was completed, the temperature was raised to 110°C to 115°C, and the reaction was carried out by keeping the temperature for 5h. The reaction liquid was cooled to 50°C, added with 100g of water, and stirred for 15min. After being acidified to pH 3-4, it was filtrated, and the filter cake obtained was transferred into a 1L beaker, added with 200g of water, stirred evenly, adjusted to pH 7-7.5 with sodium bicarbonate and filtered, the obtained filter cake was washed with appropriate amount of water, the filtrate was retained, and transferred to a clean 1L beaker. The obtained filtrate was acidified to pH 3-4 to precipitate a product. After being filtered, the obtained filter cake was air-dried at 60°C to obtain 29.4 g of Compound **I-A** with a yield of 85.5% and a purity of 97.36%.

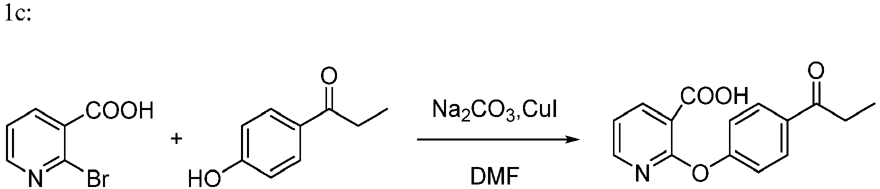

2-Bromonicotinic acid (20.0g, 1equiv.), *N,N*-dimethylformamide (20ml), cuprous iodide (1.9g, 0.1equiv.) were added into a 250mL reaction bottle, stirred and mixed, and heated to 75°C to 80°C, and sodium carbonate (21.0g, 2equiv.) and 4-hydroxypropiophenone (29.7g, 2equiv.) were added in batches, after the addition was completed, the temperature was raised to 110°C to 115°C, and the reaction was carried out by keeping the temperature for 5h. The reaction liquid was cooled to 50°C, added with 100g of water, and stirred for 15min. After being acidified to pH 3-4 and filtered, the obtained filter cake was transferred into a 1L beaker, added with 200g of water, stirred evenly, adjusted to pH 7-7.5 with sodium bicarbonate and filtered, the obtained filter cake was washed with appropriate amount of water, the filtrate was retained, and transferred to a clean 1L beaker. The obtained filtrate was acidified to pH 3-4 to precipitate a product. After filtration, the obtained filter cake was air-dried at 60°C to obtain 23.1 g of Compound **I-A** with a yield of 86.0% and a purity of 97.68%.

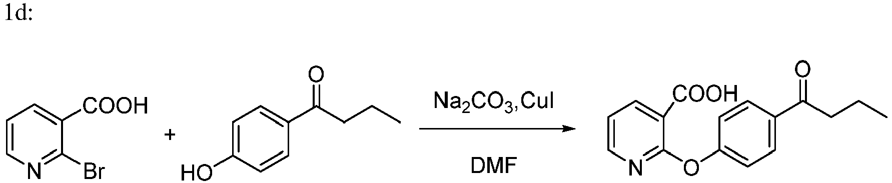

2-Bromonicotinic acid (50.0g, 1equiv.), *N,N*-dimethylformazine (50ml), cuprous iodide (4.7g, 0.1equiv.) were added into a 500ml reaction bottle, stirred and mixed, and heated to 75°C to 80°C, and sodium carbonate (52.5g, 2equiv.) and 4-hydroxybutyrophenone (81.3g, 2equiv.) were added in batches, after the addition was completed, the temperature was raised to 110°C to 115°C, and the reaction was carried out by keeping the temperature for 5h. The reaction liquid was cooled to 50°C, added with 250g of water, and stirred for 15min. After being acidified to pH 3-4 and filtered, the obtained filter cake was transferred into a 2L beaker, added with 500g of water, stirred evenly, adjusted to pH 7-7.5 with sodium bicarbonate and filtered, the obtained filter cake was washed with appropriate amount of water, the filtrate was retained and transferred to a clean 2L beaker. The resulting filtrate was acidified to pH 3-4 to precipitate a product. After being filtered, the obtained filter cake was air-dried at 60°C to obtain 58.4g of Compound **I-B** with a yield of 83.0% and a purity of 97.11%.

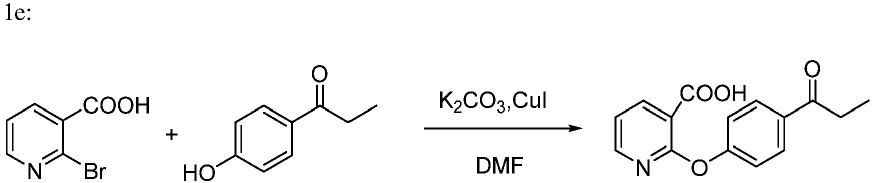

2-Bromonicotinic acid (20.0g, 1equiv.), *N,N*-dimethyl formazan (20.0g), cuprous iodide (1.9g, 0.1equiv.) were added into a 250ml reaction bottle, stirred and mixed, and heated to 75°C to 80°C, and potassium carbonate (27.4g, 2equiv.) and 4-hydroxypropiophenone (29.7g, 2equiv.) were added in batches, after the addition was completed, the temperature was raised to 110°C to 115°C, and the reaction was carried out by keeping the temperature for 5h. The reaction liquid was cooled to 50°C, added with 100g of water, and stirred for 15min. After being acidified to pH 3-4, the filter cake obtained by filtration was transferred into a 1L beaker, added with 200g of water, stirred evenly, adjusted to pH 7-7.5 with sodium bicarbonate and filtered, the obtained filter cake was washed with appropriate amount of water, the filtrate was retained, and transfered to a clean 1L beaker. The resulting filtrate was acidified to pH 3-4 to precipitate a product. After being filtered, the obtained filter cake was air-dried at 60°C to obtain 22.9 g of Compound **I-A** with a yield of 85.0% and a purity of 97.44%.

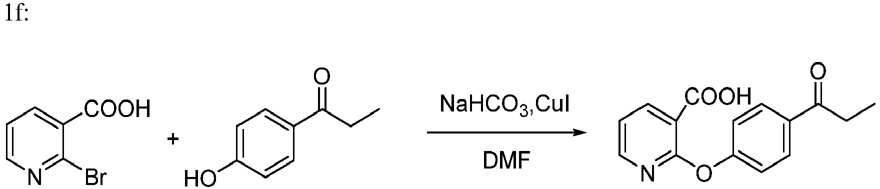

2-Bromonicotinic acid (20.0g, 1equiv.), *N,N*-dimethyl formazan (20.0g), cuprous iodide (1.9g, 0.1equiv.) were added into a 250ml reaction bottle, stirred and mixed, and heated to 75°C to 80°C, and sodium bicarbonate (16.6g, 2equiv.) and 4-hydroxypropiophenone (29.7g, 2equiv.) were added in batches, after the addition was completed, the temperature was raised to 110°C to 115°C, and the reaction was carried out by keeping the temperature for 5h. The reaction liquid was cooled to 50°C, added with 100g of water, and stirred for 15min. After being acidified to pH 3-4, the filter cake obtained by filtration was transferred into a 1L beaker, added with 200g of water, stirred evenly, adjusted to pH 7-7.5 with sodium bicarbonate and filtered, the filter cake was washed with appropriate amount of water, the filtrate was retained, and transfered to a clean 1L beaker. The resulting filtrate was acidified to pH 3-4 to precipitate a product. After filtration, the obtained filter cake was air-dried at 60°C to obtain 21.6 g of Compound **I-A** with a yield of 80.6% and a purity of 96.87%.

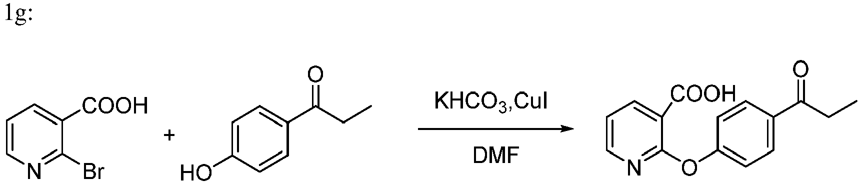

2-Bromonicotinic acid (20.0g, 1equiv.), *N,N*-dimethyl formazan (20.0g), cuprous iodide (1.9g, 0.1equiv.) were added into a 250ml reaction bottle, stirred and mixed, and heated to 75°C to 80°C, and potassium bicarbonate (19.8g, 2equiv.) and 4-hydroxypropiophenone (29.7g, 2equiv.) were added in batches, after the addition was completed, the temperature was raised to 110°C to 115°C, and the reaction was carried out by keeping the temperature for 5h. The reaction liquid was cooled to 50°C, added with 100g of water, and stirred for 15min. After being acidified to pH 3-4, the filter cake obtained by filtration was transferred into a 1L beaker, added with 200g of water, stirred evenly, adjusted to pH 7-7.5 with sodium bicarbonate and filtered, the filter cake was washed with appropriate amount of water, the filtrate was retained, and transferred to a clean 1L beaker. The resulting filtrate was acidified to pH 3-4 to precipitate a product. After filtration, the obtained filter cake was air-dried at 60°C to obtain 21.5 g of Compound **I-A** with a yield of 80.2% and a purity of 96.56%.

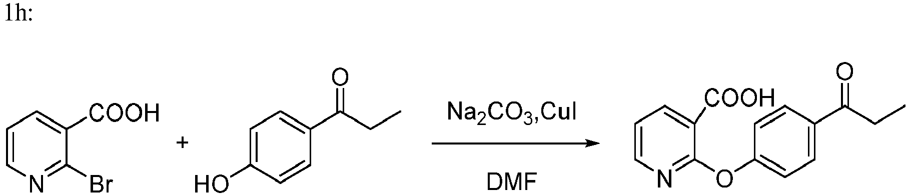

2-Bromonicotinic acid (20.0g, 1equiv.), *N,N*-dimethylformamide (20ml), cuprous iodide (1.9g, 0.1equiv.) were added into a 250mL reaction bottle, stirred and mixed, and heated to 60°C to 65°C, and sodium carbonate (21.0g, 2equiv.) and 4-hydroxypropiophenone (29.7g, 2equiv.) were added in batches, after the addition was completed, the temperature was raised to 75°C to 80°C, and the reaction was carried out by keeping the temperature for 10h. The reaction liquid was cooled to 50°C, added with 100g of water, and stirred for 15min. After being acidified to pH 3-4, the filter cake obtained by filtration was transferred into a 1L beaker, added with 200g of water, stirred evenly, adjusted to pH 7-7.5 with sodium bicarbonate and filtered, the filter cake was washed with appropriate amount of water, the filtrate was retained, and transferred to a clean 1L beaker. The resulting filtrate was acidified to pH 3-4 to precipitate a product. After filtration, the obtained filter cake was air-dried at 60°C to obtain 22.4 g of Compound **I-A** with a yield of 83.4% and a purity of 96.48%.

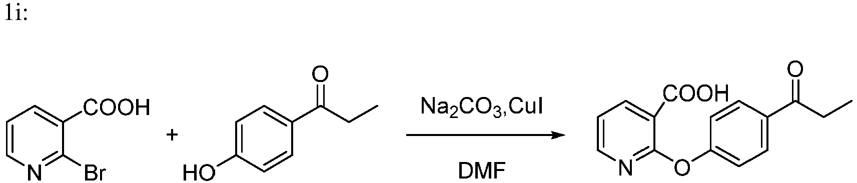

2-Bromonicotinic acid (20.0g, 1equiv.), *N,N*-dimethylformamide (20ml), cuprous iodide (1.9g, 0.1equiv.) were added into a 250mL reaction bottle, stirred and mixed, and heated to 75°C to 80°C, and sodium carbonate (21.0g, 2equiv.) and 4-hydroxypropiophenone (29.7g, 2equiv.) were added in batches, after the addition was completed, the temperature was raised to 120°C to 125°C, and the reaction was carried out by keeping the temperature for 4h. The reaction liquid was cooled to 50°C, added with 100g of water, and stirred for 15min. After being acidified to pH 3-4, the filter cake obtained by filtration was transferred into a 1L beaker, added with 200g of water, stirred evenly, adjusted to pH 7-7.5 with sodium bicarbonate and filtered, the filter cake was washed with appropriate amount of water, the filtrate was retained, and transferred to a clean 1L beaker. The resulting filtrate was acidified to pH 3-4 to precipitate a product. After filtration, the obtained filter cake was air-dried at 60°C to obtain 23.4 g of Compound I-A with a yield of 87.0% and a purity of 96.11%.

### Comparative Examples 1j to 1w

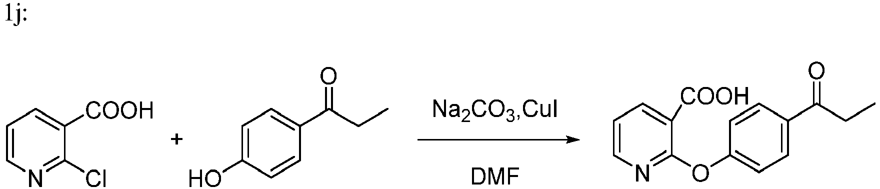

2-Chloronicotinic acid (20.0g, 1equiv.), *N,N*-dimethylformamide (20ml), cuprous iodide (4.8g, 0.2equiv.) were added into a 250mL reaction bottle, stir and mix, and heat up to 75°C to 80°C, and sodium carbonate (13.5g, 1 equiv.) and 4-hydroxypropiophenone (19.1g, 1equiv.) were added in batches, after the addition was completed, the temperature was raised to 110°C to 115°C, and the reaction was carried out by keeping the temperature for 5h. The reaction liquid was cooled to 50°C, added with 100g of water, and stirred for 15min. After being acidified to pH 3-4, the filter cake obtained by filtration was transferred into a 1L beaker, added with 200g of water, stirred evenly, adjusted to pH 7-7.5 with sodium bicarbonate and filtered, the filter cake was washed with appropriate amount of water, the filtrate was retained, and transferred to a clean 1L beaker. The resulting filtrate was acidified to pH 3-4 to precipitate a product. After filtration, the resulting filter cake was air-dried at 60°C to obtain 25.6 g of Compound **I-A** with a yield of 74.2%, and a purity of 93.88%.

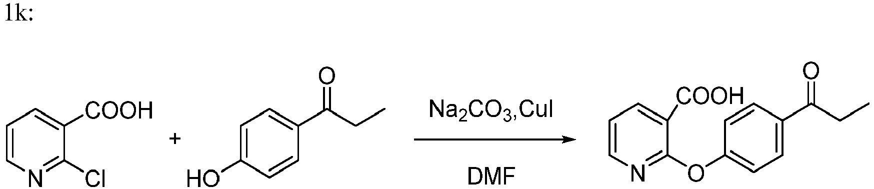

2-Chloronicotinic acid (20.0g, 1equiv.), *N,N*-dimethylformamide (20ml), cuprous iodide (4.8g, 0.2equiv.) were added into a 250mL reaction bottle, stirred and mixed, and heated to 75°C to 80°C, and sodium carbonate (13.5g, 1equiv.) and 4-hydroxypropiophenone (38.1g, 2equiv.) were added in batches, after the addition was completed, the temperature was raised to 110°C to 115°C, and the reaction was carried out by keeping the temperature for 5h. The reaction liquid was cooled to 50°C, added with 100g of water, and stirred for 15min. After being acidified to pH 3-4, the filter cake obtained by filtration was transferred into a 1L beaker, added with 200g of water, stirred evenly, adjusted to pH 7-7.5 with sodium bicarbonate and filtered, the filter cake was washed with appropriate amount of water, the filtrate was retained, and transferred to a clean 1L beaker. The resulting filtrate was acidified to pH 3-4 to precipitate a product. After filtration, the resulting filter cake was air-dried at 60°C to obtain 27.2 g of Compound **I-A** with a yield of 79.1% and a purity of 96.43%.

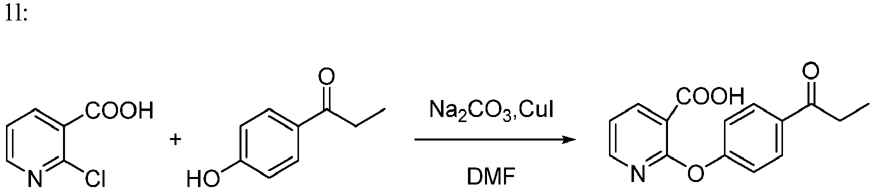

2-Chloronicotinic acid (20.0g, 1equiv.), *N,N*-dimethylformamide (20ml), cuprous iodide (4.8g, 0.2equiv.) were added into a 250mL reaction bottle, stirred and mixed, and heated to 75°C to 80°C, and sodium carbonate (13.5g, 1equiv.) and 4-hydroxypropiophenone (57.2g, 3equiv.) were added in batches, after the addition was completed, the temperature was raised to 110°C to 115°C, and the reaction was carried out by keeping the temperature for 5h. The reaction liquid was cooled to 50°C, added with 100g of water, and stirred for 15min. After being acidified to pH 3-4, the filter cake obtained by filtration was transferred into a 1L beaker, added with 200g of water, stirred evenly, adjusted to pH 7-7.5 with sodium bicarbonate and filtered, the filter cake was washed with appropriate amount of water, the filtrate was retained, and transferred to a clean 1L beaker. The resulting filtrate was acidified to pH 3-4 to precipitate a product. After filtration, the obtained filter cake was air-dried at 60°C to obtain 27.1 g of Compound **I-A** with a yield of 78.8% and a purity of 96.57%.

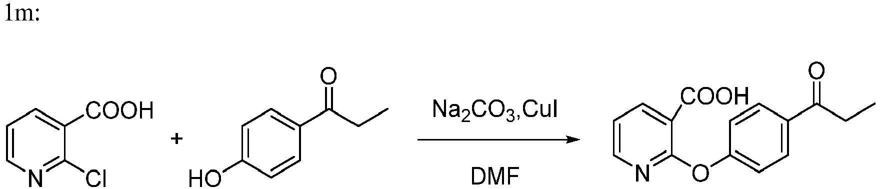

2-Chloronicotinic acid (20.0g, 1equiv.), *N,N*-dimethylformamide (20ml), cuprous iodide (4.8g, 0.2equiv.) were added into a 250mL reaction bottle, stirred and mixed, and heated to 75°C to 80°C, and sodium carbonate (13.5g, 1equiv.) and 4-hydroxypropiophenone (76.3g, 4equiv.) were added in batches, after the addition was completed, the temperature was raised to 110°C to 115°C, and the reaction was carried out by keeping the temperature for 5h. The reaction liquid was cooled to 50°C, added with 100g of water, and stirred for 15min. After being acidified to pH 3-4, the filter cake obtained by filtration was transferred into a 1L beaker, added with 200g of water, stirred evenly, adjusted to pH 7-7.5 with sodium bicarbonate and filtered, the filter cake was washed with appropriate amount of water, the filtrate was retained, and transferred to a clean 1L beaker. The resulting filtrate was acidified to pH 3-4 to precipitate a product. After filtration, the obtained filter cake was air-dried at 60°C to obtain 27.4 g of Compound **I-A** with a yield of 79.7%, and a purity of 95.29%.

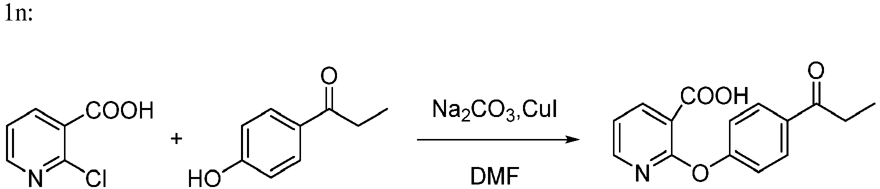

2-Chloronicotinic acid (20.0g, 1equiv.), *N,N*-dimethylformamide (20ml), cuprous iodide (4.8g, 0.2equiv.) were added into a 250mL reaction bottle, stirred and mixed, and heated to 75°C to 80°C, and sodium carbonate (13.5g, 1equiv.) and 4-hydroxypropiophenone (95.3g, 5equiv.) were added in batches, after the addition was completed, the temperature was raised to 110°C to 115°C, and the reaction was carried out by keeping the temperature for 5h. The reaction liquid was cooled to 50°C, transferred to a 500ml beaker, added with 100g of water, and stirred for 15min. After being acidified to pH 3-4, the filter cake obtained by filtration was transferred into a 1L beaker, added with 200g of water, stirred evenly, adjusted to pH 7-7.5 with sodium bicarbonate and filtered, the filter cake was washed with appropriate amount of water, the filtrate was retained, and transferred to a clean 1L beaker. The resulting filtrate was acidified to pH 3-4 to precipitate a product. After filtration, the obtained filter cake was air-dried at 60°C to obtain 27.2 g of Compound **I-A** with a yield of 78.9% and a purity of 95.11%.

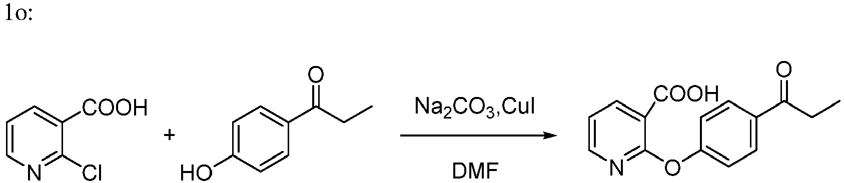

2-Chloronicotinic acid (20.0g, 1equiv.), *N,N*-dimethylformamide (20ml), cuprous iodide (4.8g, 0.2equiv.) were added into a 250mL reaction bottle, stirred and mixed, and heated to 75°C to 80°C, and sodium carbonate (26.9g, 2equiv.) and 4-hydroxypropiophenone (38.1g, 2equiv.) were added in batches, after the addition was completed, the temperature was raised to 110°C to 115°C, and the reaction was carried out by keeping the temperature for 5h. The reaction liquid was cooled to 50°C, added with 100g of water, and stirred for 15min. After being acidified to pH 3-4, the filter cake obtained by filtration was transferred into a 1L beaker, added with 200g of water, stirred evenly, adjusted to pH 7-7.5 with sodium bicarbonate and filtered, the filter cake was washed with appropriate amount of water, the filtrate was retained, and transferred to a clean 1L beaker. The resulting filtrate was acidified to pH 3-4 to precipitate a product. After filtration, the obtained filter cake was air-dried at 60°C to obtain 29.6 g of Compound **I-A** with a yield of 85.9% and a purity of 95.73%.

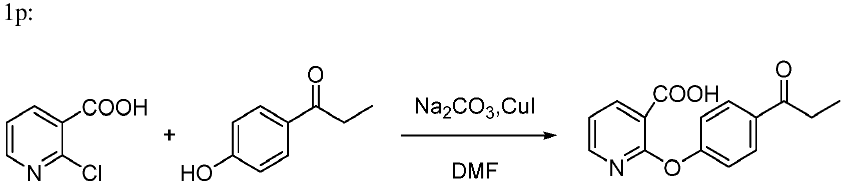

2-Chloronicotinic acid (20.0g, 1equiv.), *N,N*-dimethylformamide (20ml), cuprous iodide (4.8g, 0.2equiv.) were added into a 250mL reaction bottle, stirred and mixed, and heated to 75°C to 80°C, and sodium carbonate (40.4g, 3equiv.) and 4-hydroxypropiophenone (38.1g, 2equiv.) were added in batches, after the addition was completed, the temperature was raised to 110°C to 115°C, and the reaction was carried out by keeping the temperature for 5h. The reaction liquid was cooled to 50°C, added with 100g of water, and stirred for 15min. After being acidified to pH 3-4, the filter cake obtained by filtration was transferred into a 1L beaker, added with 200g of water, stirred evenly, adjusted to pH 7-7.5 with sodium bicarbonate and filtered, the filter cake was washed with appropriate amount of water, the filtrate was retained, and transferred to a clean 1L beaker. The resulting filtrate was acidified to pH 3-4 to precipitate a product. After filtration, the resulting filter cake was air-dried at 60°C to obtain 24.5 g of Compound **I-A** with a yield of 71.1% and a purity of 94.53%.

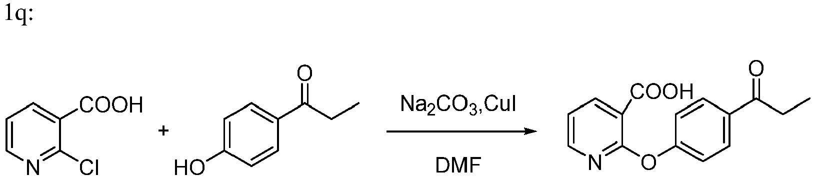

2-Chloronicotinic acid (20.0g, 1equiv.), *N,N*-dimethylformamide (20ml), cuprous iodide (4.8g, 0.2equiv.) were added into a 250mL reaction bottle, stirred and mixed, and heated to 75°C to 80°C, and sodium carbonate (53.8g, 4equiv.) and 4-hydroxypropiophenone (38.1g, 2equiv.) were added in batches, after the addition was completed, the temperature was raised to 110°C to 115°C, and the reaction was carried out by keeping the temperature for 5h. The reaction liquid was cooled to 50°C, added with 100g of water, and stirred for 15min. After being acidified to pH 3-4, the filter cake obtained by filtration was transferred into a 1L beaker, added with 200g of water, stirred evenly, adjusted to pH 7-7.5 with sodium bicarbonate and filtered, the filter cake was washed with appropriate amount of water, the filtrate was retained, and transferred to a clean 1L beaker. The resulting filtrate was acidified to pH 3-4 to precipitate a product. After filtration, the obtained filter cake was air-dried at 60°C to obtain 24.2 g of Compound **I-A** with a yield of 70.2% and a purity of 94.17%.

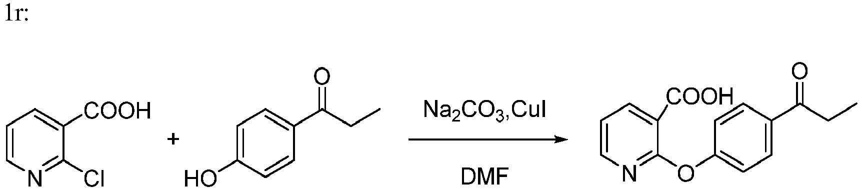

2-Chloronicotinic acid (20.0g, 1equiv.), *N,N*-dimethylformamide (20ml), cuprous iodide (12.1g, 0.5equiv.) were added into a 250mL reaction bottle, stirred and mixed, and heated to 75°C to 80°C, and sodium carbonate (26.9g, 2equiv.) and 4-hydroxypropiophenone (38.1g, 2equiv.) were added in batches, after the addition was completed, the temperature was raised to 110°C to 115°C, and the reaction was carried out by keeping the temperature for 5h. The reaction liquid was cooled to 50°C, added with 100g of water, and stirred for 15min. After being acidified to pH 3-4, the filter cake obtained by filtration was transferred into a 1L beaker, added with 200g of water, stirred evenly, adjusted to pH 7-7.5 with sodium bicarbonate and filtered, the filter cake was washed with appropriate amount of water, the filtrate was retained, and transferred to a clean 1L beaker. The resulting filtrate was acidified to pH 3-4 to precipitate a product. After filtration, the resulting filter cake was air-dried at 60°C to obtain 29.7 g of Compound **I-A** a yield of 86.1% and a purity of 94.77%.

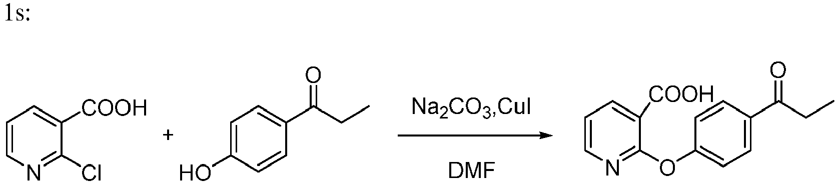

2-Chloronicotinic acid (20.0g, 1 equiv.), *N,N*-dimethylformamide (20ml), cuprous iodide (24.2, 1.0 equiv.) were added into a 250mL reaction bottle, stirred and mixed, and heated to 75°C to 80°C, and sodium carbonate (26.9g, 2equiv.) and 4-hydroxypropiophenone (38.1g, 2equiv.) were added in batches, after the addition was complete, the temperature was raised to 110°C to 115°C, and the reaction was carried out by keeping the temperature for 5h. The reaction liquid was cooled to 50°C, added with 100g of water, and stirred for 15min. After being acidified to pH 3-4, the filter cake obtained by filtration was transferred into a 1L beaker, added with 200g of water, stirred evenly, adjusted to pH 7-7.5 with sodium bicarbonate and filtered, the filter cake was washed with appropriate amount of water, the filtrate was retained, and transferred to a clean 1L beaker. The resulting filtrate was acidified to pH 3-4 to precipitate a product. After filtration, the resulting filter cake was air-dried at 60°C to obtain 30.1 g of Compound **I-A** with a yield of 87.3% and a purity of 93.85%.

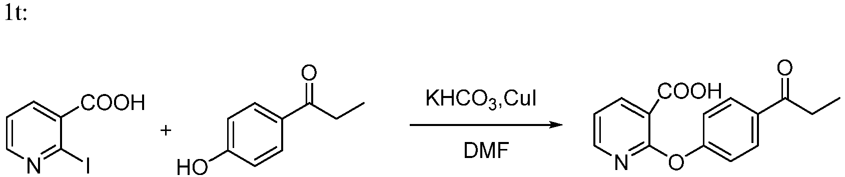

2-Iodonicotinic acid (20.0g, 1equiv.), *N,N*-dimethylformamide (20ml), cuprous iodide (1.5g, 0.1equiv.) were added into a 250ml reaction bottle, stirred and mixed, and heated to 75°C to 80°C, and sodium carbonate (17.0g, 2equiv.) and 4-hydroxypropiophenone (24.1g, 2equiv.) were added in batches, after the addition was completed, the temperature was raised to 110°C to 115°C, and the reaction was carried out by keeping the temperature for 4h. The reaction liquid was cooled to 50°C, added with 100g of water, and stirred for 15min. After being acidified to pH 3-4, the filter cake obtained by filtration was transferred into a 1L beaker, added with 200g of water, stirred evenly, adjusted to pH 7-7.5 with sodium bicarbonate and filtered, the filter cake was washed with appropriate amount of water, the filtrate was retained, and transferred to a clean 1L beaker. The resulting filtrate was acidified to pH 3-4 to precipitate a product. After filtration, the obtained filter cake was air-dried at 60°C to obtain 19.5 g of Compound **I-A** with a yield of 89.4% and a purity of 91.98%.

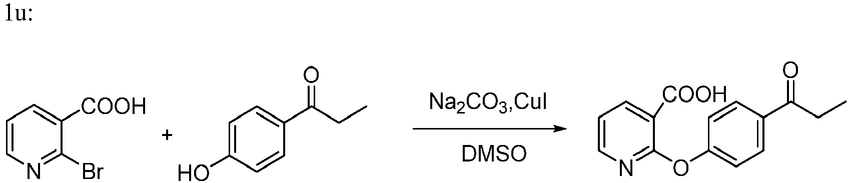

2-Bromonicotinic acid (20.0g, 1equiv.), dimethyl sulfoxide (20.0g), cuprous iodide (1.9g, 0.1equiv.) were added into a 250ml reaction bottle, stirred and mixed, and heated to 75°C to 80°C, and sodium carbonate (21.0g, 2equiv.) and 4-hydroxypropiophenone (29.7g, 2equiv.) were added in batches, after the addition was completed, the temperature was raised to 110°C to 115°C, and the reaction was carried out by keeping the temperature for 5h. The reaction liquid was cooled to 50°C, added with 100g of water, and stirred for 15min. After being acidified to pH 3-4, the filter cake obtained by filtration was transferred into a 1L beaker, added with 200g of water, stirred evenly, adjusted to pH 7-7.5 with sodium bicarbonate and filtered, the filter cake was washed with appropriate amount of water, the filtrate was retained, and transferred to a clean 1L beaker. The resulting filtrate was acidified to pH 3-4 to precipitate a product. After filtration, the obtained filter cake was air-dried at 60°C to obtain 21.2 g of Compound **I-A** with a yield of 78.9% and a purity of 96.38%.

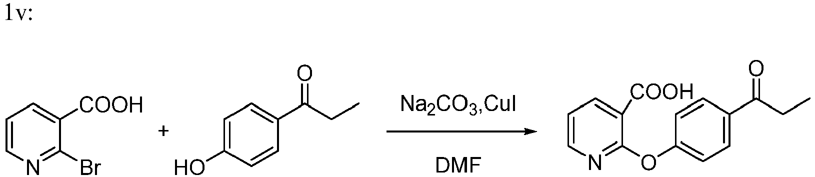

2-Bromonicotinic acid (20.0g, 1equiv.), *N,N*-dimethylformamide (20ml), cuprous iodide (1.9g, 0.1equiv.) were added into a 250mL reaction bottle, stirred and mixed, and heated to 75°C to 80°C, and sodium carbonate (21.0g, 2equiv.) and 4-hydroxypropiophenone (29.7g, 2equiv.) were added in batches, after the addition was completed, the temperature was raised to 130°C to 135°C, and the reaction was carried out by keeping the temperature for 4h. The reaction liquid was cooled to 50°C, added with 100g of water, and stirred for 15min. After being acidified to pH 3-4, the filter cake obtained by filtration was transferred into a 1L beaker, added with 200g of water, stirred evenly, adjusted to pH 7-7.5 with sodium bicarbonate and filtered, the filter cake was washed with appropriate amount of water, the filtrate was retained, and transferred to a clean 1L beaker. The resulting filtrate was acidified to pH 3-4 to precipitate a product. After filtration, the obtained filter cake was air-dried at 60°C to obtain 22.4 g of Compound **I-A** with a yield of 87.3% and a purity of 93.14%.

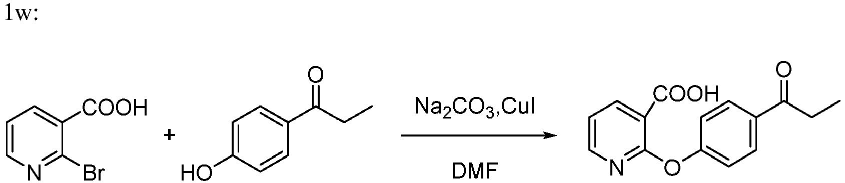

2-Bromonicotinic acid (20.0g, 1equiv.), *N,N*-dimethylformamide (20ml), cuprous iodide (1.9g, 0.1equiv.) were added into a 250mL reaction bottle, stirred and mixed, and heated to 75°C to 80°C, and sodium carbonate (21.0g, 2equiv.) and 4-hydroxypropiophenone (29.7g, 2equiv.) were added in batches, after the addition was completed, the temperature was raised to 140°C to 150°C, and the reaction was carried out by keeping the temperature for 3h. The reaction liquid was cooled to 50°C, added with 100g of water, and stirred for 15min. After being acidified to pH 3-4, the filter cake obtained by filtration was transferred into a 1L beaker, added with 200g of water, stirred evenly, adjusted to pH 7-7.5 with sodium bicarbonate and filtered, the filter cake was washed with appropriate amount of water, the filtrate was retained, and transferred to a clean 1L beaker. The resulting filtrate was acidified to pH 3-4 to precipitate a product. After filtration, the resulting filter cake was air-dried at 60°C to obtain 23.3 g of Compound **I-A** with a yield of 86.8% and a purity of 91.32%.

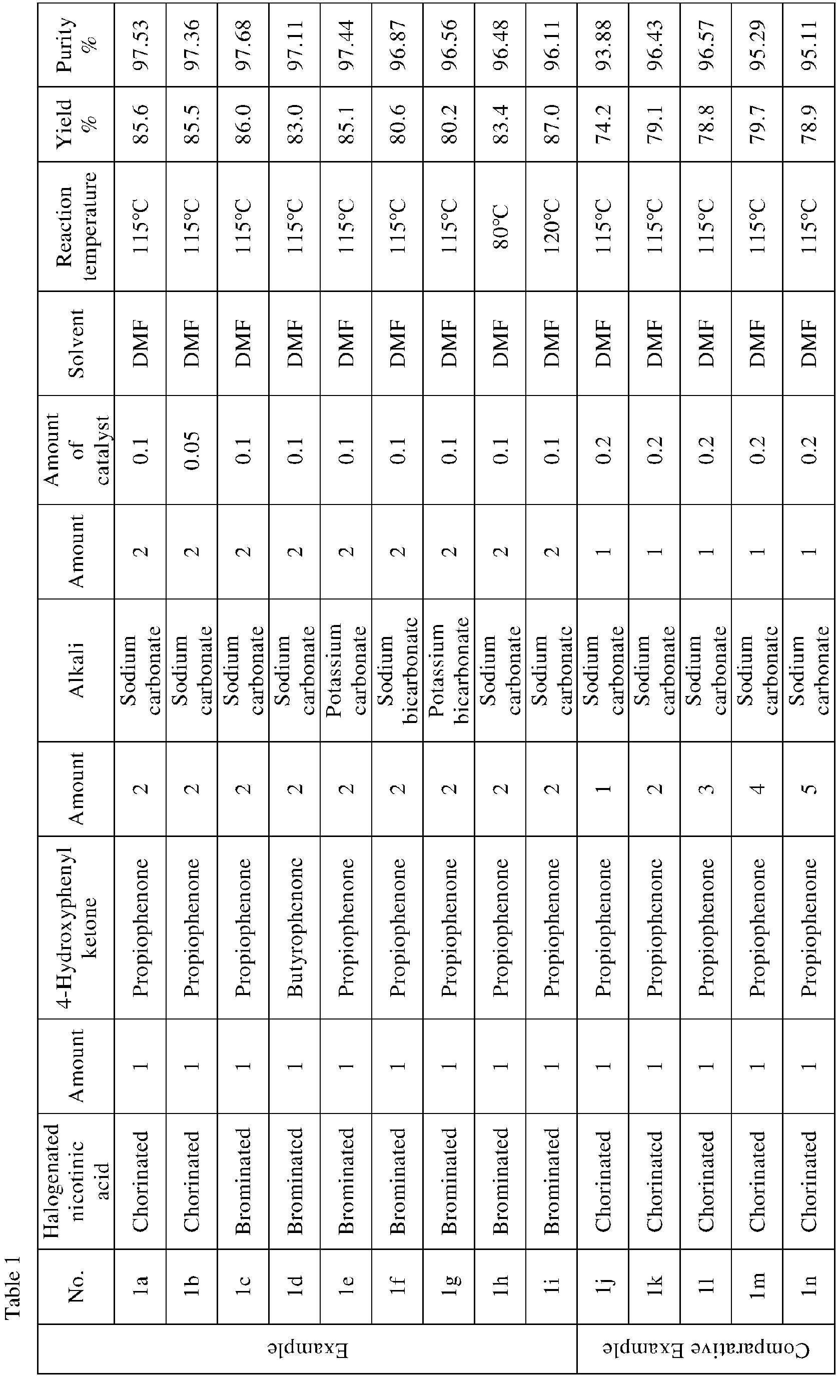

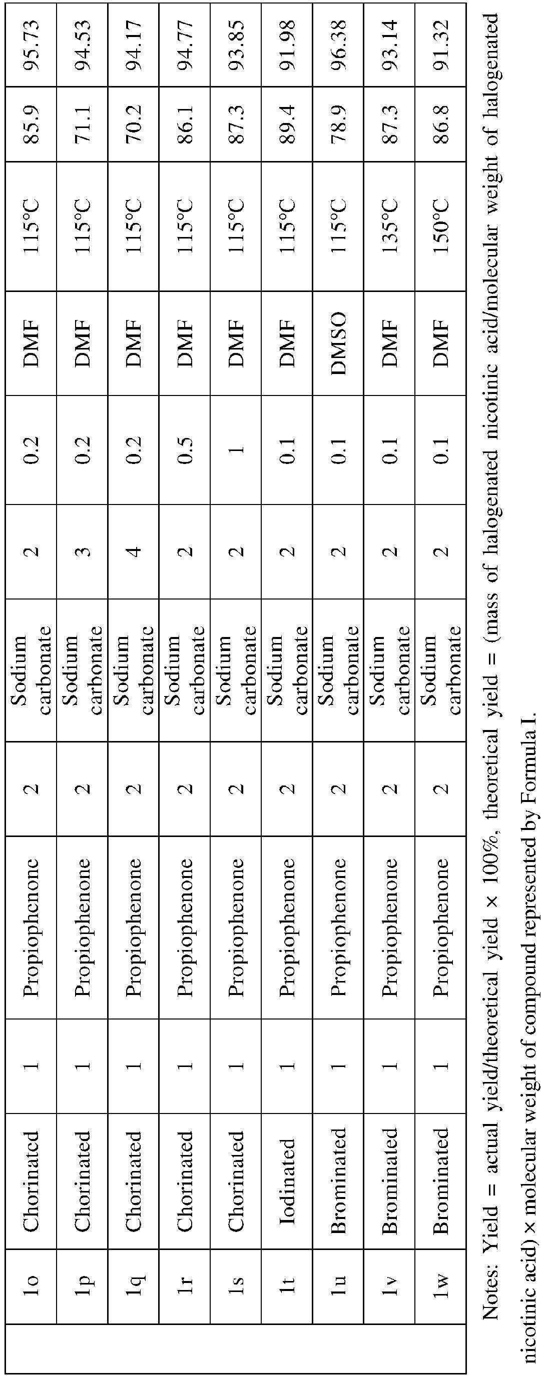

### Preparation Examples 2a to 2d: Preparation of 2-[4-(2-methoxy-1-methyl-2-oxoethyl) phenoxyl-3-pyridinecarboxylic acid (Compound II-A) and 2-[4-(2-ethoxy-1-methyl-2-oxoethyl) phenoxyl-3-pyridinecarboxylic acid (Compound II-B)

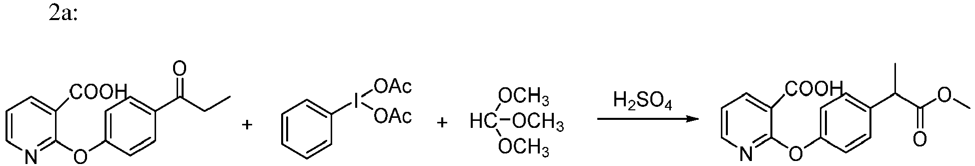

Compound **I-A** (20.0 g, 1 equiv.) and trimethyl orthoformate (60 g) were added into a 250 ml reaction bottle, stirred and mixed evenly, then the reaction solution was cooled to 0°C. Concentrated sulfuric acid (11.1g, 1.5 equiv.) was slowly added dropwise at temperature controlled at below 10°C, after the dropwise addition, the reaction solution was stirred for 15min, then iodobenzene diacetate (47.5g, 2equiv.) was added slowly at temperature controlled at 15°C to 20°C within about 10 to 15min; after the addition, the reaction solution was stirred at 15°C to 20°C for 15min. The temperature was lowered to 0°C, concentrated sulfuric acid (11.1g, 1.5 equiv.) was slowly added dropwise at temperature controlled at below 10°C. After the dropwise addition of concentrated sulfuric acid, the temperature was slowly returned to 10°C to 20°C, and the reaction was carried out by keeping the temperature for 1 hour. Sodium bicarbonate aqueous solution was added for neutralization, the water phase after extraction with dichloromethane was retained, and acidified to pH 3-4, after filtration, the filter cake was washed with water until the filtrate was nearly neutral, and blow dried at 60°C to constant weight to obtain 16.9 g of off-white Compound **II-A** with a yield of 76.0% and a purity of 97.34%.

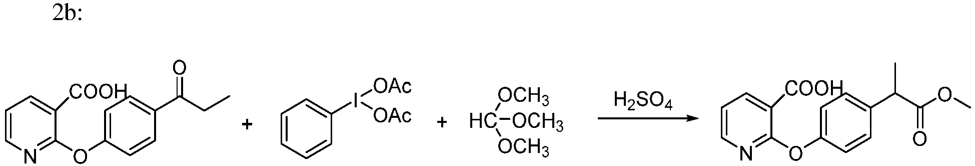

Compound **I-A** (20.0 g, 1 equiv.) and trimethyl orthoformate (60 g) were added into a 250 ml reaction bottle, stirred and mixed evenly, then the reaction solution was cooled to 0°C. Concentrated sulfuric acid (11.1g, 1.5 equiv.) was slowly added dropwise at temperature controlled at below 5°C, after the dropwise addition, the reaction solution was stirred for 15min, iodobenzene diacetate (47.5g, 2equiv.) was slowly added at temperature controlled at 5°C to 10°C within about 10 to 15min, after the addition was completed, the reaction solution was stirred at 5°C to 10°C for 15min. The temperature was lowered to 0°C, concentrated sulfuric acid (11.1g, 1.5 equiv.) was slowly added dropwise at temperature controlled at below 10°C, after the dropwise addition of concentrated sulfuric acid, the temperature was controlled at 5°C to 10°C, and the reaction was carried out by keeping the temperature for 1h. Sodium bicarbonate aqueous solution was added for neutralization, the water phase after extraction with dichloromethane was retained, and acidified to pH 3-4, after filtration, the filter cake was washed with water until the filtrate was nearly neutral, and blow dried at 60°C to constant weight to obtain 16.1 g of off-white Compound **II-A** with a yield of 72.4% and a purity of 96.28%.

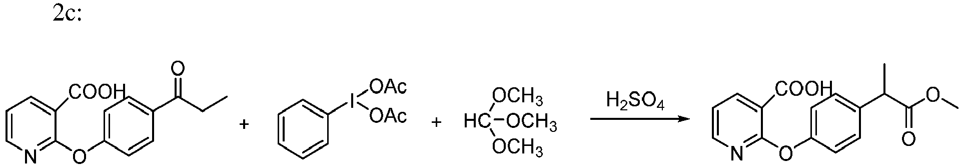

Compound **I-A** (20.0 g, 1 equiv.) and trimethyl orthoformate (60 g) were added into a 250 ml reaction bottle, stirred and mixed well, then the reaction solution was cooled to -5°C. Concentrated sulfuric acid (11.1g, 1.5 equiv.) was slowly added dropwise at temperature controlled at below -5°C, after the dropwise addition, the reaction solution was stirred for 15 minutes, iodobenzene diacetate (47.5g, 2equiv.) was slowly added at temperature controlled at -5°C to 0°C within about 10 to 15min, after the addition, the reaction solution was stirred at -5°C to 0°C for 15min. The temperature was lowered to -5°C, concentrated sulfuric acid (11.1g, 1.5 equiv.) was slowly added dropwise at temperature controlled at below -5°C, after the dropwise addition of concentrated sulfuric acid, the temperature was controlled at -5°C to 0°C, and the reaction was carried out by keeping the temperature for 1h. Sodium bicarbonate aqueous solution was added for neutralization, the water phase after extraction with dichloromethane was retained, and acidified to pH 3-4, after filtration, the filter cake was washed with water until the filtrate was nearly neutral, and blow dried at 60°C to constant weight to obtain 15.9 g of off-white Compound **II-A** with a yield of 71.6% and a purity of 95.61%.

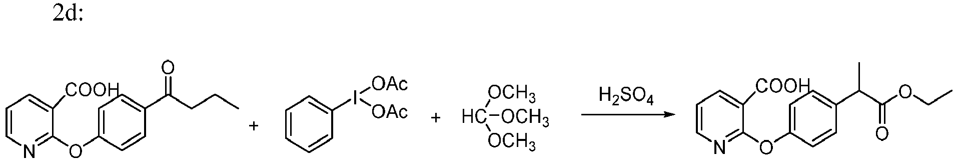

Compound **I-B** (50.0 g, 1 equiv.) and trimethyl orthoformate (150 g) were added into a 500 ml reaction bottle, stirred and mixed evenly, then the reaction solution was cooled to 0°C. Concentrated sulfuric acid (26.4g, 1.5 equiv.) was slowly added dropwise at temperature controlled at below 10°C, after the dropwise addition, the reaction solution was stirred for 15min, iodobenzene diacetate (113.3g, 2equiv.) was slowly added at temperature controlled at 10°C to 20°C within about 10 to 15min, after the addition, the reaction solution was stirred at 10°C to 20°C for 15min. The temperature was lowered to 0°C, concentrated sulfuric acid (26.4g, 1.5 equiv.) was slowly added dropwise at temperature controlled at below 10°C. After the dropwise addition of concentrated sulfuric acid, the temperature was slowly returned to 10°C to 20°C, and the reaction was carried out by keeping the temperature for 1 hour. Sodium bicarbonate aqueous solution was added for neutralization, the water phase after extraction with dichloromethane was retained, and acidified to pH 3-4, after filtration, the filter cake was washed with water until the filtrate was nearly neutral, and blow dried at 60°C to constant weight to obtain 41.5 g of off-white Compound **II-B** with a yield of 75.4% and a purity of 96.98%.

### Comparative Examples 2e to 2p

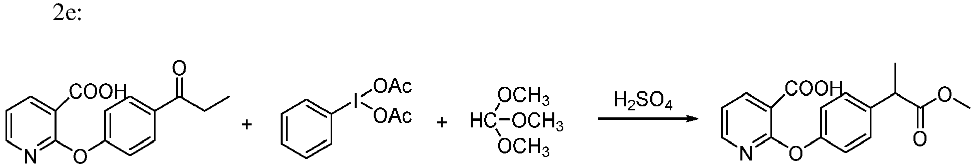

Compound **I-A** (20.0g, 1equiv.) and trimethyl orthoformate (200g) were added into a 500ml reaction bottle, stirred and mixed well, then slowly added dropwise with concentrated sulfuric acid (3.7g, 0.5equiv.), after the dropwise addition, the reaction solution was stirred for 5min, then iodobenzene diacetate (23.7 g, 1 equiv.) was slowly added, after the addition, the reaction solution was stirred for 15 min. Concentrated sulfuric acid (3.7 g, 0.5 equiv.) was slowly added dropwise, after the dropwise addition of concentrated sulfuric acid, the temperature was raised to 105°C for refluxing, and the reaction was carried out by keeping the temperature for 1 h. After cooling to room temperature, sodium bicarbonate aqueous solution was added for neutralization, the water phase after extraction with dichloromethane was retained, and acidified to pH 3-4, the filter cake after filtration was washed with water until the filtrate was nearly neutral, and blow dried at 60°C until constant weight to obtain 16.5 g of off-white Compound **II-A** with a yield of 74.5% and a purity of 89.73%.

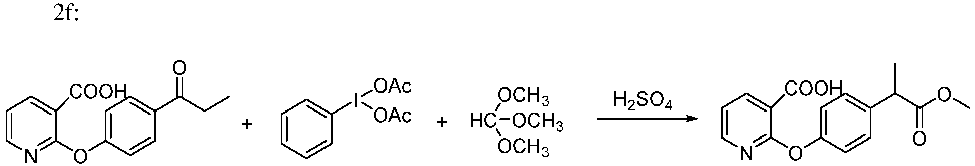

Compound **I-A** (20.0g, 1equiv.) and trimethyl orthoformate (100g) were added into a 250ml reaction bottle, stirred and mixed well, then slowly added dropwise with concentrated sulfuric acid (3.7g, 0.5equiv.), after the dropwise addition, the reaction solution was stirred for 5min, iodobenzene diacetate (23.7 g, 1 equiv.) was slowly added, and stirred for 15 min after the addition. Concentrated sulfuric acid (3.7 g, 0.5 equiv.) was slowly added dropwise, after the dropwise addition of concentrated sulfuric acid, the mixture was heated to 70°C to 75°C and reacted by keeping the temperature for 1 hour. After cooling to room temperature, sodium bicarbonate aqueous solution was added for neutralization, the water phase after extraction with dichloromethane was retained, and acidified to pH 3-4, the filter cake after filtration was washed with water until the filtrate was nearly neutral, and blow dried at 60°C until constant weight to obtain 16.1 g of off-white Compound **II-A** with a yield of 72.4% and a purity of 92.86%.

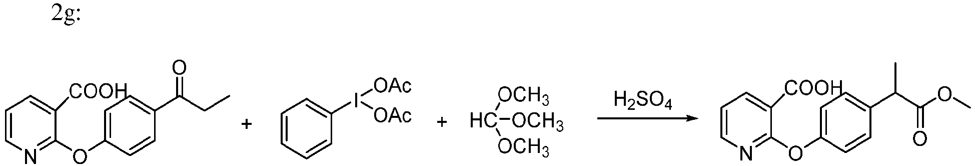

Compound **I-A** (20.0g, 1equiv.) and trimethyl orthoformate (100g) were added into a 250ml reaction bottle, stirred and mixed well, then slowly added dropwise with concentrated sulfuric acid (3.7g, 0.5equiv.), after the addition, the reaction solution was stirred for 5min, then iodobenzene diacetate (23.7 g, 1 equiv.) was added, and stirred for 15 min after the addition. Concentrated sulfuric acid (3.7 g, 0.5 equiv.) was slowly added dropwise, and after the dropwise addition of concentrated sulfuric acid, the mixture was heated to 45°C to 50°C and reacted by keeping the temperature for 1 hour. After cooling to room temperature, sodium bicarbonate aqueous solution was added for neutralization, the water phase after extraction with dichloromethane was retained, and acidified to pH 3-4, the filter cake after filtration was washed with water until the filtrate was nearly neutral, and blow dried at 60°C until constant weight to obtain 15.7 g of off-white Compound **II-A** with a yield of 70.8% and a purity of 93.16%.

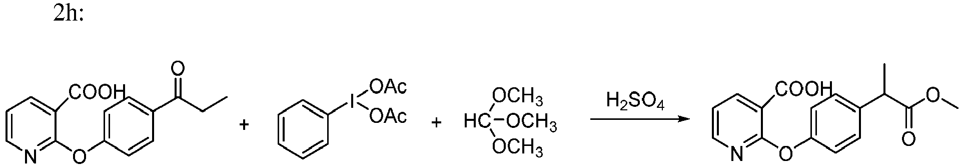

Compound **I-A** (20.0g, 1equiv.) and trimethyl orthoformate (100g) were added into a 250ml reaction bottle, stirred and mixed well, then slowly added dropwise with concentrated sulfuric acid (3.7g, 0.5equiv.), after the dropwise addition, the reaction solution was stirred for 5min, then iodobenzene diacetate (47.5 g, 2 equiv.) was slowly added, and stirred for 15 min after the addition. Concentrated sulfuric acid (3.7 g, 0.5 equiv.) was slowly added dropwise, and after the dropwise addition of concentrated sulfuric acid, the mixture was heated to 45°C to 50°C and reacted by keeping the temperature for 1 hour. After cooling to room temperature, sodium bicarbonate aqueous solution was added for neutralization, the water phase after extraction with dichloromethane was retained, and acidified to pH 3-4, the filter cake after filtration was washed with water until the filtrate was nearly neutral, and blow dried air at 60°C until constant weight to obtain 16.0 g of off-white Compound **II-A** with a yield of 71.9% and a purity of 93.68%.

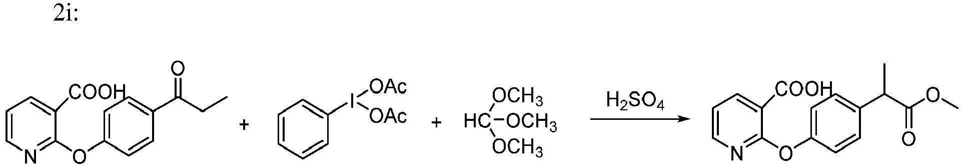

Compound **I-A** (20.0g, 1equiv.) and trimethyl orthoformate (100g) were added into a 250ml reaction bottle, stirred and mixed well, then slowly added with concentrated sulfuric acid (3.7g, 0.5equiv.), after the dropwise addition, the reaction solution was stirred for 5min, iodobenzene diacetate (71.2 g, 2 equiv.) was slowly added, and stirred for 15 min after the addition. Concentrated sulfuric acid (3.7 g, 0.5 equiv.) was slowly added dropwise, and after the dropwise addition of concentrated sulfuric acid, the mixture was heated to 45°C to 50°C and reacted by keeping the temperature for 1 hour. After cooling to room temperature, sodium bicarbonate aqueous solution was added for neutralization, the water phase after extraction with dichloromethane was retained, and acidified to pH 3-4, the filter cake after filtration was washed with water until the filtrate was nearly neutral, and blow dried at 60°C until constant weight to obtain 15.9 g of off-white Compound **II-A** with a yield of 71.7% and a purity of 93.01%.

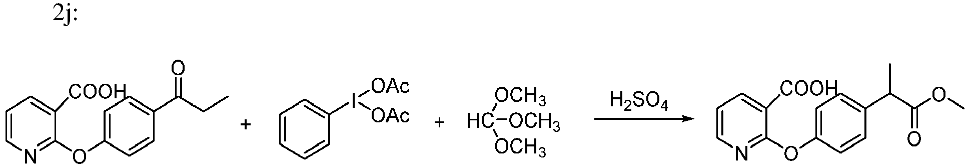

Compound **I-A** (20.0g, 1equiv.) and trimethyl orthoformate (100g) were added into a 250ml reaction bottle, stirred and mixed well, then slowly added with concentrated sulfuric acid (7.4g, lequiv.), after the dropwise addition, the reaction solution was stirred for 5min, iodobenzene acetate (47.5 g, 2 equiv.) was slowly added, and stirred for 15 min after the addition. Concentrated sulfuric acid (7.4g, 1 equiv.) was slowly added dropwise, and after the dropwise addition of concentrated sulfuric acid, the mixture was heated to 45°C to 50°C and reacted by keeping the temperature for 1h. After cooling to room temperature, sodium bicarbonate aqueous solution was added for neutralization, the water phase after extraction with dichloromethane was retained, and acidified to pH 3-4, the filter cake after filtration was washed with water until the filtrate was nearly neutral, and blow dried at 60°C until constant weight to obtain 16.1 g of off-white Compound **II-A** with a yield of 72.3% and a purity of 94.28%.

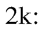

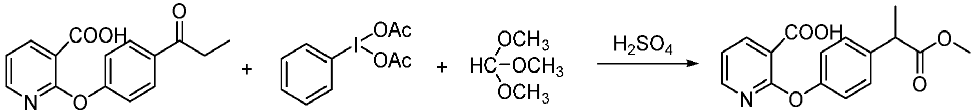

Compound **I-A** (20.0g, 1equiv.) and trimethyl orthoformate (100g) were added into a 250ml reaction bottle, stirred and mixed well, then slowly added with concentrated sulfuric acid (11.1g, 1.5equiv.), after the dropwise addition, the reaction solution was stirred for 5min, iodobenzene diacetate (47.5 g, 2 equiv.) was slowly added, and stirred for 15 min after the addition. Concentrated sulfuric acid (11.1 g, 1.5 equiv.) was slowly added dropwise, and after the dropwise addition of concentrated sulfuric acid, the mixture was heated to 45°C to 50°C and reacted by keeping the temperature for 1 hour. After cooling to room temperature, sodium bicarbonate aqueous solution was added for neutralization, the water phase after extraction with dichloromethane was retained, and acidified to pH 3-4, the filter cake after filtration was washed with water until the filtrate was nearly neutral, and blow dried at 60°C until constant weight to obtain 16.2 g of off-white Compound **II-A** with a yield of 72.9% and a purity of 94.64%.

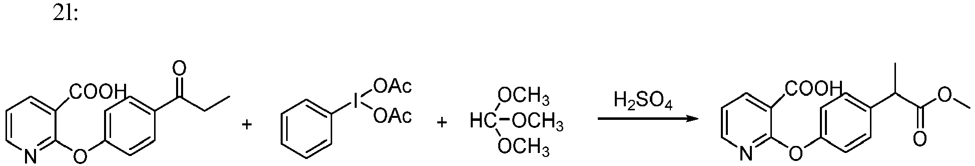

Compound **I-A** (20.0g, 1equiv.) and trimethyl orthoformate (100g) were added into a 250ml reaction bottle, stirred and mixed well, then slowly added with concentrated sulfuric acid (14.8g, 2equiv.), after the dropwise addition, the reaction solution was stirred for 5min, iodobenzene acetate (47.5 g, 2 equiv.) was slowly added, and stirred for 15 min after the addition. Concentrated sulfuric acid (14.8 g, 2 equiv.) was slowly added dropwise, and after the dropwise addition of concentrated sulfuric acid, the mixture was heated to 45°C to 50°C and reacted by keeping the temperature for 1 hour. After cooling to room temperature, sodium bicarbonate aqueous solution was added for neutralization, the water phase after extraction with dichloromethane was retained, and acidified to pH 3-4, the filter cake after filtration was washed with water until the filtrate was nearly neutral, and blow dried at 60°C until constant weight to obtain 16.1 g of off-white Compound **II-A** with a yield of 72.3% and a purity of 93.45%.

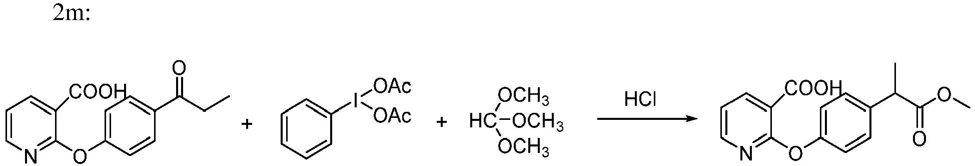

Compound **I-A** (20.0g, 1equiv.) and trimethyl orthoformate (60.0g) were added into a 250ml reaction bottle, stirred and mixed evenly, then slowly added with hydrochloric acid (30.1g, 1.5equiv.), after the dropwise addition, the reaction solution was stirred for 15min, iodobenzene diacetate (47.5 g, 2 equiv.) was slowly added, and stirred for 15 min after the addition. Hydrochloric acid (30.1 g, 1.5 equiv.) was slowly added dropwise, and after the dropwise addition of hydrochloric acid, the mixture was heated to 45°C to 50°C and reacted by keeping the temperature for 1h. Sodium bicarbonate aqueous solution was added for neutralization, the water phase after extraction with dichloromethane was retained, and acidified to pH 3-4, the filter cake after filtration was washed with water until the filtrate was nearly neutral, and blow dried at 60°C to constant weight to obtain 14.9 g of off-white Compound **II-A** with a yield of 67.1% and a purity of 93.33%.

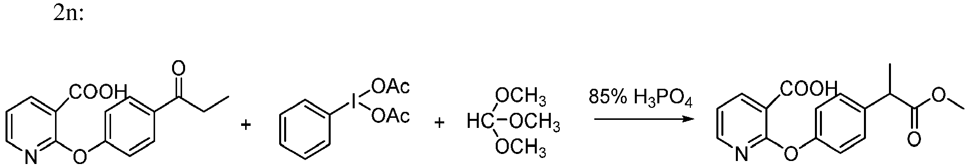

Compound **I-A** (20.0g, 1equiv.) and trimethyl orthoformate (60.0g) were added into a 250ml reaction bottle, stirred and mixed evenly, then slowly added dropwise with 85% phosphoric acid (12.8g, 1.5equiv.), after the dropwise addition, the reaction solution was stirred for 15min, then iodobenzene diacetate (47.5 g, 2 equiv.) was slowly added, and stirred for 15 min after the addition. 85% Phosphoric acid (12.8 g, 1.5 equiv.) was slowly added dropwise, and after the dropwise addition, the mixture was heated to 45°C to 50°C, and reacted by keeping the temperature for 1h. Sodium bicarbonate aqueous solution was added for neutralization, the water phase after extraction with dichloromethane was retained, and acidified to pH 3-4, the filter cake after filtration was wash with water until the filtrate was nearly neutral, and blow dried at 60°C to constant weight to obtain 14.3 g of off-white Compound **II-A** with a yield of 64.5% and a purity of 91.79%.

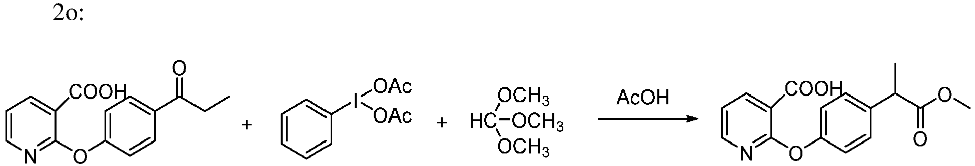

Compound **I-A** (20.0g, 1equiv.) and trimethyl orthoformate (60.0g) were added into a 250ml reaction bottle, stirred and mixed evenly, then slowly added with acetic acid (10.8g, 1.5equiv.), after the dropwise addition, the reaction solution was stirred for 15min, iodobenzene diacetate (47.5 g, 2 equiv.) was slowly added, and stirred for 15 min after the addition. Acetic acid (10.8 g, 1.5 equiv.) was slowly added dropwise, after the dropwise addition, the mixture was heated to 45°C to 50°C, and reacted by keeping the temperature for 1h. Sodium bicarbonate aqueous solution was added for neutralization, the water phase after extraction with dichloromethane was retained, and acidified to pH 3-4, the filter cake after filtration was washed with water until the filtrate was nearly neutral, and blow dried at 60°C to constant weight to obtain 12.4 g of off-white Compound **II-A** with a yield of 55.7% and a purity of 88.67%.

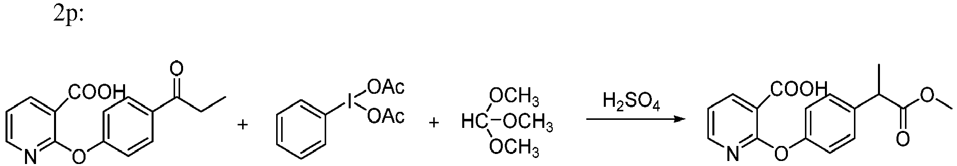

Compound **I-A** (20.0 g, 1 equiv.) and trimethyl orthoformate (60 g) were added into a 250 ml reaction bottle, stirred and mixed evenly, then the reaction solution was cooled to 0°C to 10°C. Concentrated sulfuric acid (11.1g, 1.5 equiv.) was slowly added dropwise at temperature controlled at below 10°C, after the dropwise addition, the reaction solution was stirred for 15 minutes, iodobenzene diacetate (47.5g, 2equiv.) was slowly added at temperature controlled at below 20°C within about 10 to 15min, after the addition, the reaction solution was stirred for 15 minutes. After cooling down to 0°C to 10°C, concentrated sulfuric acid (11.1g, 1.5 equiv.) was slowly added dropwise at temperature controlled at below 10°C, after the dropwise addition of concentrated sulfuric acid, the mixture was heated to 25°C to 30°C, and reacted by keeping the temperature for 1h. Sodium bicarbonate aqueous solution was added for neutralization, the water phase after extraction with dichloromethane was retained, and acidified to pH 3-4, the filter cake after filtration was washed with water until the filtrate was nearly neutral, and blow dried at 60°C to constant weight to obtain 16.2 g of off-white Compound **II-A** with a yield of 73.1% and a purity of 94.96%.

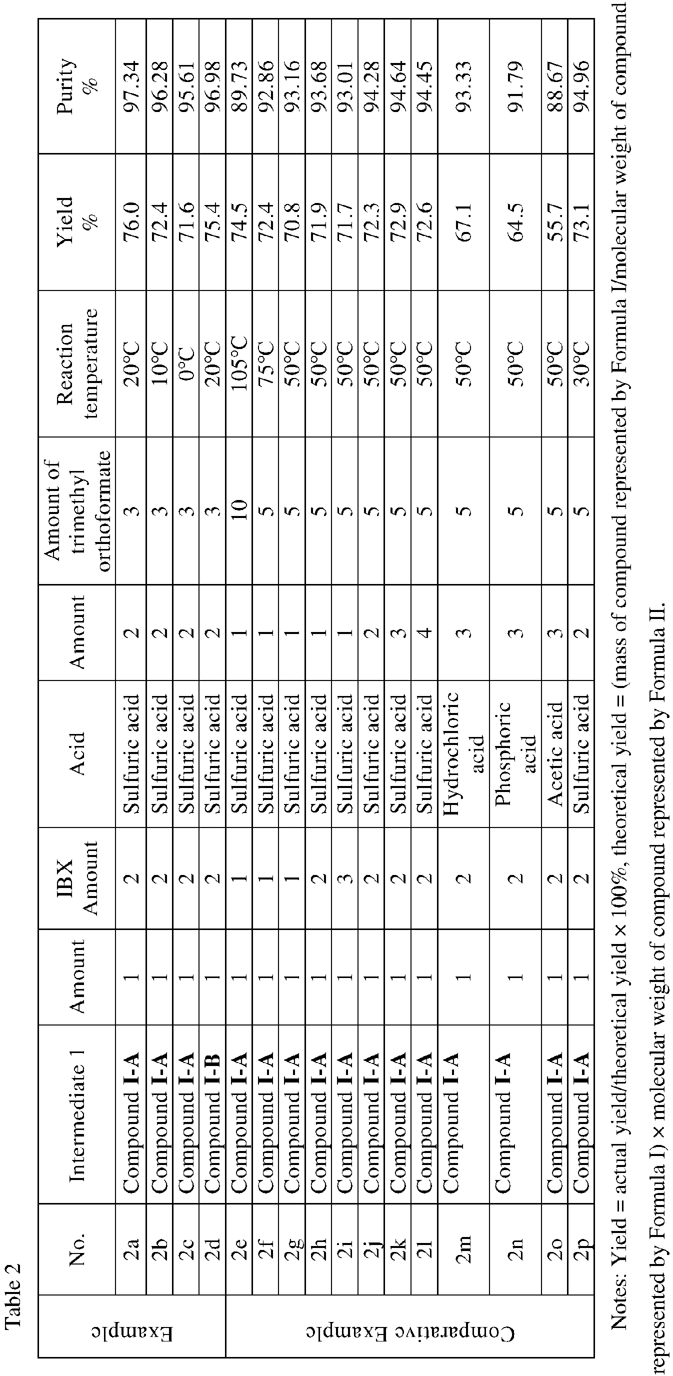

### Preparation Examples 3a to 3g: Preparation of methyl 2-(10-oxo-9-oxa-1-azaanthracen-6-yl)propionate (Compound III-A) and ethyl 2-(10-oxo-9-oxa-1-azaanthracen-6-yl)propionate (Compound III-B)

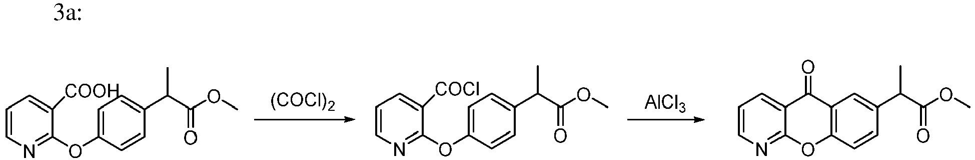

Compound **II-A** (20.0g, 1 equiv.), dichloromethane (100ml), *N,N*-dimethylformamide (0.5g) were added into 250ml reaction bottle A, stirred and mixed, then added dropwise with oxalyl chloride (12.6g, 1.5equiv.) at temperature controlled at 15°C to 25°C, after the addition, the temperature was kept for 1h after the dropwise addition. In addition, anhydrous aluminum trichloride (17.7 g, 2 equiv.) and dichloromethane (100 ml) were added into 500 ml reaction bottle B, stirred and mixed. The acyl chloride solution in the reaction bottle A was transferred dropwise to the reaction bottle B at temperature controlled at 15°C to 25°C, the reaction was carried out by keeping the temperature for 1 to 1.5h; after the dropwise transferring, the reaction solution was cooled to -10°C after the reaction was completed. The reaction solution was slowly transferred into cold water to quench at temperature controlled at below 20°C. After the addition, the reaction solution was stirred for 15 to 30min, and separated into layers, the dichloromethane phase was retained, the water phase was extracted with dichloromethane (40ml), and separated into layers, then the dichloromethane phases were combined, adjusted to pH 7-8 with sodium bicarbonate solution, ans separated into layers, and the dichloromethane layer was washed once with purified water (100 ml). Dichloromethane was recovered by concentration under reduced pressure; after the distillate was no longer evaporated, methanol (100ml) was added for beating at 20°C to 25°C for 5 to 6h, after filtration, the filter cake was rinsed with methanol, and blow dried at 45°C to obtain 16.3 g of Compound **III-A** with a yield of 86.8% and a purity of 99.55%.

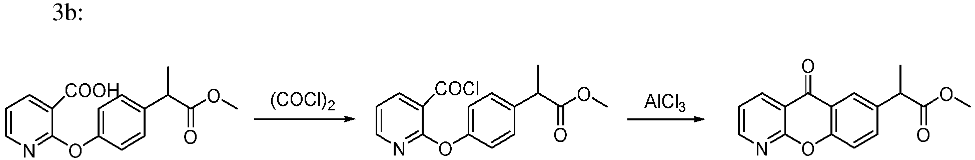

Compound **II-A** (20.0g, lequiv.), dichloromethane (100ml), N,N-dimethylformamide (0.5g) were added into 250ml reaction bottle A, stirred and mixed, then added dropwise with oxalyl chloride (12.6g, 1.5equiv.) at temperature controlled at 15°C to 25°C, after the dropwise addition, the temperature was kept at 15°C to 25°C for 1h. In addition, anhydrous aluminum trichloride (26.6 g, 3 equiv.) and dichloromethane (100 ml) were added into 500 ml reaction bottle B, stirred and mixed. The acyl chloride solution in the reaction bottle A was transferred dropwise to the reaction bottle B at temperature controlled at 15°C to 25°C, the reaction was carried out by keeping the temperature for 1 to 1.5h after the dropwise addition, and the reaction solution was cooled to -10°C after the reaction was completed. The reaction solution was slowly transferred into cold water to quench at temperature controlled at below 20°C. After the addition, the reaction solution was stirred for 15 to 30min, and separated into layers, the dichloromethane phase was retained, the water phase was extracted with dichloromethane (40ml), and separated into layers, the dichloromethane phases were combined, adjusted to pH 7-8 with sodium bicarbonate solution, and separated into layers, and the dichloromethane layer was washed once with purified water (100 ml), then concentrated under reduced pressure to recover dichloromethane. When the distillate was no longer evaporated, methanol (100ml) was added for beating at 20°C to 25°C for 5 to 6h, after filtration, the filter cake was washed with methanol, and blow dried at 45°C to obtain 15.8 g of Compound **III-A** with a yield of 84.1% and a purity of 99.59%.

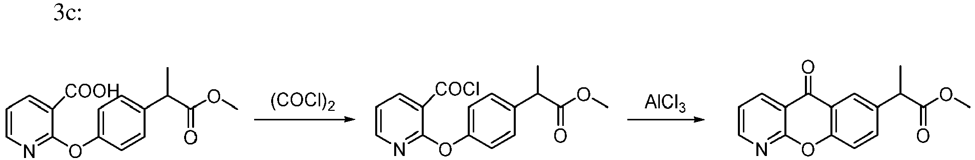

Compound **II-A** (20.0g, lequiv.), dichloromethane (100ml), *N,N*-dimethylformamide (0.5g) were added into 250ml reaction bottle A, stirred and mixed, then added dropwise with oxalyl chloride (12.6g, 1.5equiv.) at temperature controlled at 15°C to 25°C, after the dropwise addition, the temperature was kept at 15°C to 25°C for 1h. In addition, anhydrous aluminum trichloride (35.4 g, 4 equiv.) and dichloromethane (100 ml) were added into 500 ml reaction bottle B, stirred and mixed. The acyl chloride solution in the reaction bottle A was transferred dropwise into the reaction bottle B at temperature controlled at 15°C to 25°C, the reaction was carried out by keeping the temperature for 1 to 1.5h, and the reaction solution was cooled to -10°C after the reaction was completed. The reaction solution was slowly transferred into cold water to quench at temperature controlled at below 20°C. The reaction solution was stirred for 15 to 30min after the addition, and separated into layers, the dichloromethane phase was retained, the water phase was extracted with dichloromethane (40ml), and separated into layers, the dichloromethane phases were combined, adjusted to pH 7-8 with sodium bicarbonate solution, and separated into layers, and the dichloromethane layer was washed once with purified water (100 ml), and concentrated under reduced pressure to recover dichloromethane, when the distillate was no longer evaporated, methanol (100ml) was added for beating at 20°C to 25°C for 5 to 6h, the filter cake after filtration was rinsed with methanol, and blow dried at 45°C to obtain 15.9 g of Compound **III-A** with a yield of 84.3% and a purity of 99.54%.

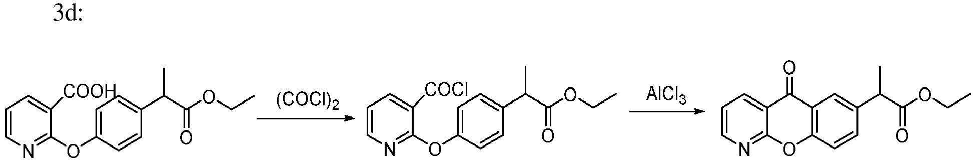

Compound **II-B** (40.0g, 1 equiv.), dichloromethane (200ml), N,N-dimethylformamide (0.9g) were added into 500ml reaction bottle A, stirred and mixed, then added dropwise with oxalyl chloride (24.2g, 1.5equiv.) at temperature controlled at 15°C to 25°C , and the temperature was kept at 15°C to 25°C for 1h after the dropwise addition. In addition, anhydrous aluminum trichloride (33.9 g, 2 equiv.) and dichloromethane (200 ml) were added into 1000 ml reaction bottle B, stirred and mixed. The acyl chloride solution in the reaction bottle A was transferred dropwise into the reaction bottle B at temperature controlled at 15°C to 25°C, the reaction was carried out by keeping the temperature for 1 to 1.5h, and the reaction solution was cooled to -10°C after the reaction was completed. The reaction solution was slowly transferred into cold water to quench at temperature controlled at below 20°C. The reaction solution was stirred for 15 to 30min after the addition, and separated into layers, the dichloromethane phase was retained, the water phase was extracted with dichloromethane (80ml), and separated into layers, the dichloromethane phases were combined, adjusted to pH 7-8 with sodium bicarbonate solution, and separated into layers, and the dichloromethane layer was washed once with purified water (200ml), and concentrated under reduced pressure to recover dichloromethane, when the distillate was no longer evaporated, methanol (200ml) was added for beating at 20°C to 25°C for 5 to 6h, the filter cake after filtration was rinsed with methanol, and blow dried at 45°C to obtain 32.2 g of Compound **III-B** with a yield of 85.7% and a purity of 99.26%.

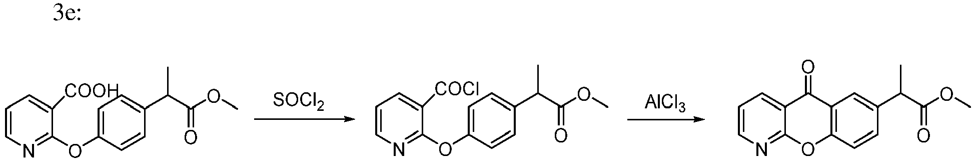

Compound **II-A** (20.0g, lequiv.), dichloromethane (100ml), N,N-dimethylformamide (0.5g) were added into 250ml reaction bottle A, stirred and mixed, then added with thionyl chloride (11.8, 1.5 equiv.) at temperature controlled at 15°C to 25°C, and the temperature was kept at 15°C to 25°C for 1h after the dropwise addition. In addition, anhydrous aluminum trichloride (17.7 g, 2 equiv.) and dichloromethane (100 ml) were added into 500 ml reaction bottle B, stirred and mixed. The acyl chloride solution in the reaction bottle A was transferred dropwise into the reaction bottle B at temperature controlled at 15°C to 25°C, the reaction was carried out by keeping the temperature for 1 to 1.5h, and the reaction solution was cooled to -10°C after the reaction was completed. The reaction solution was slowly transferred into cold water to quench at temperature controlled at below 20°C. The reaction solution was stirred for 15 to 30min after the addition, and separated into layers, the dichloromethane phase was retained, the water phase was extracted with dichloromethane (40ml), and separated into layers, the dichloromethane phases were combined, adjusted to pH 7-8 with sodium bicarbonate solution, and separated into layers, and the dichloromethane layer was washed once with purified water (100 ml), and concentrated under reduced pressure to recover dichloromethane, when the distillate was no longer evaporated, methanol (100ml) was added for beating at 20°C to 25°C for 5 to 6h, the filter cake after filtration was rinsed with methanol, and blow dried at 45°C to obtain 16.4 g of Compound **III-A** with a yield of 87.1% and a purity of 99.67%.

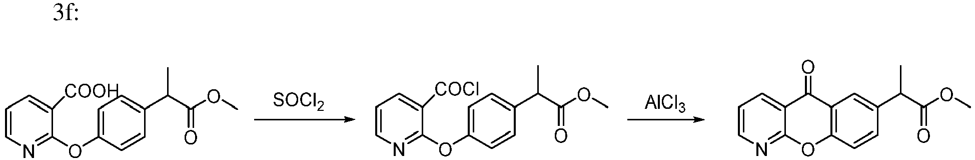

Compound **II-A** (20.0g, lequiv.), dichloromethane (100ml), *N,N*-dimethylformamide (0.5g) were added into 250ml reaction bottle A, stirred and mixed, then added with thionyl chloride (11.8, 1.5 equiv.) at temperature controlled at 15°C to 25°C, and the temperature was kept at 15°C to 25°C for 1h after the dropwise addition. In addition, anhydrous aluminum trichloride (17.7 g, 2 equiv.) and dichloromethane (100 ml) were added into 500 ml reaction bottle B, stirred and mixed. The acyl chloride solution in the reaction bottle A was transferred dropwise into the reaction bottle B at temperature controlled at 15°C to 25°C, the reaction was carried out by keeping the temperature for 1 to 1.5h, and the reaction solution was cooled to -10°C after the reaction was completed. The reaction solution was slowly transferred into cold water to quench at temperature controlled at below 20°C. The reaction solution was stirred for 15 to 30min after the addition, and separated into layers, the dichloromethane phase was retained, the water phase was extracted with dichloromethane (40ml), and separated into layers, the dichloromethane phases were combined, adjusted to pH 7-8 with sodium bicarbonate solution, and separated into layers, and the dichloromethane layer was washed once with purified water (100 ml), and concentrated under reduced pressure to recover dichloromethane, when the distillate was no longer evaporated, methanol/dichloromethane mixed solvent (100ml, solvent ratio 1:0.1) was added for beating at 20°C to 25°C for 5 to 6h, the filter cake after filtration was rinsed with methanol, and blow dried at 45°C to obtain 16.1 g of Compound **III-A** with a yield of 85.6% and a purity of 99.71%.

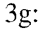

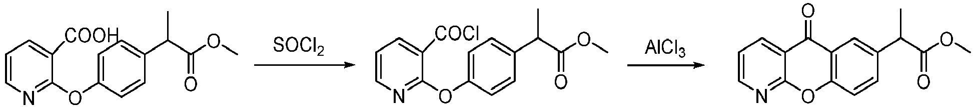

Compound **II-A** (20.0g, lequiv.), dichloromethane (100ml), N,N-dimethylformamide (0.5g) were added into 250ml reaction bottle A, stirred and mixed, then added with thionyl chloride (11.8, 1.5 equiv.) at temperature controlled at 15°C to 25°C, and the temperature was kept at 15°C to 25°C for 1h after the dropwise addition. In addition, anhydrous aluminum trichloride (17.7 g, 2 equiv.) and dichloromethane (100 ml) were added into 500 ml reaction bottle B, stirred and mixed. The acyl chloride solution in the reaction bottle A was transferred dropwise into the reaction bottle B at temperature controlled at 15°C to 25°C, the reaction was carried out by keeping the temperature for 1 to 1.5h, and the reaction solution was cooled to -10°C after the reaction was completed. The reaction solution was slowly transferred into cold water to quench at temperature controlled at below 20°C. The reaction solution was stirred for 15 to 30min after the addition, and separated into layers, the dichloromethane phase was retained, the water phase was extracted with dichloromethane (40ml), and separated into layers, the dichloromethane phases were combined, adjusted to pH 7-8 with sodium bicarbonate solution, and separated into layers, and the dichloromethane layer was washed once with purified water (100 ml), and concentrated under reduced pressure to recover dichloromethane, when the distillate was no longer evaporated, methanol/dichloromethane mixed solvent (100ml, solvent ratio 1:0.2) was added for beating at (100ml, solvent ratio 1:0.2) when the distillate no longer evaporates, beat at 20°C to 25°C for 5 to 6h, , the filter cake after filtration was rinsed with methanol, and blow dried at 45°C to obtain 15.7 g of Compound **III-A** with a yield of 83.5% and a purity of 99.73%.

### Comparative Examples 3h to 3p

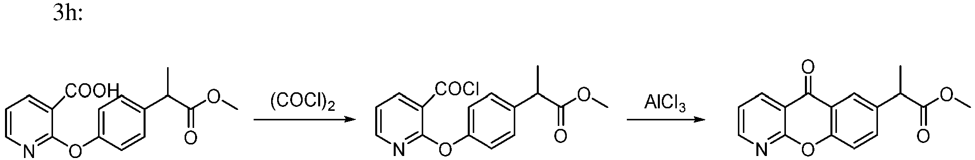

Compound **II-A** (20.0g, lequiv.), dichloromethane (100ml), N,N-dimethylformamide (0.5g) were added into 250ml reaction bottle A, stirred and mixed, then added dropwise with oxalyl chloride (9.3 g, 1.1 equiv.) at temperature controlled at 15°C to 25°C, and the temperature was kept at 15°C to 25°C for 1 hour after the dropwise addition. In addition, anhydrous aluminum trichloride (9.7 g, 1.1 equiv.) and dichloromethane (100 ml) were added into 500 ml reaction bottle B, stirred and mixed. The acyl chloride solution in the reaction bottle A was transferred dropwise into the reaction bottle B at temperature controlled at 15°C to 25°C, the reaction was carried out by keeping the temperature for 1 to 1.5h, and the reaction solution was cooled to -10°C after the reaction was completed. The reaction solution was slowly transferred into cold water to quench at temperature controlled at below 20°C. The reaction solution was stirred for 15 to 30min after the addition, and separated into layers, the dichloromethane phase was retained, the water phase was extracted with dichloromethane (40ml), and separated into layers, the dichloromethane phases were combined, adjusted to pH 7-8 with sodium bicarbonate solution, and separated into layers, and the dichloromethane layer was washed once with purified water (100 ml), and concentrated under reduced pressure to recover dichloromethane, when the distillate was no longer evaporated, methanol (100ml) was added for beating at 20°C to 25°C for 5 to 6h, the filter cake after filtration was rinsed with methanol, and blow dried at 45°C to obtain 14.4 g of Compound **III-A** with a yield of 76.8% and a purity of 91.26%.

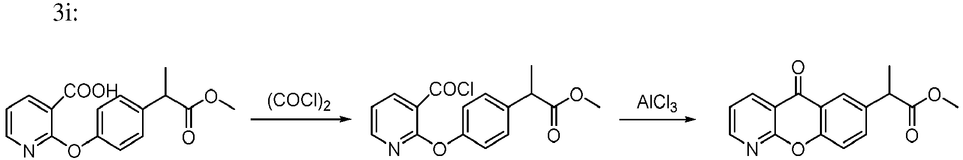

Compound **II-A** (20.0g, lequiv.), dichloromethane (100ml), *N,N*-dimethylformamide (0.5g) were added into 250ml reaction bottle A, stirred and mixed, then added dropwise with oxalyl chloride (12.6g, 1.5equiv.) at temperature controlled at 15°C to 25°C, and the temperature was kept at 15°C to 25°C for 1h after the dropwise addition. In addition, anhydrous aluminum trichloride (9.7 g, 1.1 equiv.) and dichloromethane (100 ml) were added into 500 ml reaction bottle B, stirred and mixed. The acyl chloride solution in the reaction bottle A was transferred dropwise into the reaction bottle B at temperature controlled at 15°C to 25°C, the reaction was carried out by keeping the temperature for 1 to 1.5h, and the reaction solution was cooled to -10°C after the reaction was completed. The reaction solution was slowly transferred into cold water to quench at temperature controlled at below 20°C. The reaction solution was stirred for 15 to 30min after the addition, and separated into layers, the dichloromethane phase was retained, the water phase was extracted with dichloromethane (40ml), and separated into layers, the dichloromethane phases were combined, adjusted to pH 7-8 with sodium bicarbonate solution, and separated into layers, and the dichloromethane layer was washed once with purified water (100 ml), and concentrated under reduced pressure to recover dichloromethane, when the distillate was no longer evaporated, methanol (100ml) was added for beating at 20°C to 25°C for 5 to 6h, the filter cake after filtration was rinsed with methanol, and blow dried at 45°C to obtain 14.9 g of Compound **III-A** with a yield of 79.2% and a purity of 94.07%.

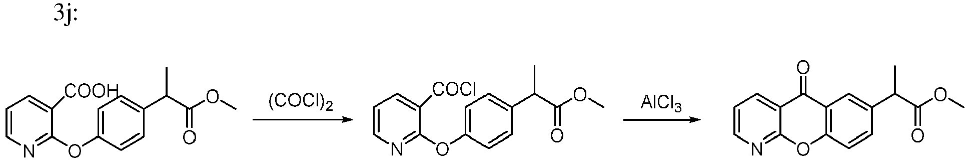

Compound **II-A** (20.0g, lequiv.), dichloromethane (100ml), N,N-dimethylformamide (0.5g) were added into 250ml reaction bottle A, stirred and mixed, then added dropwise with oxalyl chloride (16.9g, 2equiv.) at temperature controlled at 15°C to 25°C, and the temperature was kept at 15°C to 25°C for 1h after the dropwise addition. In addition, anhydrous aluminum trichloride (9.7 g, 1.1 equiv.) and dichloromethane (100 ml) were added into 500 ml reaction bottle B, stirred and mixed. The acyl chloride solution in the reaction bottle A was transferred dropwise into the reaction bottle B at temperature controlled at 15°C to 25°C, the reaction was carried out by keeping the temperature for 1 to 1.5h, and the reaction solution was cooled to -10°C after the reaction was completed. The reaction solution was slowly transferred into cold water to quench at temperature controlled at below 20°C. The reaction solution was stirred for 15 to 30min after the addition, and separated into layers, the dichloromethane phase was retained, the water phase was extracted with dichloromethane (40ml), and separated into layers, the dichloromethane phases were combined, adjusted to pH 7-8 with sodium bicarbonate solution, and separated into layers, and the dichloromethane layer was washed once with purified water (100 ml), and concentrated under reduced pressure to recover dichloromethane, when the distillate was no longer evaporated, methanol (100ml) was added for beating at 20°C to 25°C for 5 to 6h, the filter cake after filtration was rinsed with methanol, and blow dried at 45°C to obtain 14.8 g of Compound **III-A** with a yield of 78.7% and a purity of 94.11%.

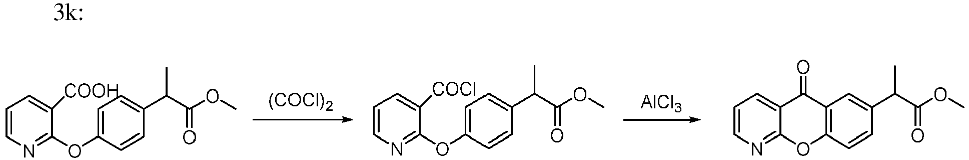

Compound **II-A** (20.0g, lequiv.), dichloromethane (100ml), *N,N*-dimethylformamide (0.5g) were added into 250ml reaction bottle A, stirred and mixed, then added dropwise with oxalyl chloride (25.3g, 3equiv.) at temperature controlled at 15°C to 25°C, and the temperature was kept at 15°C to 25°C for 1h after the dropwise addition. In addition, anhydrous aluminum trichloride (9.7 g, 1.1 equiv.) and dichloromethane (100 ml) were added into 500 ml reaction bottle B, stirred and mixed. The acyl chloride solution in the reaction bottle A was transferred dropwise into the reaction bottle B at temperature controlled at 15°C to 25°C, the reaction was carried out by keeping the temperature for 1 to 1.5h, and the reaction solution was cooled to -10°C after the reaction was completed. The reaction solution was slowly transferred into cold water to quench at temperature controlled at below 20°C. The reaction solution was stirred for 15 to 30min after the addition, and separated into layers, the dichloromethane phase was retained, the water phase was extracted with dichloromethane (40ml), and separated into layers, the dichloromethane phases were combined, adjusted to pH 7-8 with sodium bicarbonate solution, and separated into layers, and the dichloromethane layer was washed once with purified water (100 ml), and concentrated under reduced pressure to recover dichloromethane, when the distillate was no longer evaporated, methanol (100ml) was added for beating at 20°C to 25°C for 5 to 6h, the filter cake after filtration was rinsed with methanol, and blow dried at 45°C to obtain 14.7 g of Compound **III-A** with a yield of 78.2% and a purity of 93.84%.

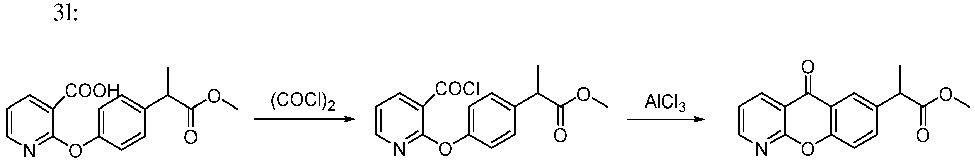

Compound **II-A** (20.0g, lequiv.), dichloromethane (100ml), *N,N*-dimethylformamide (0.5g) were added into 250ml reaction bottle A, stirred and mixed, then added dropwise with oxalyl chloride (12.6g, 1.5equiv.) at temperature controlled at 15°C to 25°C, and the temperature was kept at 15°C to 25°C for 1h after the dropwise addition. In addition, anhydrous aluminum trichloride (13.3 g, 1.5 equiv.) and dichloromethane (100 ml) were added into 500 ml reaction bottle B, stirred and mixed. The acyl chloride solution in the reaction bottle A was transferred dropwise into the reaction bottle B at temperature controlled at 15°C to 25°C, the reaction was carried out by keeping the temperature for 1 to 1.5h, and the reaction solution was cooled to -10°C after the reaction was completed. The reaction solution was slowly transferred into cold water to quench at temperature controlled at below 20°C. The reaction solution was stirred for 15 to 30min after the addition, and separated into layers, the dichloromethane phase was retained, the water phase was extracted with dichloromethane (40ml), and separated into layers, the dichloromethane phases were combined, adjusted to pH 7-8 with sodium bicarbonate solution, and separated into layers, and the dichloromethane layer was washed once with purified water (100 ml), and concentrated under reduced pressure to recover dichloromethane, when the distillate was no longer evaporated, methanol (100ml) was added for beating at 20°C to 25°C for 5 to 6h, the filter cake after filtration was rinsed with methanol, and blow dried at 45°C to obtain 15.4 g of Compound **III-A** with a yield of 81.8% and a purity of 98.57%.

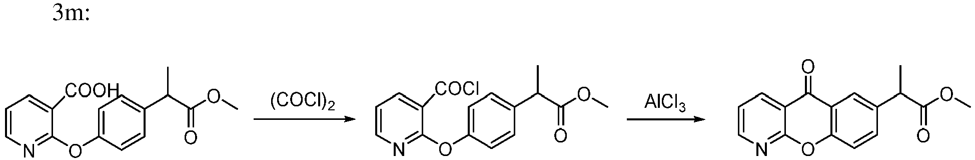

Compound **II-A** (20.0g, lequiv.), dichloromethane (100ml), N,N-dimethylformamide (0.5g) were added into 250ml reaction bottle A, stirred and mixed, added dropwise with oxalyl chloride (12.6g, 1.5equiv.) at temperature controlled at 15°C to 25°C, and the temperature was kept at 15°C to 25°C for 1h after the dropwise addition. In addition, anhydrous aluminum trichloride (44.3 g, 5 equiv.) and dichloromethane (100 ml) were added into 500 ml reaction bottle B, stirred and mixed. The acyl chloride solution in the reaction bottle A was transferred dropwise into the reaction bottle B at temperature controlled at 15°C to 25°C, the reaction was carried out by keeping the temperature for 1 to 1.5h, and the reaction solution was cooled to -10°C after the reaction was completed. The reaction solution was slowly transferred into cold water to quench at temperature controlled at below 20°C. The reaction solution was stirred for 15 to 30min after the addition, and separated into layers, the dichloromethane phase was retained, the water phase was extracted with dichloromethane (40ml), and separated into layers, the dichloromethane phases were combined, adjusted to pH 7-8 with sodium bicarbonate solution, and separated into layers, and the dichloromethane layer was washed once with purified water (100 ml), and concentrated under reduced pressure to recover dichloromethane, when the distillate was no longer evaporated, methanol (100ml) was added for beating at 20°C to 25°C for 5 to 6h, the filter cake after filtration was rinsed with methanol, and blow dried at 45°C to obtain 16.0 g of Compound **III-A** with a yield of 85.1% and a purity of 98.71%.

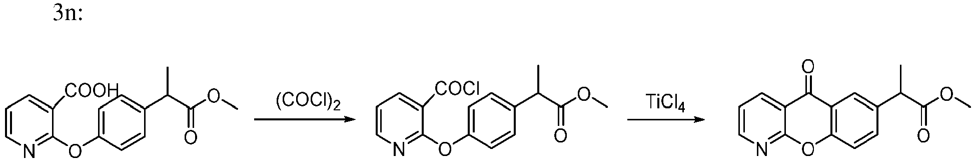

Compound **II-A** (20.0g, lequiv.), dichloromethane (100ml), N,N-dimethylformamide (0.5g) were added into 250ml reaction bottle A, stirred and mixed, then added with thionyl chloride (11.8g, 1.5equiv.) at temperature controlled at 15°C to 25°C, and the temperature was kept at 15°C to 25°C for 1h after the dropwise addition. In addition, titanium tetrachloride (25.2 g, 2 equiv.) and dichloromethane (100 ml) were put into 500 ml reaction bottle B, and stirred and mixed. The acyl chloride solution in the reaction bottle A was transferred dropwise into the reaction bottle B at temperature controlled at 15°C to 25°C, the reaction was carried out by keeping the temperature for 1 to 1.5h, and the reaction solution was cooled to -10°C after the reaction was completed. The reaction solution was slowly transferred into cold water to quench at temperature controlled at below 20°C. The reaction solution was stirred for 15 to 30min after the addition, and separated into layers, the dichloromethane phase was retained, the water phase was extracted with dichloromethane (40ml), and separated into layers, the dichloromethane phases were combined, adjusted to pH 7-8 with sodium bicarbonate solution, and separated into layers, and the dichloromethane layer was washed once with purified water (100 ml), and concentrated under reduced pressure to recover dichloromethane, when the distillate was no longer evaporated, methanol (100ml) was added for beating at 20°C to 25°C for 5 to 6h, the filter cake after filtration was rinsed with methanol, and blow dried at 45°C to obtain 13.6 g of Compound III-A with a yield of 72.4% and a purity of 95.43%.

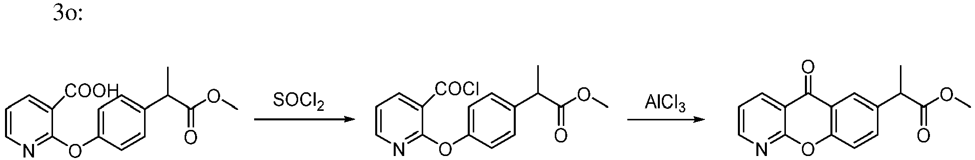

Compound **II-A** (20.0g, lequiv.), dichloromethane (100ml), *N,N*-dimethylformamide (0.5g) were added into 250ml reaction bottle A, stirred and mixed, then added with thionyl chloride (11.8, 1.5 equiv.) at temperature controlled at 15°C to 25°C, and the temperature was kept at 15°C to 25°C for 1h after the dropwise addition. In addition, anhydrous aluminum trichloride (17.7 g, 2 equiv.) and dichloromethane (100 ml) were added into 500 ml reaction bottle B, stirred and mixed. The acyl chloride solution in the reaction bottle A was transferred dropwise into the reaction bottle B at temperature controlled at 15°C to 25°C, the reaction was carried out by keeping the temperature for 1 to 1.5h, and the reaction solution was cooled to -10°C after the reaction was completed. The reaction solution was slowly transferred into cold water to quench at temperature controlled at below 20°C. The reaction solution was stirred for 15 to 30min after the addition, and separated into layers, the dichloromethane phase was retained, the water phase was extracted with dichloromethane (40ml), and separated into layers, the dichloromethane phases were combined, adjusted to pH 7-8 with sodium bicarbonate solution, and separated into layers, and the dichloromethane layer was washed once with purified water (100 ml), and concentrated under reduced pressure to recover dichloromethane, when the distillate was no longer evaporated, methanol/dichloromethane mixed solvent (100ml, solvent ratio 1:0.3) was added for beating at 20°C to 25°C for 5 to 6h, the filter cake after filtration was rinsed with methanol, and blow dried at 45°C to obtain 15.0 g of Compound **III-A** with a yield of 79.9% and a purity of 99.78%.

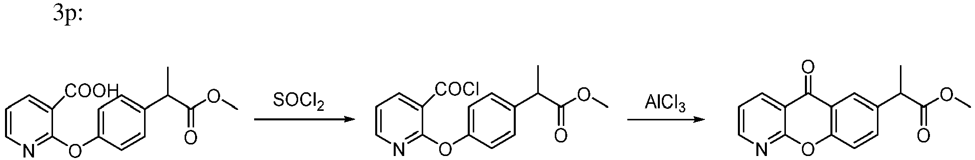

Compound **II-A** (20.0g, lequiv.), dichloromethane (100ml), *N,N*-dimethylformamide (0.5g) were added into 250ml reaction bottle A, stirred and mixed, then added with thionyl chloride (11.8, 1.5 equiv.) at temperature controlled at 15°C to 25°C, and the temperature was kept at 15°C to 25°C for 1h after the dropwise addition. In addition, anhydrous aluminum trichloride (17.7 g, 2 equiv.) and dichloromethane (100 ml) were added into 500 ml reaction bottle B, stirred and mixed. The acyl chloride solution in the reaction bottle A was transferred dropwise into the reaction bottle B at temperature controlled at 15°C to 25°C, the reaction was carried out by keeping the temperature for 1 to 1.5h, and the reaction solution was cooled to -10°C after the reaction was completed. The reaction solution was slowly transferred into cold water to quench at temperature controlled at below 20°C. The reaction solution was stirred for 15 to 30min, separated into layers, the dichloromethane phase was retained, the aqueous phase was extracted with dichloromethane (40ml), and separated into layers, the dichloromethane phases were combined, adjusted to pH 7-8 with sodium bicarbonate solution, and separated into layers, and the dichloromethane layer was washed once with purified water (100 ml), and concentrated under reduced pressure to recover dichloromethane, when the distillate was no longer evaporated, methanol/dichloromethane mixed solvent (100ml, solvent ratio 1:0.4) was added for beating at 20°C to 25°C for 5 to 6h, the filter cake after filtration was rinsed with methanol, and blow dried at 45°C to obtain 14.5 g of Compound **III-A** with a yield of 77.0% and a purity of 99.81%.

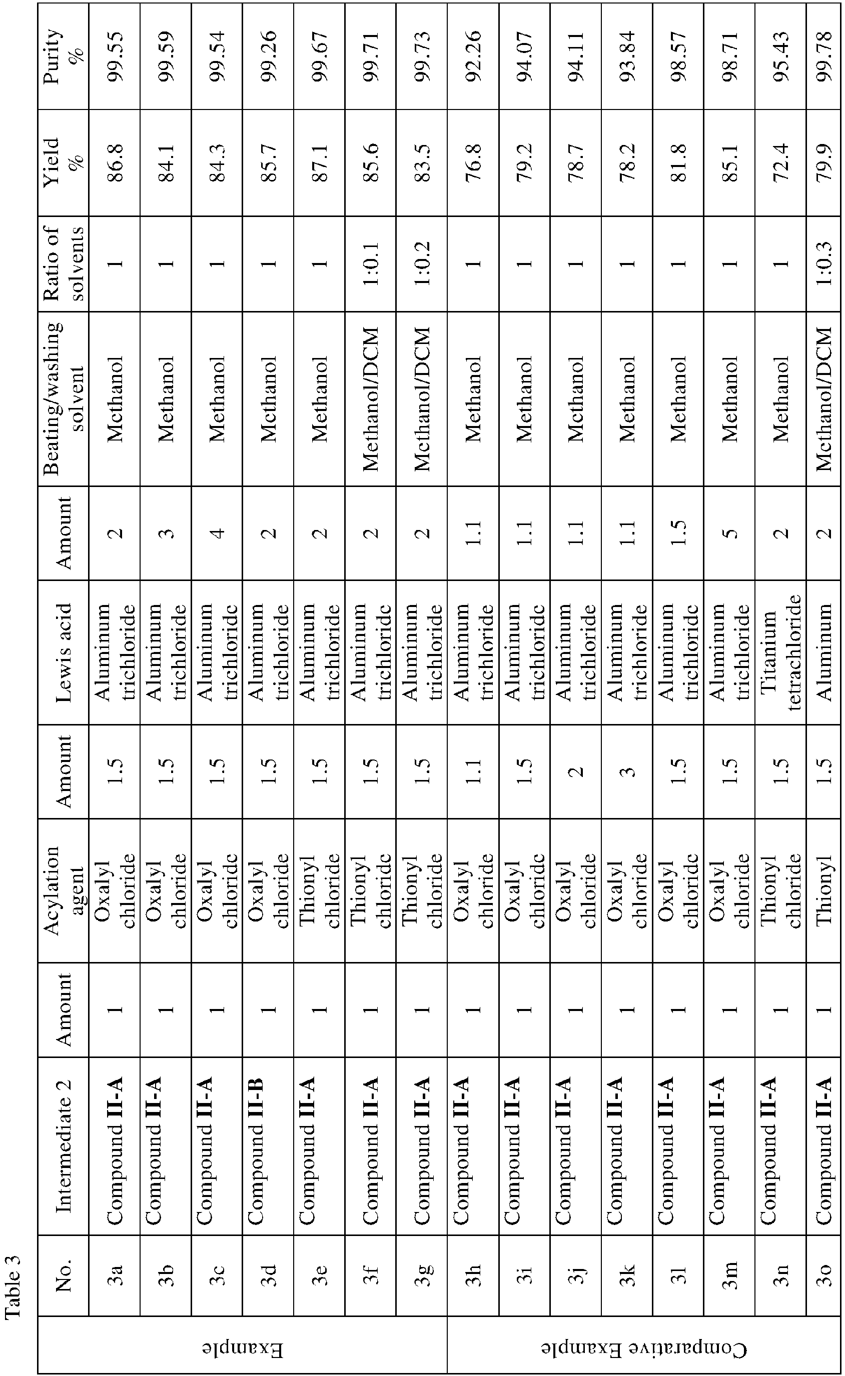

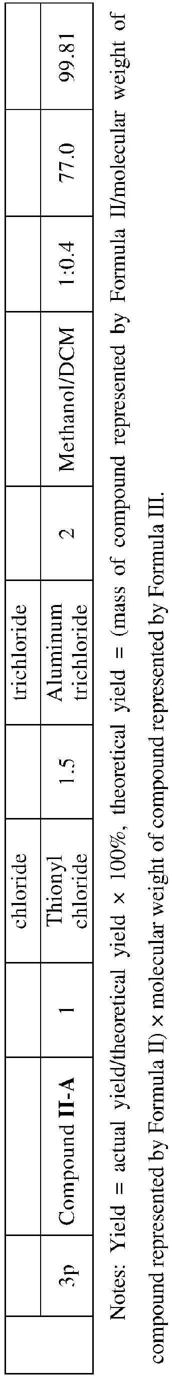

### Preparation Examples 4a to 4c: Preparation of methyl 2-(10-hydroxyl-9-oxa-1-azaanthracen-6-yl)propionate (Compound IV-A) and ethyl 2-(10-hydroxyl-9-oxo-1-azaanthracen-6-yl)propionate (Compound IV-B)

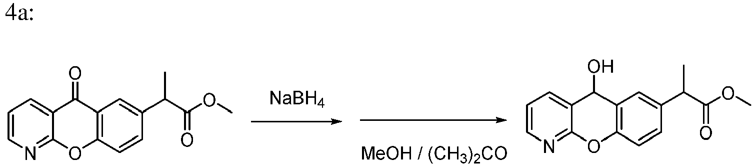

Compound **III-A** (20.0g, 1 equiv.), dichloromethane (80ml) and methanol (80ml) were added to a 500ml reaction flask, stirred for dissolution at 15°C to 30°C for 5 to 10min to obtain a clear solution, slowly added with sodium borohydride ( 2.7g, 1 equiv.) within about 30min, the temperature was kept at 25°C to 30°C for 8h, then lowered to -5°C to 5°C, 30% acetic acid aqueous solution was slowly added dropwise to pH5.5-6.5, after the dropwise addition, the reaction solution was stirred at -5°C to 5°C for 15 minutes, added with purified water (120ml), stirred and separated into layers, and the organic phase was separated out; the aqueous phase was extracted once with dichloromethane (40ml), the dichloromethane phases were combined, washed once with saturated aqueous sodium bicarbonate (20ml); and washed again with purified water (40ml). The resulting dichloromethane solution was concentrated to dryness (-0.09Mpa) under reduced pressure at 35°C to obtain a yellow-green oil (20.0g), which was added to a mixed solution of methanol/acetone (50ml/50ml), dissolved and dispersed at 25°C to 30°C under stirring for 15 minutes to obtain a yellow-green clear solution. Purified water (200ml) was added dropwise for crystallization for 2 hours, the filter cake after suction filtration was washed with purified water, and dried under vacuum at 40°C in the dark to obtain 16.4 g of Compound **IV-A** with a yield of 81.5% and a purity of 96.72%.

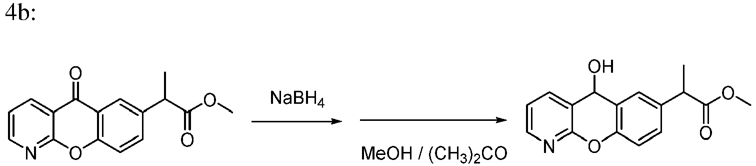

Compound **III-A** (20.0g, 1 equiv.), dichloromethane (80ml) and methanol (80ml) were added to a 500ml reaction flask, stirred for dissolution at 15°C to 30°C for 5 to 10min to obtain a clear solution, slowly added with sodium borohydride ( 2.7g, 1 equiv.) within about 30min, after the addition, the temperature was kept at 35°C to 40°C for 6h, then lowered to -5°C to 5°C, 30% acetic acid aqueous solution was slowly added dropwise to pH5.5-6.5, after the dropwise addition, the reaction solution was stirred at -5°C to 5°C for 15 minutes, added with purified water (120ml), stirred and separated into layers, and the organic phase was separated out; the aqueous phase was extracted once with dichloromethane (40ml), the dichloromethane phases were combined, washed once with saturated aqueous sodium bicarbonate (20ml); and washed again with purified water (40ml). The resulting dichloromethane solution was concentrated to dryness (-0.09Mpa) under reduced pressure at 35°C to obtain a yellow-green oil (20.0g), which was added to a mixed solution of methanol/acetone (50ml/50ml), dissolved and dispersed at 25°C to 30°C under stirring for 15 minutes to obtain a yellow-green clear solution. Purified water (200ml) was added dropwise for crystallization for 2h, the filter cake after suction filtration was washed with purified water, and dried under vacuum at 40°C in the dark to obtain 16.6 g of Compound **IV-A** with a yield of 82.5% and a purity of 98.46%.

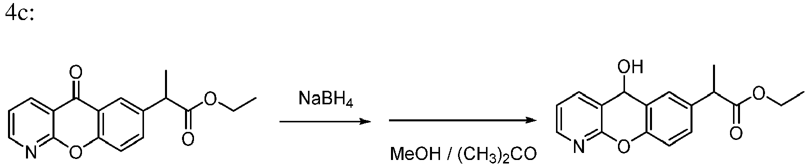

Compound **III-B** (30.0g, 1 equiv.), dichloromethane (120ml) and methanol (120ml) were added to a 500ml reaction flask, stirred for dissolution at 15°C to 30°C for 5 to 10min to obtain a clear solution, slowly added with sodium borohydride (3.8 g, 1 equiv.) within about 30 minutes, after the addition, the temperature was kept at 35°C to 40°C for 6h, then lowered to -5°C to 5°C, the reaction solution was transferred to a 1L beaker and slowly added dropwise with 30% acetic acid aqueous solution to pH 5.5-6.5, after the dropwise addition, the reaction solution was stirred at -5°C to 5°C for 15 minutes, added with purified water (180ml), stirred and separated into layers, the organic phase was separated out; the water phase was extracted once with dichloromethane (60ml), the dichloromethane phases were combined, washed once with saturated aqueous sodium bicarbonate solution (30ml); and washed once again with purified water (60ml). The obtained dichloromethane solution was concentrated to dryness (-0.09Mpa) under reduced pressure at 35°C to obtain a yellow-green oil (30.0g), which was added to a mixed solution of methanol/acetone (75ml/75ml), dissolved and dispersed at 20°C to 30°C under stirring for 15 minutes to obtain a yellow-green clear solution. Purified water (300ml) was slowly added dropwise for crystallization for 2 hours, the filter cake after suction filtration was washed with purified water, and dried under vacuum at 40°C in the dark to obtain 24.1 g of Compound **IV-B** with a yield of 79.8% and a purity of 98.77%.

### Comparative Examples 4d to 41

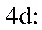

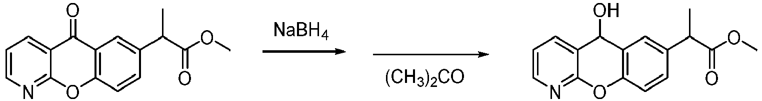

Compound **III-A** (20.0g, 1 equiv.), dichloromethane (80ml) and methanol (80ml) were added to a 500ml reaction flask, stirred for dissolution at 15°C to 30°C for 5 to 10min to obtain a clear solution, slowly added with sodium borohydride ( 1.3g, 0.5equiv.) within about 30min, after the addition, the temperature was kept at 15°C to 20°C for 12h, then lowered to -5°C to 5°C, 30% acetic acid aqueous solution was slowly added dropwise to pH5.5-6.5, after the addition, the reaction was stirred at -5°C to 5°C for 15 minutes, added with purified water (120ml), stirred and separated into layers, and the organic phase was separated out; the aqueous phase was extracted once with dichloromethane (40ml), and the dichloromethane phases were combined, washed once with saturated aqueous sodium bicarbonate (20ml); and wash once again with purified water (40ml). The resulting dichloromethane solution was concentrated to dryness (-0.09Mpa) under reduced pressure at 35°C to obtain a yellow-green oil (20.0g), which was added into acetone (100ml), dissolved and dispersed at 25°C to 30°C under stirring for 15min to obtain a yellow-green clear solution. Purified water (200ml) was added dropwise for crystallization for 2h, the filter cake after suction filtration was washed with purified water, and dried under vacuum at 40°C in the dark to obtain 15.0 g of Compound **IV-A** with a yield of 74.7% and a purity of 89.86%.

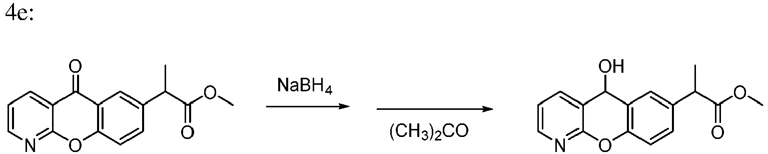

Compound **III-A** (20.0g, 1 equiv.), dichloromethane (80ml) and methanol (80ml) were added to a 500ml reaction flask, stirred for dissolution at 15°C to 30°C for 5 to 10min to obtain a clear solution, slowly added with sodium borohydride ( 1.6g, 0.6equiv.) within about 30min, after the addition, the temperature was kept at 15°C to 20°C for 12h, then lowered to -5°C to 5°C, 30% acetic acid aqueous solution was slowly added dropwise to pH5.5-6.5, after the dropwise addition, the reaction solution was stirred at -5°C to 5°C for 15 minutes, added with purified water (120ml), stirred and separated into layers, and the organic phase was separated out; the aqueous phase was extracted once with dichloromethane (40ml), and the dichloromethane phases were combined, washed once with saturated aqueous sodium bicarbonate (20ml); and washed once again with purified water (40ml). The resulting dichloromethane solution was concentrated to dryness (-0.09Mpa) under reduced pressure at 35°C to obtain a yellow-green oil (20.0g), which was added into acetone (100ml), dissolved and dispersed at 25°C to 30°C under stirring for 15min to obtain a yellow-green clear solution. Purified water (200ml) was added dropwise for crystallization for 2 hours, the filter cake after suction filtration was washed with purified water, and dried under vacuum at 40°C in the dark to obtain 15.4 g of Compound **IV-A** with a yield of 76.4% and a purity of 92.58%.

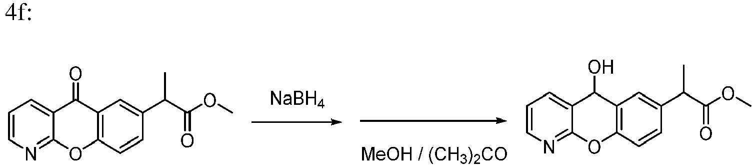

Compound **III-A** (20.0g, 1 equiv.), dichloromethane (80ml) and methanol (80ml) were added to a 500ml reaction flask, stirred for dissolution at 15°C to 30°C for 5 to 10min to obtain a clear solution, slowly added with sodium borohydride ( 2.7g, 1 equiv.) within about 30min, after the addition, the temperature was kept at 15°C to 20°C for 10h, then lowered to -5°C to 5°C, 30% acetic acid aqueous solution was slowly added dropwise to pH5.5-6.5, after the dropwise addition, the reaction solution was stirred at -5°C to 5°C for 15 minutes, added with purified water (120ml), stirred and separated into layers, and the organic phase was separated out; the aqueous phase was extracted once with dichloromethane (40ml), the dichloromethane phases were combined, washed once with saturated aqueous sodium bicarbonate (20ml); and washed again with purified water (40ml). The resulting dichloromethane solution was concentrated to dryness (-0.09Mpa) under reduced pressure at 35°C to obtain a yellow-green oil (20.0g), which was added into acetone (100ml), dissolved and dispersed at 25°C to 30°C under stirring for 15min to obtain a yellow-green clear solution. Purified water (200ml) was added dropwise for crystallization for 2 hours, the filter cake after suction filtration was washed with purified water, and dried under vacuum at 40°C in the dark to obtain 16.4 g of Compound **IV-A** with a yield of 81.4% and a purity of 94.86%.

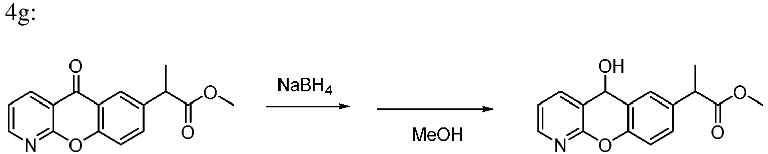

Compound **III-A** (20.0g, 1 equiv.), dichloromethane (80ml) and methanol (80ml) were added to a 500ml reaction flask, stirred for dissolution at 15°C to 30°C for 5 to 10min to obtain a clear solution, slowly added with sodium borohydride ( 2.7g, 1 equiv.) within about 30min, after the addition, the temperature was kept at 15°C to 20°C for 10h, then lowered to -5°C to 5°C, 30% acetic acid aqueous solution was slowly added dropwise to pH5.5-6.5, after the dropwise addition, the reaction solution was stirred at -5°C to 5°C for 15 minutes, added with purified water (120ml), stirred and separated into layers, and the organic phase was separated out; the aqueous phase was extracted once with dichloromethane (40ml), the dichloromethane phases were combined, washed once with saturated aqueous sodium bicarbonate (20ml); and washed again with purified water (40ml). The obtained dichloromethane solution was concentrated to dryness (-0.09Mpa) under reduced pressure at 35°C to obtain a yellow-green oil (20.0g), which was added into methanol (100ml), dissolved and dispersed at 25°C to 30°C under stirring for 15min to obtain a yellow-green clear solution. Purified water (200ml) was added dropwise for crystallization for 2 hours, the filter cake after suction filtration was washed with purified water, and dried under vacuum at 40°C in the dark to obtain 17.8 g of Compound **IV-A** with a yield of 83.5% and a purity of 93.97%.

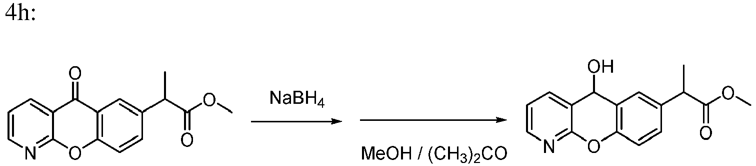

Compound **III-A** (20.0g, 1 equiv.), dichloromethane (80ml) and methanol (80ml) were added to a 500ml reaction flask, stirred for dissolution at 15°C to 30°C for 5 to 10min to obtain a clear solution, slowly added with sodium borohydride ( 2.7g, 1 equiv.) within about 30min, after the addition, the temperature was kept at 15°C to 20°C for 10h, then lowered to -5°C to 5°C, 30% acetic acid aqueous solution was slowly added dropwise to pH5.5-6.5, after the dropwise addition, the reaction solution was stirred at -5°C to 5°C for 15 minutes, added with purified water (120ml), stirred and separated into layers, and the organic phase was separated out; the aqueous phase was extracted once with dichloromethane (40ml), the dichloromethane phases were combined, washed once with saturated aqueous sodium bicarbonate (20ml); and washed again with purified water (40ml). The obtained dichloromethane solution was concentrated to dryness (-0.09Mpa) under reduced pressure at 35°C to obtain a yellow-green oil (20.0g), which was added to a mixed solution of methanol/acetone (50ml/50ml), dissolved and dispersed at 25°C to 30°C under stirring for 15 minutes to obtain a yellow-green clear solution. Purified water (200ml) was added dropwise for crystallization for 2h, the filter cake after suction filtration was washed with purified water, and dried under vacuum at 40°C in the dark to obtain 16.3 g of Compound **IV-A** with a yield of 81.0% and a purity of 95.23%.

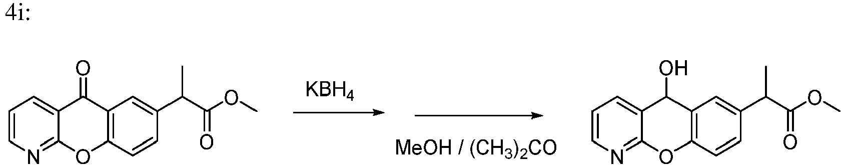

Compound **III-A** (20.0g, 1 equiv.), dichloromethane (80ml) and methanol (80ml) were added to a 500ml reaction flask, stirred for dissolution at 15°C to 30°C for 5 to 10min to obtain a clear solution, slowly added with potassium borohydride ( 3.8g, 1 equiv.) within about 30min, after the addition, the temperature was kept at 15°C to 20°C for 10h, then lowered to -5°C to 5°C, 30% acetic acid aqueous solution was slowly added dropwise to pH5.5-6.5, after the dropwise addition, the reaction solution was stirred at -5°C to 5°C for 15 minutes, added with purified water (120ml), stirred and separated into layers, and the organic phase was separated out; the aqueous phase was extracted once with dichloromethane (40ml), the combined dichloromethane phases were combined, washed once with saturated aqueous sodium bicarbonate (20ml); and washed again with purified water (40ml). The obtained dichloromethane solution was concentrated to dryness (-0.09Mpa) under reduced pressure at 35°C to obtain a yellow-green oil (20.0g), which was added to a mixed solution of methanol/acetone (50ml/50ml), dissolved and dispersed at 25°C to 30°C under stirring for 15 minutes to obtain a yellow-green clear solution. Purified water (200ml) was slowly added dropwise for crystallization for 2h, the filter cake after suction filtration was washed with purified water, and dried under vacuum at 40°C in the dark to obtain 16.1 g of Compound **IV-A** with a yield of 80.0% and a purity of 95.28%.

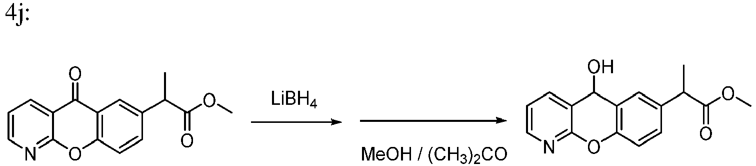

Compound **III-A** (20.0g, 1 equiv.), dichloromethane (80ml) and methanol (80ml) were added to a 500ml reaction flask, stirred for dissolution at 15°C to 30°C for 5 to 10min to obtain a clear solution, slowly added with lithium borohydride ( 3.8g, 1 equiv.) within about 30min, after the addition, the temperature was kept at 15°C to 20°C for 10h, then lowered to -5°C to 5°C, 30% acetic acid aqueous solution was slowly added dropwise to pH5.5-6.5, after the dropwise addition, the reaction solution was stirred at -5°C to 5°C for 15 minutes, added with purified water (120ml), stirred and separated into layers, and the organic phase was separated out; the aqueous phase was extracted once with dichloromethane (40ml), the dichloromethane phases were combined, washed once with saturated aqueous sodium bicarbonate (20ml); and washed again with purified water (40ml). The obtained dichloromethane solution was concentrated to dryness (-0.09Mpa) under reduced pressure at 35°C to obtain a yellow-green oil (20.0g), which was added to a mixed solution of methanol/acetone (50ml/50ml), dissolved and dispersed at 25°C to 30°C under stirring for 15 minutes to obtain a yellow-green clear solution. Purified water (200ml) was added dropwise for crystallization for 2 hours, the filter cake after suction filtration was washed with purified water, and dried under vacuum at 40°C in the dark to obtain 15.9 g of Compound **IV-A** with a yield of 78.7% and a purity of 94.03%.

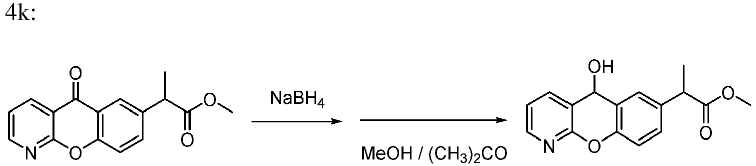

Compound **III-A** (20.0g, 1 equiv.), dichloromethane (80ml) and methanol (80ml) were added to a 500ml reaction flask, stirred for dissolution at 15°C to 30°C for 5 to 10min to obtain a clear solution, slowly added with sodium borohydride ( 3.8g, 1 equiv.) within about 30min, after the addition, the temperature was kept at 20°C to 25°C for 8h, then lowered to -5°C to 5°C, 30% acetic acid aqueous solution was slowly added dropwise to pH5.5-6.5, after the dropwise addition, the reaction solution was stirred at -5°C to 5°C for 15 minutes, added with purified water (120ml), stirred and separated into layers, and the organic phase was separated out; the aqueous phase was extracted once with dichloromethane (40ml), the dichloromethane phases were combined, washed once with saturated aqueous sodium bicarbonate (20ml); and washed again with purified water (40ml). The obtained dichloromethane solution was concentrated to dryness (-0.09Mpa) under reduced pressure at 35°C to obtain a yellow-green oil (20.0g), which was added to a mixed solution of methanol/acetone (50ml/50ml), dissolved and dispersed at 25°C to 30°C under stirring for 15 minutes to obtain a yellow-green clear solution. Purified water (200ml) was added dropwise for crystallization for 2 hours, the filter cake after suction filtration was washed with purified water, and dried under vacuum at 40°C in the dark to obtain 16.4 g of Compound **IV-A** with a yield of 81.5% and a purity of 95.86%.

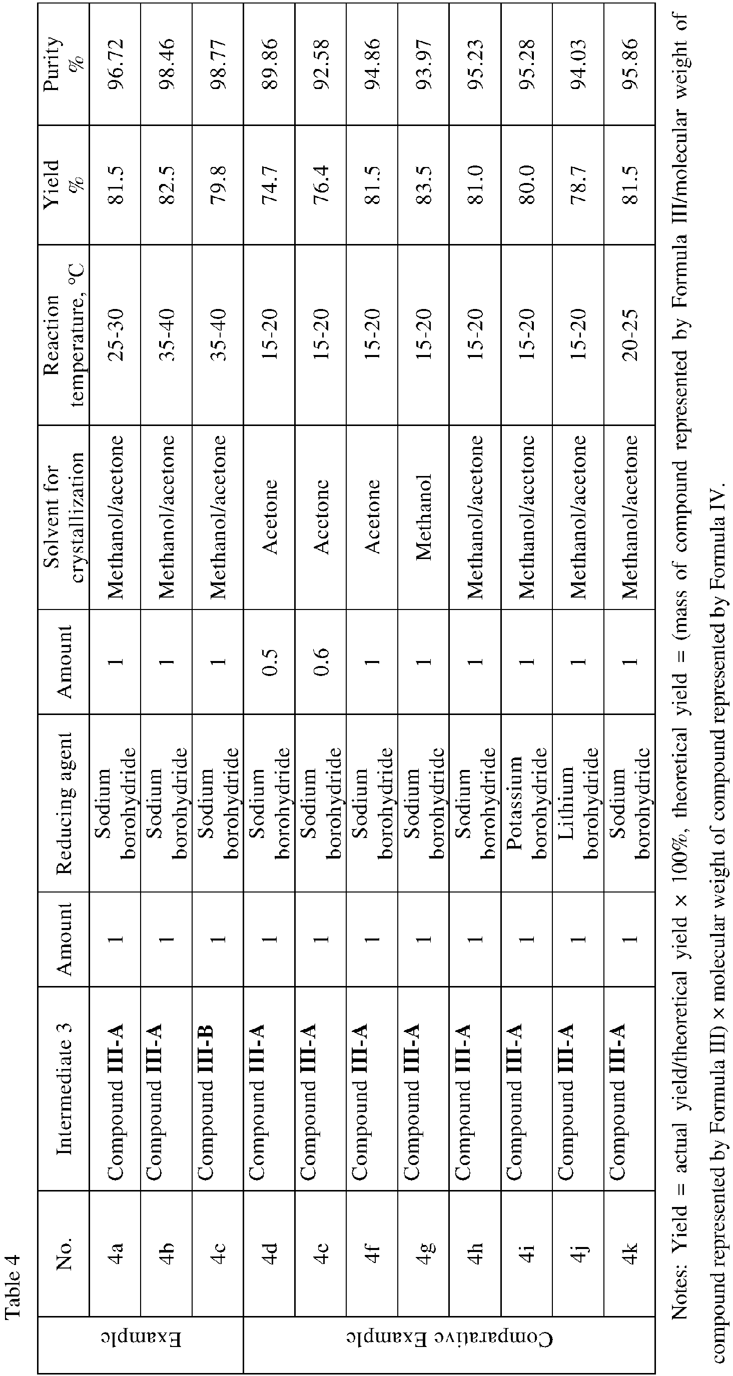

### Preparation Examples 5a to 5g: Preparation of pranoprofen

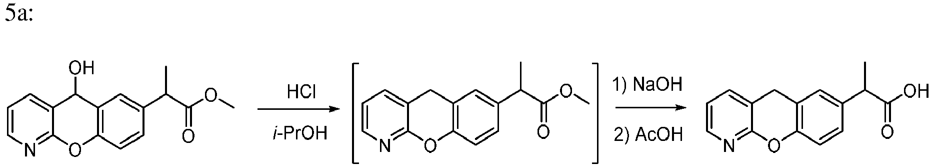

Compound **IV-A** (20.0g, lequiv.), hydrochloric acid (24.8g, 3.5equiv.), isopropanol (240ml) were added into a 250ml reaction flask, stirred for dissolution and heated to 80°C to 85°C for reaction under refluxing for 2h, and concentrated at 55°C under reduced pressure until no distillate was distilled off to obtain a foamy oil (24 g). After cooling to room temperature, methanol (10ml) was added, stirred for dissolution, then added with a sodium hydroxide solution prepared with sodium hydroxide (11.2g, 4 equiv.) and purified water (40ml), and reacted at 50°C to 55°C for 2h. The reaction solution was transferred to a 500ml beaker, added with purified water (300ml), then the aqueous phase was extracted with ethyl acetate (100ml) 4 times, after the aqueous phase was separated out, it was adjusted with 20% acetic acid aqueous solution to pH 5-6, and gradually precipitated a white powdery solid. The filter cake after filtration was rinsed with purified water and cold methanol in turn, and dried in vacuum at 40°C to obtain 14.3 g of crude pranoprofen with a yield of about 79.9% and a purity of 99.62%. The crude product of pranoprofen was recrystallized with methanol to obtain a finished product of pranoprofen with a purity of >99.9% and a single impurity content of less than 0.05%.

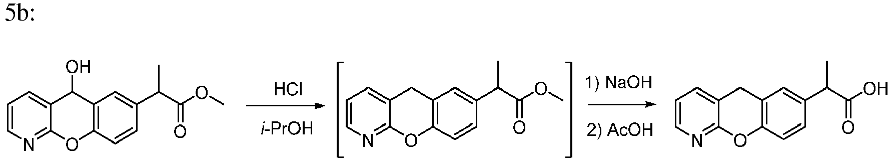

Compound **IV-A** (20.0 g, 1 equiv.), hydrochloric acid (24.8 g, 3.5 equiv.), isopropanol (240 ml) were added into a 250 ml reaction flask, stirred for dissolution and heated to 60°C to 65°C for reaction for 2.5h, and concentrated at 55°C under reduced pressure until no distillate was distilled off to obtain a foamy oil (24 g). After cooling to room temperature, methanol (10ml) was added, stirred for dissolution, then added with a sodium hydroxide solution prepared with sodium hydroxide (11.2g, 4 equiv.) and purified water (40ml), and reacted at 50°C to 55°C for 2h. The reaction solution was transferred to a 500ml beaker, added with purified water (300ml), then the aqueous phase was extracted with ethyl acetate (100ml) 4 times, after the aqueous phase was separated out, it was adjusted with 20% acetic acid aqueous solution to pH 5-6, and gradually precipitated a white powdery solid. The filter cake after filtration was rinsed with purified water and cold methanol in turn, and dried in vacuum at 40°C to obtain 14.4 g of crude pranoprofen with a yield of about 80.5% and a purity of 99.27%. The crude product of pranoprofen was recrystallized with methanol to obtain a finished product of pranoprofen with a purity of >99.9% and a single impurity content of less than 0.05%.

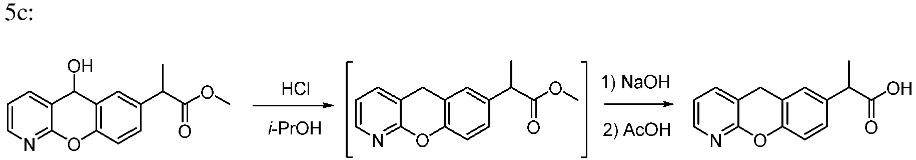

Compound **IV-A** (20.0g, 1 equiv.), hydrochloric acid (24.8g, 3.5 equiv.), isopropanol (240ml) were added into a 250ml reaction flask, stirred for dissolution and heated to 70°C to 75°C for reaction for 2.5h, and concentrated at 55°C under reduced pressure until no distillate was distilled off to obtain a foamy oil (24 g). After cooling to room temperature, methanol (10ml) was added, stirred for dissolution, then added with a sodium hydroxide solution prepared with sodium hydroxide (11.2g, 4 equiv.) and purified water (40ml), and reacted at 50°C to 55°C for 2h. The reaction solution was transferred to a 500ml beaker, added with purified water (300ml), then the aqueous phase was extracted with ethyl acetate (100ml) 4 times, after the aqueous phase was separated out, it was adjusted with 20% acetic acid aqueous solution to pH 5-6, and gradually precipitated a white powdery solid. The filter cake after filtration was rinsed with purified water and cold methanol in turn, and dried in vacuum at 40°C to obtain 14.3 g of crude pranoprofen with a yield of about 79.9% and a purity of 99.48%. The crude product of pranoprofen was recrystallized with methanol to obtain a finished product of pranoprofen with a purity of >99.9% and a single impurity content of less than 0.05%.

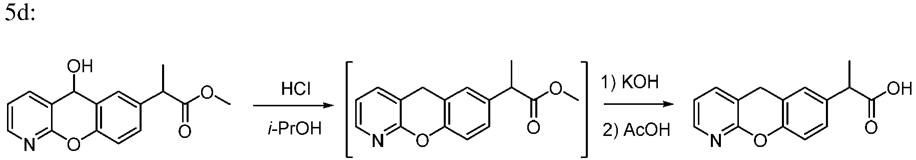

Compound **IV-A** (20.0g, lequiv.), hydrochloric acid (24.8g, 3.5equiv.), isopropanol (240ml) were added into a 250ml reaction flask, stirred for dissolution and heated to 80°C to 85°C for reaction under refluxing for 2h, and concentrated at 55°C under reduced pressure until no distillate was distilled off to obtain a foamy oil (24 g). After cooling to room temperature, methanol (10ml) was added, stirred for dissolution, then added with a potassium hydroxide solution prepared with potassium hydroxide (15.7g, 4 equiv.) and purified water (40ml), and reacted at 50°C to 55°C for 2h. The reaction solution was transferred to a 500ml beaker, added with purified water (300ml), then the aqueous phase was extracted with ethyl acetate (100ml) 4 times, after the aqueous phase was separated out, it was adjusted with 20% acetic acid aqueous solution to pH 5-6, and gradually precipitated a white powdery solid. The filter cake after filtration was rinsed with purified water and cold methanol in turn, and dried in vacuum at 40°C to obtain 14.2 g of crude pranoprofen with a yield of about 79.4% and a purity of 99.63%. The crude product of pranoprofen was recrystallized with methanol to obtain a finished product of pranoprofen with a purity of >99.9% and a single impurity content of less than 0.05%.

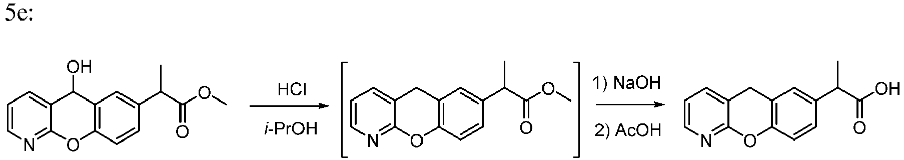

Compound **IV-A** (20.0g, lequiv.), hydrochloric acid (24.8g, 3.5equiv.), isopropanol (240ml) were added into a 250ml reaction flask, stirred for dissolution and heated to 80°C to 85°C for reaction under refluxing for 2h, and concentrated at 55°C under reduced pressure until no distillate was distilled off to obtain a foamy oil (24 g). After cooling to room temperature, methanol (10ml) was added, stirred for dissolution, then added with a sodium hydroxide solution prepared with sodium hydroxide (11.2g, 4equiv.) and purified water (40ml), and reacted at 40°C to 45°C for 2h. The reaction solution was transferred to a 500ml beaker, added with purified water (300ml), then the aqueous phase was extracted with ethyl acetate (100ml) 4 times, after the aqueous phase was separated out, it was adjusted with 20% acetic acid aqueous solution to pH 5-6, and gradually precipitated a white powdery solid. The filter cake after filtration was rinsed with purified water and cold methanol in turn, and dried in vacuum at 40°C to obtain 14.6 g of crude pranoprofen with a yield of about 81.6% and a purity of 99.68%. The crude product of pranoprofen was recrystallized with methanol to obtain a finished product of pranoprofen with a purity of >99.9% and a single impurity content of less than 0.05%.

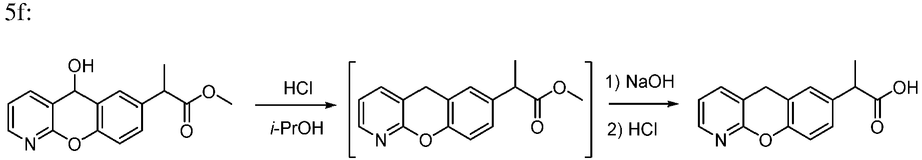

Compound **IV-A** (20.0g, lequiv.), hydrochloric acid (24.8g, 3.5equiv.), isopropanol (240ml) were added into a 250ml reaction flask, stirred for dissolution and heated to 80°C to 85°C for reaction under refluxing for 2h, and concentrated at 55°C under reduced pressure until no distillate was distilled off to obtain a foamy oil (24 g). After cooling to room temperature, methanol (10ml) was added, stirred for dissolution, then added with a sodium hydroxide solution prepared with sodium hydroxide (11.2g, 4equiv.) and purified water (40ml), and reacted at 40°C to 45°C for 2h. The reaction solution was transferred to a 500ml beaker, added with purified water (300ml), then the aqueous phase was extracted with ethyl acetate (100ml) 4 times, after the aqueous phase was separated out, it was adjusted with 10% dilute hydrochloric acid to pH 5-6, and gradually precipitated a white powdery solid. The filter cake after filtration was rinsed with purified water and cold methanol in turn, and dried in vacuum at 40°C to obtain 14.6 g of crude pranoprofen with a yield of about 81.6% and a purity of 99.67%. The crude product of pranoprofen was recrystallized with methanol to obtain a finished product of pranoprofen with a purity of >99.9% and a single impurity content of less than 0.05%.

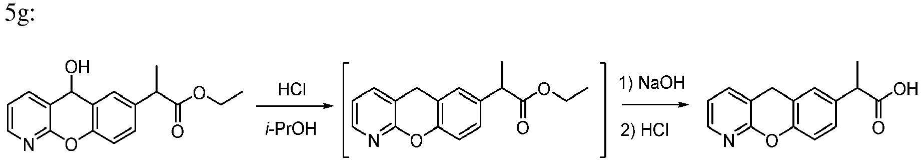

Compound **IV-B** (20.0g, 1 equiv.), hydrochloric acid (23.8g, 3.5 equiv.), isopropanol (240ml) were added into a 250ml reaction flask, stirred for dissolution, heated to 80°C to 85°C for reaction under refluxing for 2h, and concentrated at 55°C under reduced pressure until no distillate was distilled off to obtain a foamy oil (24 g). After cooling to room temperature, methanol (10ml) was added, stirred for dissolution, then added with a sodium hydroxide solution prepared with sodium hydroxide (10.7g, 4 equiv.) and purified water (40ml), and reacted at 40°C to 45°C for 2h. The reaction solution was transferred to a 500ml beaker, added with purified water (300ml), then the water phase was extracted with ethyl acetate (100ml) for 4 times, after the water phase was separated out, it was adjusted with 10% dilute hydrochloric acid to pH 5-6, and gradually precipitated a white powdery solid. The filter cake after filtration was rinsed with purified water and cold methanol in turn, and dried in vacuum at 40°C to obtain 13.6 g of crude pranoprofen with a yield of about 79.5% and a purity of 99.62%. The crude product of pranoprofen was recrystallized with methanol to obtain a finished product of pranoprofen with a purity of >99.9% and a single impurity content of less than 0.05%.

### Comparative Examples 5h to 5t

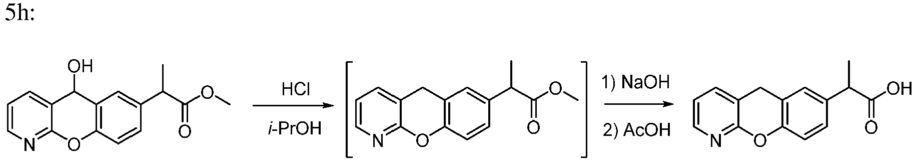

Compound **IV-A** (20.0g, lequiv.), hydrochloric acid (10.6g, 1.5equiv.), isopropanol (240ml) were added into a 250ml reaction flask, stirred for dissolution, heated to 50°C to 55°C for reaction for 4h, and concentrated at 55°C under reduced pressure until no distillate was distilled off to obtain a foamy oil (24 g). After cooling to room temperature, methanol (10ml) was added, stirred for dissolution, then added with a sodium hydroxide solution prepared with sodium hydroxide (11.2g, 4 equiv.) and purified water (40ml), and reacted at 50°C to 55°C for 2h. The reaction solution was transferred to a 500ml beaker, added with purified water (300ml), then the aqueous phase was extracted with ethyl acetate (100ml) 4 times, after the aqueous phase was separated out, it was adjusted with 20% acetic acid aqueous solution to pH 5-6, and gradually precipitated a white powdery solid. The filter cake after filtration was rinsed with purified water and cold methanol in turn, and dried in vacuum at 40°C to obtain 13.1 g of crude pranoprofen with a yield of about 73.2% and a purity of 97.76%.

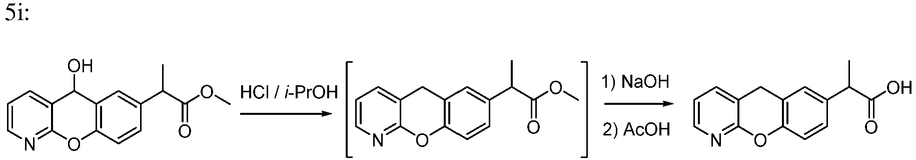

Compound **IV-A** (20.0g, lequiv.), isopropanol hydrogen chloride (29.0g, 13.2wt.%, 1.5equiv.), isopropanol (208ml) were added to a 250ml reaction flask, stirred for dissolution, heated to 50°C to 55°C for reaction for 4h, then concentrated under reduced pressure at 55°C until no distillate was distilled off to obtain a foamy oil (24g). After cooling to room temperature, methanol (10ml) was added, stirred for dissolution, then added with a sodium hydroxide solution prepared with sodium hydroxide (11.2g, 4 equiv.) and purified water (40ml), and reacted at 50°C to 55°C for 2h. The reaction solution was transferred to a 500ml beaker, added with purified water (300ml), then the aqueous phase was extracted with ethyl acetate (100ml) 4 times, after the aqueous phase was separated out, it was adjusted with 20% acetic acid aqueous solution to pH 5-6, and gradually precipitated a white powdery solid. The filter cake after filtration was rinsed with purified water and cold methanol in turn, and dried in vacuum at 40°C to obtain 13.1 g of crude pranoprofen with a yield of about 73.2% and a purity of 97.78%.

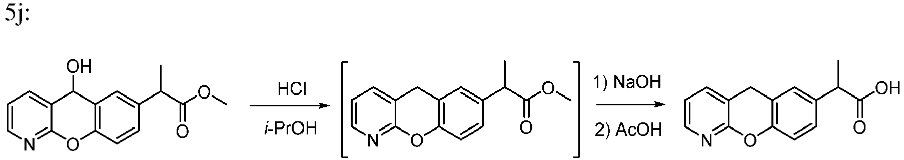

Compound **IV-A** (20.0g, lequiv.), hydrochloric acid (14.2g, 2equiv.), and isopropanol (240ml) were added into a 250ml reaction flask, stirred for dissolution, heated to 50°C to 55°C for reaction for 4 hours, then concentrated at 55°C under reduced pressure until no distillate was distilled off to obtain a foamy oil (24 g). After cooling to room temperature, methanol (10ml) was added, stirred for dissolution, then added with a sodium hydroxide solution prepared with sodium hydroxide (11.2g, 4equiv.) and purified water (40ml), and reacted at 50°C to 55°C for 2h. The reaction solution was transferred to a 500ml beaker, added with purified water (300ml), then the aqueous phase was extracted with ethyl acetate (100ml) 4 times, after the aqueous phase was separated out, it was adjusted with 20% acetic acid aqueous solution to pH 5-6, and gradually precipitated a white powdery solid. The filter cake after filtration was rinsed with purified water and cold methanol in turn, and dried in vacuum at 40°C to obtain 13.3 g of crude pranoprofen with a yield of about 74.3% and a purity of 98.12%.

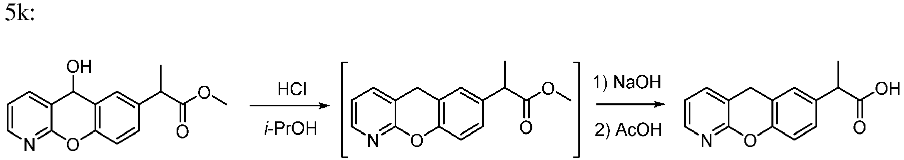

Compound **IV-A** (20.0g, lequiv.), hydrochloric acid (17.7g, 2.5equiv.), isopropanol (240ml) were added into a 250ml reaction flask, stirred for dissolution and heated to 50°C to 55°C for reaction for 4h, and concentrated at 55°C under reduced pressure until no distillate was distilled off to obtain a foamy oil (24 g). After cooling to room temperature, methanol (10ml) was added, stirred for dissolution, then added with a sodium hydroxide solution prepared with sodium hydroxide (11.2g, 4 equiv.) and purified water (40ml), and reacted at 50°C to 55°C for 2h. The reaction solution was transferred to a 500ml beaker, added with purified water (300ml), then the aqueous phase was extracted with ethyl acetate (100ml) 4 times, after the aqueous phase was separated out, it was adjusted with 20% acetic acid aqueous solution to pH 5-6, and gradually precipitated a white powdery solid. The filter cake after filtration was rinsed with purified water and cold methanol in turn, and dried in vacuum at 40°C to obtain 13.6 g of crude pranoprofen with a yield of about 76.0% and a purity of 98.63%.

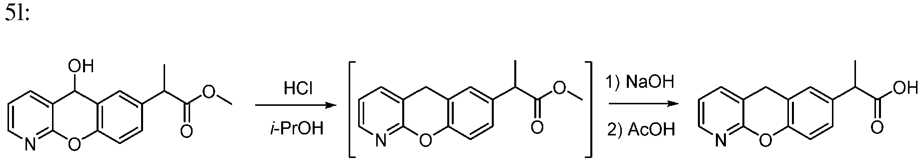

Compound **IV-A** (20.0g, lequiv.), hydrochloric acid (24.8g, 3.5equiv.), isopropanol (240ml) were added into a 250ml reaction flask, stirred for dissolution and heated to 50°C to 55°C for reaction for 4h, and concentrated at 55°C under reduced pressure until no distillate was distilled off to obtain a foamy oil (24 g). After cooling to room temperature, methanol (10ml) was added, stirred for dissolution, then added with a sodium hydroxide solution prepared with sodium hydroxide (11.2g, 4 equiv.) and purified water (40ml), and reacted at 50°C to 55°C for 2h. The reaction solution was transferred to a 500ml beaker, added with purified water (300ml), then the aqueous phase was extracted with ethyl acetate (100ml) 4 times, after the aqueous phase was separated out, it was adjusted with 20% acetic acid aqueous solution to pH 5-6, and gradually precipitated a white powdery solid. The filter cake after filtration was rinsed with purified water and cold methanol in turn, and dried in vacuum at 40°C to obtain 13.7 g of crude pranoprofen with a yield of about 76.6% and a purity of 98.77%.

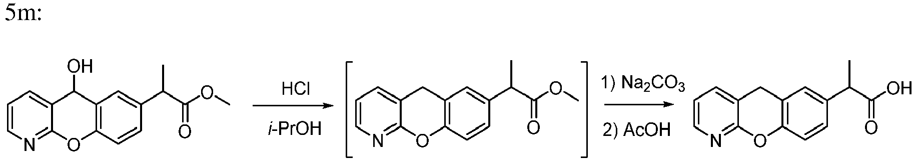

Compound **IV-A** (20.0g, lequiv.), hydrochloric acid (24.8g, 3.5equiv.), isopropanol (240ml) were added into a 250ml reaction flask, stirred for dissolution and heated to 80°C to 85°C for reaction under refluxing for 2h, and concentrated at 55°C under reduced pressure until no distillate was distilled off to obtain a foamy oil (24 g). After cooling to room temperature, methanol (10ml) was added, stirred for dissolution, then added with a sodium carbonate solution prepared with sodium carbonate (29.7g, 4 equiv.) and purified water (40ml), and reacted at 50°C to 55°C for 2h. The reaction solution was transferred to a 500ml beaker, added with purified water (300ml), then the aqueous phase was extracted with ethyl acetate (100ml) 4 times, after the aqueous phase was separated out, it was adjusted with 20% acetic acid aqueous solution to pH 5-6, and gradually precipitated a white powdery solid. The filter cake after filtration was rinsed with purified water and cold methanol in turn, and dried in vacuum at 40°C to obtain 13.9 g of crude pranoprofen with a yield of about 77.7% and a purity of 98.79%.

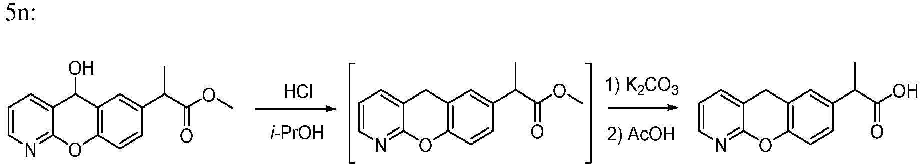

Compound **IV-A** (20.0g, lequiv.), hydrochloric acid (24.8g, 3.5equiv.), isopropanol (240ml) were added into a 250ml reaction flask, stirred for dissolution and heated to 80°C to 85°C for reaction under refluxing for 2h, and concentrated at 55°C under reduced pressure until no distillate was distilled off to obtain a foamy oil (24 g). After cooling to room temperature, methanol (10ml) was added, stirred to dissolve, then added with a potassium carbonate solution prepared with potassium carbonate (38.8g, 4 equiv.) and purified water (40ml), and reacted at 50°C to 55°C for 2h. The reaction solution was transferred to a 500ml beaker, added with purified water (300ml), then the aqueous phase was extracted with ethyl acetate (100ml) 4 times, after the aqueous phase was separated out, it was adjusted with 20% acetic acid aqueous solution to pH 5-6, and gradually precipitated a white powdery solid. The filter cake after filtration was rinsed with purified water and cold methanol in turn, and dried in vacuum at 40°C to obtain 13.8 g of crude pranoprofen with a yield of about 77.1% and a purity of 98.92%.

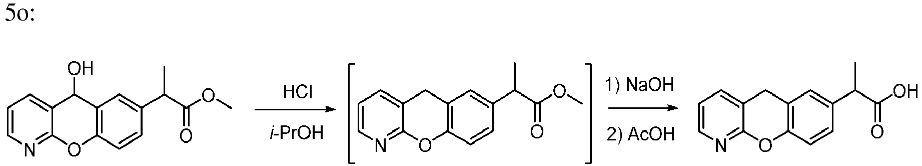

Compound **IV-A** (20.0g, lequiv.), hydrochloric acid (24.8g, 3.5equiv.), isopropanol (240ml) were added into a 250ml reaction flask, stirred for dissolution and heated to 80°C to 85°C for reaction under refluxing for 2h, and concentrated at 55°C under reduced pressure until no distillate was distilled off to obtain a foamy oil (24 g). After cooling to room temperature, methanol (10ml) was added, stirred for dissolution, then added with a sodium hydroxide solution prepared with sodium hydroxide (11.2g, 4 equiv.) and purified water (40ml), and reacted at 60°C to 65°C for 2h. The reaction solution was transferred to a 500ml beaker, added with purified water (300ml), then the aqueous phase was extracted with ethyl acetate (100ml) 4 times, after the aqueous phase was separated out, it was adjusted with 20% acetic acid aqueous solution to pH 5-6, and gradually precipitated a white powdery solid. The filter cake after filtration was rinsed with purified water and cold methanol in turn, and dried in vacuum at 40°C to obtain 14.0 g of crude pranoprofen with a yield of about 78.2% and a purity of 98.86%.

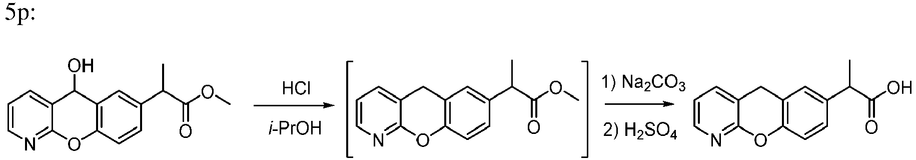

Compound **IV-A** (20.0g, lequiv.), hydrochloric acid (24.8g, 3.5equiv.), isopropanol (240ml) were added into a 250ml reaction flask, stirred for dissolution and heated to 80°C to 85°C for reaction under refluxing for 2h, and concentrated at 55°C under reduced pressure until no distillate was distilled off to obtain a foamy oil (24 g). After cooling to room temperature, methanol (10ml) was added, stirred for dissolution, then added with a sodium hydroxide solution prepared with sodium hydroxide (11.2g, 4equiv.) and purified water (40ml), and reacted at 40°C to 45°C for 2h. The reaction solution was transferred to a 500ml beaker, added with purified water (300ml), then the aqueous phase was extracted with ethyl acetate (100ml) 4 times, after the aqueous phase was separated out, it was adjusted with 10% dilute sulfuric acid to pH 5-6, and gradually precipitated a white powdery solid. The filter cake after filtration was rinsed with purified water and cold methanol in turn, and dried in vacuum at 40°C to obtain 13.7 g of crude pranoprofen with a yield of about 76.6% and a purity of 98.81%.

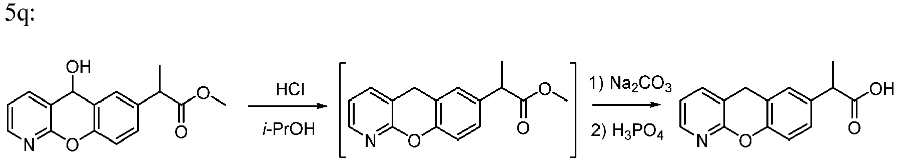

Compound **IV-A** (20.0g, lequiv.), hydrochloric acid (24.8g, 3.5equiv.), isopropanol (240ml) were added into a 250ml reaction flask, stirred for dissolution and heated to 80°C to 85°C for reaction under refluxing for 2h, and concentrated at 55°C under reduced pressure until no distillate was distilled off to obtain a foamy oil (24 g). After cooling to room temperature, methanol (10ml) was added, stirred for dissolution, then added with a sodium hydroxide solution prepared with sodium hydroxide (11.2g, 4equiv.) and purified water (40ml), and reacted at 40°C to 45°C for 2h. The reaction solution was transferred to a 500ml beaker, added with purified water (300ml), then the aqueous phase was extracted with ethyl acetate (100ml) 4 times, after the aqueous phase was separated out, it was adjusted with 10% dilute phosphoric acid to pH 5-6, and gradually precipitated a white powdery solid. The filter cake after filtration was rinsed with purified water and cold methanol in turn, and dried in vacuum at 40°C to obtain 13.9 g of crude pranoprofen with a yield of about 77.7% and a purity of 98.64%.

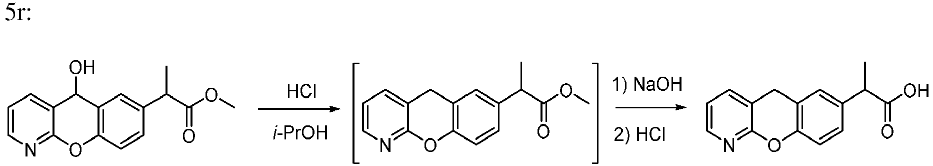

Compound **IV-A** (20.0g, lequiv.), hydrochloric acid (24.8g, 3.5equiv.), isopropanol (240ml) were added into a 250ml reaction flask, stirred for dissolution and heated to 80°C to 85°C for reaction under refluxing for 2h, and concentrated at 55°C under reduced pressure until no distillate was distilled off to obtain a foamy oil (24 g). After cooling to room temperature, methanol (10ml) was added, stirred for dissolution, then added with a sodium hydroxide solution prepared with sodium hydroxide (11.2g, 4equiv.) and purified water (40ml), and reacted at 40°C to 45°C for 2h. The reaction solution was transferred to a 500ml beaker, added with purified water (300ml), then the aqueous phase was extracted with ethyl acetate (100ml) 4 times, after the aqueous phase was separated out, it was adjusted with 10% dilute hydrochloric acid to pH 4-5, and gradually precipitated a white powdery solid. The filter cake after filtration was rinsed with purified water and cold methanol in turn, and dried in vacuum at 40°C to obtain 14.7 g of crude pranoprofen with a yield of about 82.1% and a purity of 98.74%.

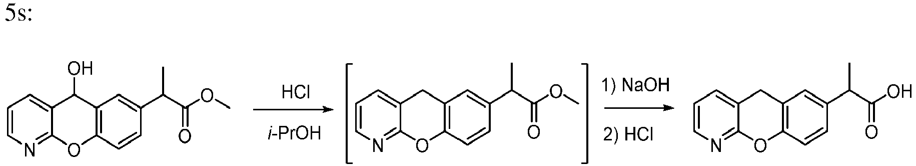

Compound **IV-A** (20.0g, lequiv.), hydrochloric acid (24.8g, 3.5equiv.), isopropanol (240ml) were added into a 250ml reaction flask, stirred for dissolution and heated to 80°C to 85°C for reaction under refluxing for 2h, and concentrated at 55°C under reduced pressure until no distillate was distilled off to obtain a foamy oil (24 g). After cooling to room temperature, methanol (10ml) was added, stirred for dissolution, then added with a sodium hydroxide solution prepared with sodium hydroxide (11.2g, 4equiv.) and purified water (40ml), and reacted at 40°C to 45°C for 2h. The reaction solution was transferred to a 500ml beaker, added with purified water (300ml), then the aqueous phase was extracted with ethyl acetate (100ml) 4 times, after the aqueous phase was separated out, it was adjusted with 10% dilute hydrochloric acid to pH 6-7, and gradually precipitated a white powdery solid. The filter cake after filtration was rinsed with purified water and cold methanol in turn, and dried in vacuum at 40°C to obtain 13.3 g of crude pranoprofen with a yield of about 74.3% and a purity of 99.77%.

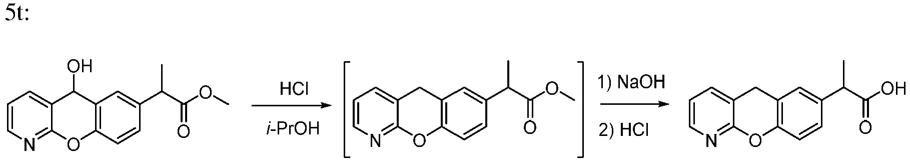

Compound **IV-A** (20.0g, lequiv.), hydrochloric acid (24.8g, 3.5equiv.), isopropanol (240ml) were added into a 250ml reaction flask, stirred for dissolution and heated to 80°C to 85°C for reaction under refluxing for 2h, and concentrated at 55°C under reduced pressure until no distillate was distilled off to obtain a foamy oil (24 g). After cooling to room temperature, methanol (10ml) was added, stirred for dissolution, then added with a sodium hydroxide solution prepared with sodium hydroxide (11.2g, 4equiv.) and purified water (40ml), and reacted at 40°C to 45°C for 2h. The reaction solution was transferred to a 500ml beaker, added with purified water (300ml), then the aqueous phase was extracted with ethyl acetate (100ml) 4 times, after the aqueous phase was separated out, it was adjusted to pH 3-4 with 10% dilute hydrochloric acid, and gradually precipitated a white powdery solid. The filter cake after filtration was rinsed with purified water and cold methanol in turn, and dried in vacuum at 40°C to obtain 15.0 g of crude pranoprofen with a yield of about 83.8% and a purity of 98.53%.

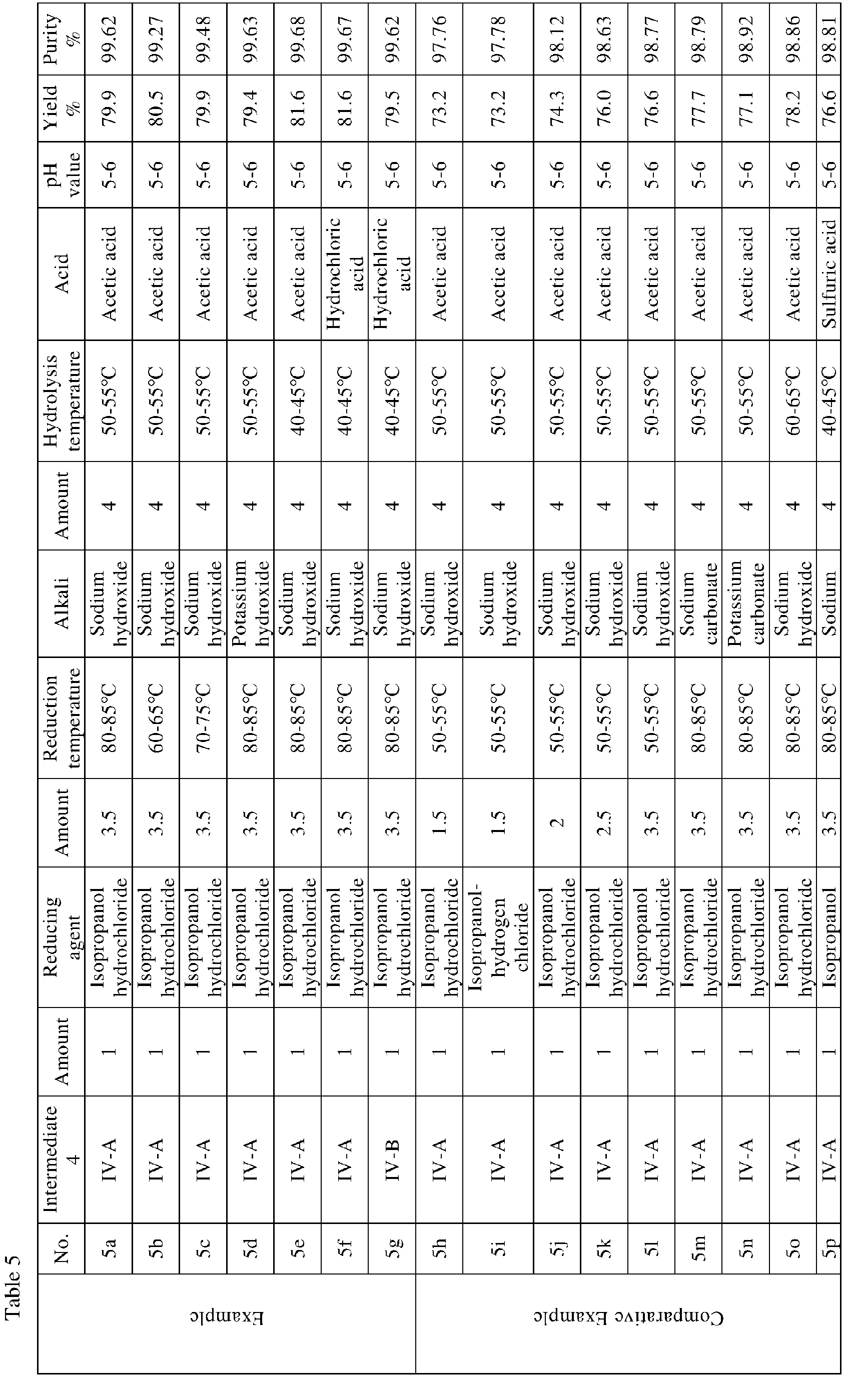

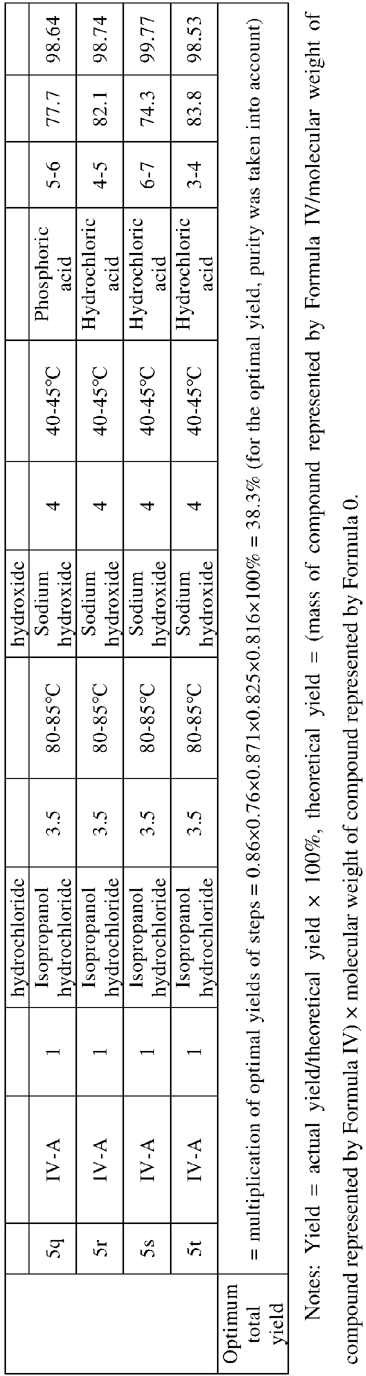

### Experimental Example 1: Structural characterization

The pranoprofen sample was synthesized according to the process described above in the present invention, and the synthesis process of this batch of sample was carried out according to the preparation conditions of 1a, 2c, 3c, 4b, and 5a. Standard product of pranoprofen was purchased directly from the Chinese National Institute for Food and Drug Control.

Structural confirmation was carried out.

### Compound element composition:

### (1) High resolution mass spectrometry (HRMS)

Instrument model.: Bruker SolariX 7.0T (FT-ICR MS).

Test conditions: ESI ionization mode.

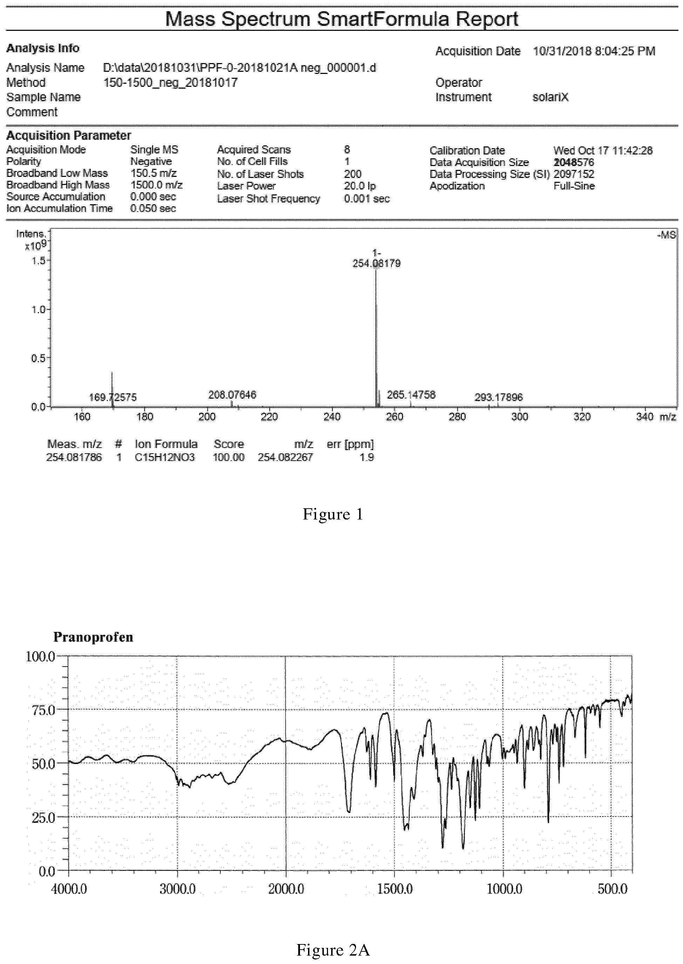

Test results: as shown in Figure 1. The results showed that the quasi-molecular ion peak m/z 254.081876 in the high-resolution mass spectrum was the [M-H]⁻ peak, and the corresponding element composition was C₁₅H₁₂NO₃, so the element composition of the sample was C₁₅H₁₃NO₃, which was consistent with the element composition of the free base moiety of the molecule.

### (2) Infrared spectrum (IR)

Instrument model: Bruker IFS-55 infrared photometer (corrected according to the General Rule 0402 of the Chinese Pharmacopoeia, 2015 edition).

Experimental method: KBr tablet method.

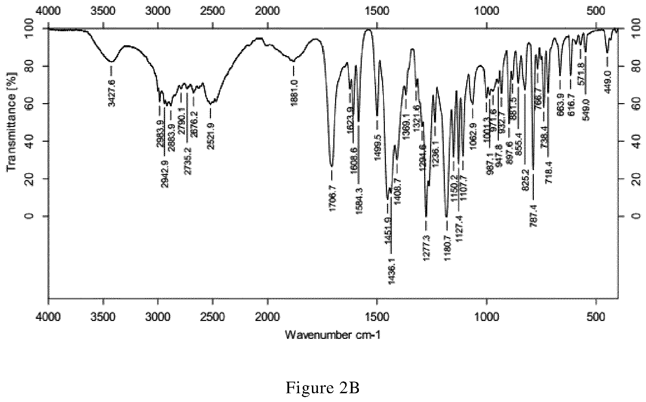

Experimental results: The measured data were shown in Table 6 below. The standard infrared spectrum was shown in Figure 2A. The infrared spectrum of the sample was shown in Figure 2B.

**Table 6: Infrared spectrum determination data of sample**

| Absorption peak wave number (cm⁻¹) | Vibration type | Group | Absorption intensity |
|---|---|---|---|
| 3427.6 | -O-H stretching vibration | COOH | w |
| 2983.9, 2942.9, 2883.9 | methyl, methylene C-H stretching vibration | CH₃, CH₂ | w |
| 1706.7 | Carboxylic acid carbonyl C=O stretching vibration | C=O | s |
| 1608.6, 1584.3, 1499.5 | Benzene ring C=C stretching vibration | C=C | m |
| 1451.9, 1436.1, 1408.7, 1369.1 | Methylmethylene C-H bending vibration | CH₃, CH₂ | s |
| 804.0 | Benzene ring C=C bending vibration | C=C | w |
| 767.8 | Benzene ring C-H bending vibration | =C-H | m |

| | | | |
|---|---|---|---|
| Analysis: a. The absorption peak at 3427.6cm⁻¹ was -O-H stretching vibration peak, indicating that there could be a carboxyl group in the molecule. b. The absorption peaks at 2968.2, 2943.1, 2881.9 and 1465-1376 cm⁻¹ were C-H stretching and bending vibration peaks, indicating that there could be a methyl group and methylene group in the molecule. c. The absorption peak at 1706.7cm⁻¹ was C=O stretching vibration peak, indicating that there could be a carbonyl group in the molecule. d. The absorption peaks at 1608.6, 1584.3, and 1499.5cm⁻¹ were C=C stretching vibration peaks of benzene ring, 767.8cm⁻¹ was C-H bending vibration peak of benzene ring, and 804.0cm⁻¹ was C=C bending vibration peak of benzene ring, indicating that there was a benzene ring in the molecule. | | | |

Conclusion: The positions, peak shapes and strengths of absorption peaks of functional groups in the infrared spectrum of the sample were consistent with the molecular structure of pranoprofen; the infrared spectrum of the sample was consistent with that of the reference substance, and was consistent with the corresponding spectrum of the Japanese Pharmacopoeia, JP17 version.

### (3) Ultraviolet spectrum (UV)

Instrument model: Agilent cary-60 UV spectrometer

Solution preparation: 0.02g of this sample was taken, added to 1mol/L hydrochloric acid solution for dissolution and diluted to 100ml as a mother liquor; 10ml of the above mother liquor was taken, added with water for dilution to 100ml as a neutral test sample.

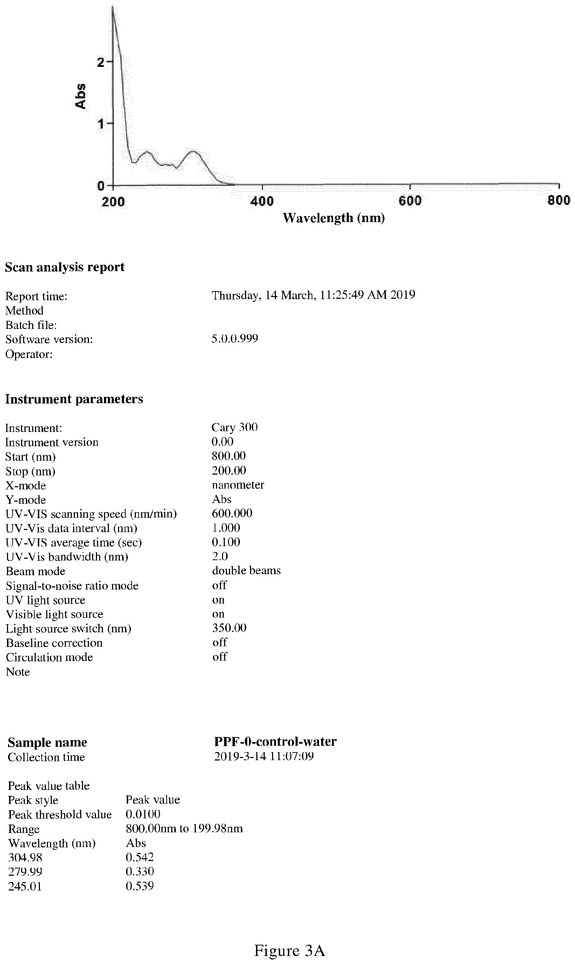

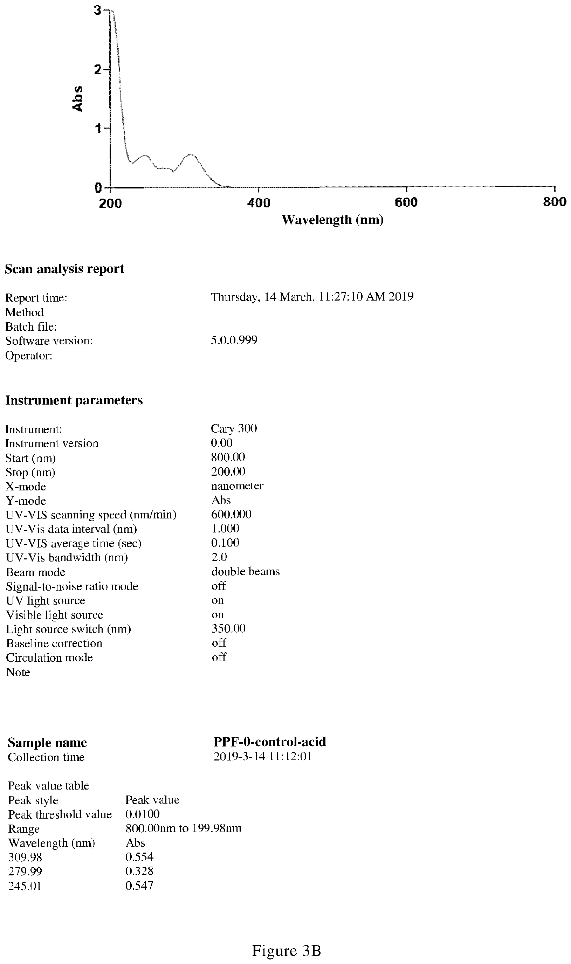

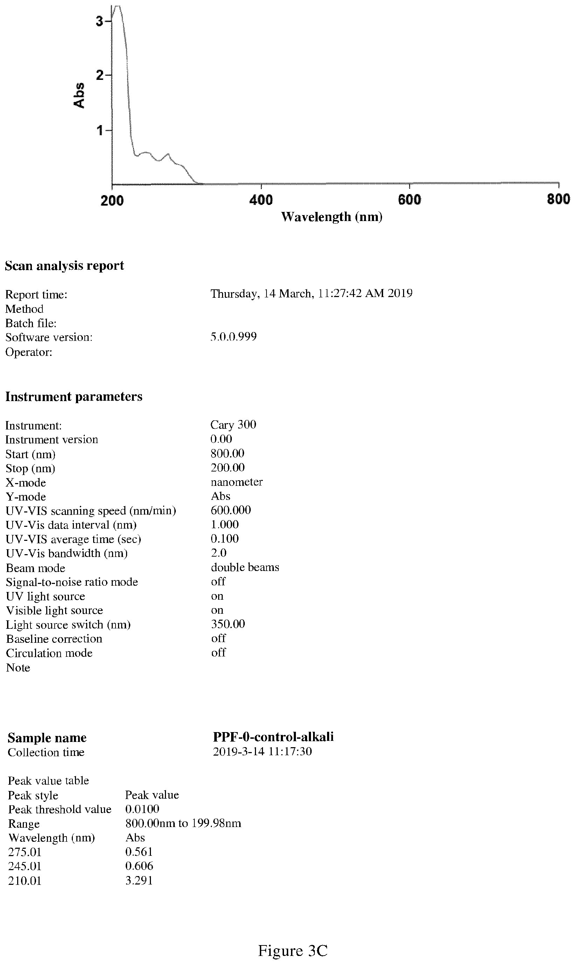

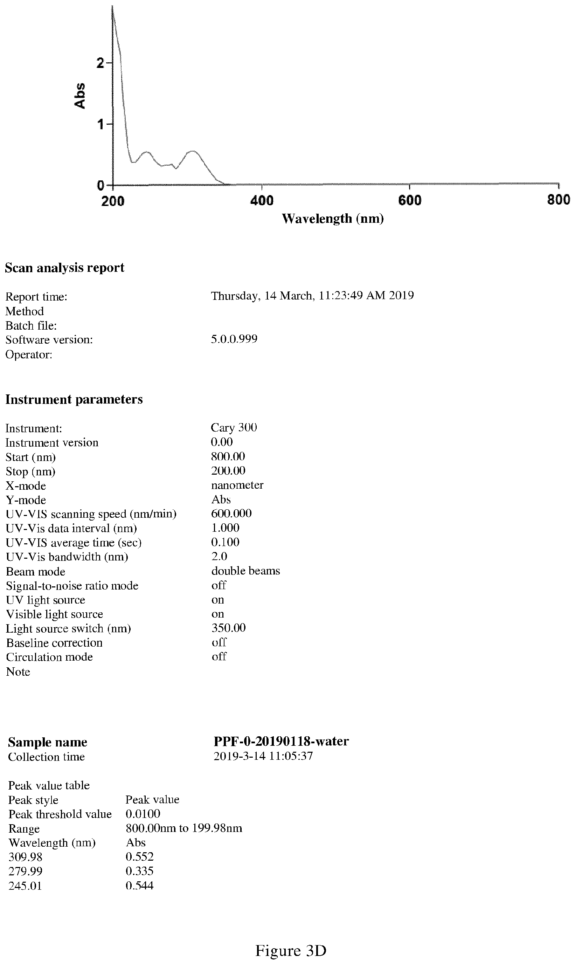

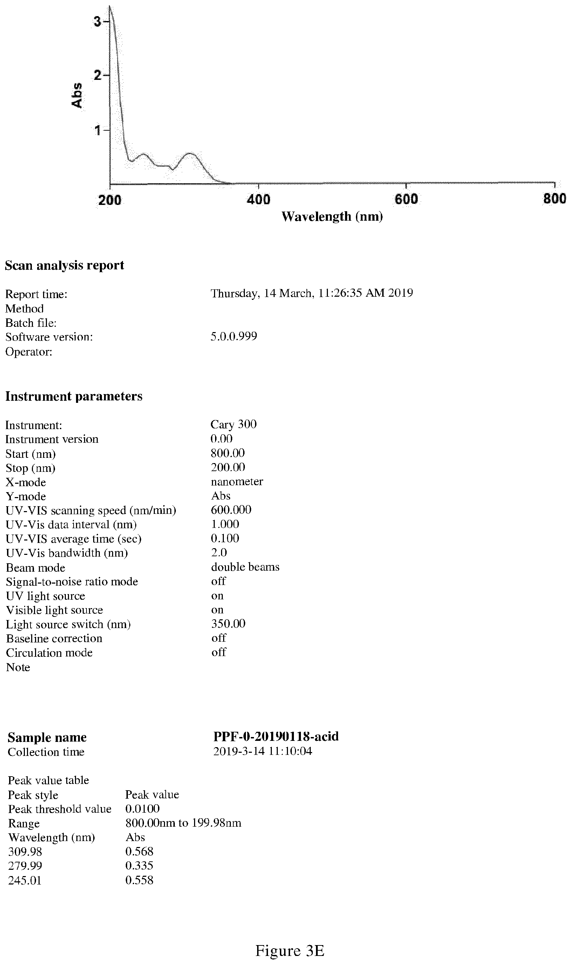

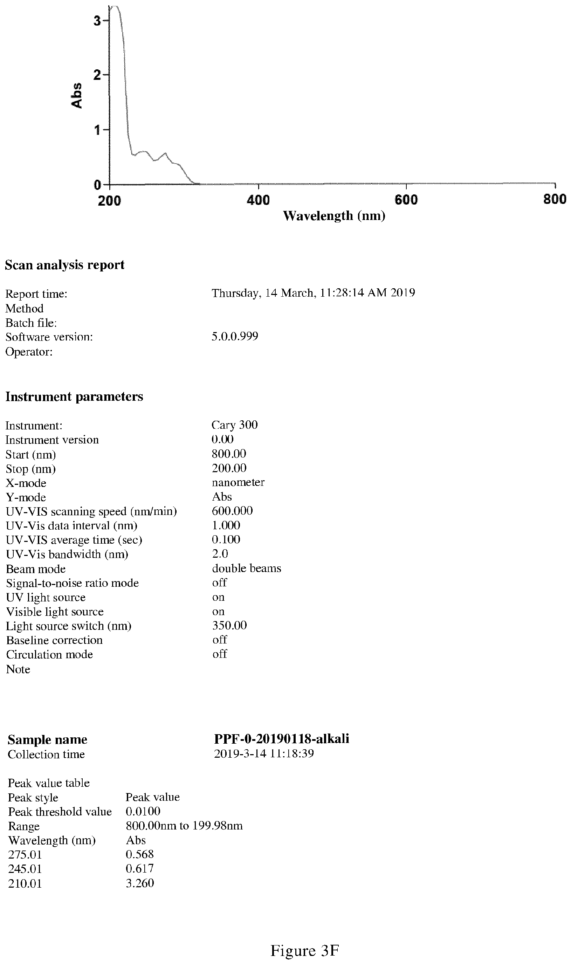

10ml of the above mother liquor was taken, added with 1mol/L hydrochloric acid solution for dilution to 100ml as an acidic test sample; 10ml of the above mother liquor was taken, added with 1mol/L sodium hydroxide solution for dilution to 100ml as an alkaline test sample. The test results were shown in Table 7 below and in Figures 3A to 3F.

**Table 7: UV measurement results of sample and standard product**

| Solvent | Maximum absorption wavelength | | Molar absorption coefficient (Abs) | |
|---|---|---|---|---|
| | Sample | Reference | Sample | Reference |
| Water | 309.98 | 304.98 | 0.552 | 0.542 |
| | 279.99 | 279.99 | 0.335 | 0.330 |
| | 245.01 | 245.01 | 0.544 | 0.539 |
| 0.1M hydrochloric acid solution | 309.98 | 309.98 | 0.568 | 0.554 |
| | 279.99 | 279.99 | 0.335 | 0.328 |
| | 245.01 | 245.01 | 0.558 | 0.547 |
| 0.1M sodium hydroxide solution | 275.01 | 275.01 | 0.568 | 0.561 |
| | 245.01 | 245.01 | 0.617 | 0.606 |
| | 210.01 | 210.01 | 3.260 | 3.291 |

| | | | | |
|---|---|---|---|---|
| Analysis: The peak shape and maximum absorption wavelength of the sample in the UV spectrum of the sample were consistent with those of the reference substance. | | | | |

Conclusion: The ultraviolet absorption of the sample was consistent with the molecular structure of pranoprofen; and the ultraviolet spectrum of the sample was consistent with that of the reference substance.

### (4) Nuclear magnetic resonance (NMR)

### ¹H-NMR

Instrument model: Bruker AV-600 nuclear magnetic resonance instrument.

Test conditions: solvent DMSO-d6, TMS internal standard.

The test data were shown in Table 8 below.

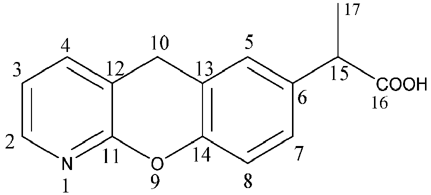

**Table 8: Analysis of NMR H spectrum data of sample**

| No. | Chemical shift | | Hydrogen number | Peak shape | Assignment |
|---|---|---|---|---|---|
| | Sample ppm | Standard ppm | | | |
| 1 | 1.36 | 1.36 | 3 | d | 17-position methyl hydrogen atom |
| 2 | 3.66 | 3.66 | 1 | q | 15-position isopropyl hydrogen atom |
| 3 | 4.11 | 4.11 | 2 | s | 10-position methylene hydrogen atom |
| 4 | 8.13 | 8.13 | 1 | m | 2-position pyridine ring hydrogen atom |
| 5 | 7.72 | 7.72 | 1 | m | 4-position pyridine ring hydrogen atom |
| 6 | 7.15 | 7.15 | 1 | m | 3-position pyridine ring hydrogen atom |
| 7 | 7.18 | 7.18 | 1 | m | 5-position benzene ring hydrogen atom |
| 8 | 7.16 | 7.16 | 1 | m | 7-position benzene ring hydrogen atom |
| 9 | 7.09 | 7.08 | 1 | d | 8-position benzene ring hydrogen atom |
| 10 | 12.34 | 12.31 | 1 | s | 10-position carboxyl hydrogen atom |

| | | | | | |
|---|---|---|---|---|---|
| Analysis: The single peak at δ12.34ppm was carboxyl hydrogen. The 1H at δ7.09ppm was the hydrogen on the 8-position of the benzene ring. The 1H at δ7.16ppm was the hydrogen on the 7-position of the benzene ring. The 1H at δ7.18 was the hydrogen on the 5-position of the benzene ring. The 1H at δ7.15ppm was the hydrogen on the 3-position of the pyridine ring. The 1H at δ7.72ppm was the hydrogen on the 4-position of the pyridine ring. The 1H at δ8.13ppm was the hydrogen on the 2-position pyridine ring. The 2H singlet at δ4.11ppm was the hydrogen on the 10-position methylene. The 1H quartet at δ3.66ppm was the 15-position isopropyl hydrogen, which was coupled with the 17-position methyl hydrogen, and was quartet due to splitting. The 3H doublet at δ1.36ppm was the 17-position methyl hydrogen, which was coupled with the 15-position isopropyl hydrogen, and was doublet due to splitting. | | | | | |

Conclusion: The ¹H-NMR spectrum data of the sample was consistent with the structure of pranoprofen, and the spectrum of the sample was consistent with that of the reference substance.

### ¹³C-NMR

Instrument model: Bruker AV-600 nuclear magnetic resonance instrument.

Test conditions: solvent DMSO-d6, TMS internal standard.

The test data were shown in Table 9 below.

**Table 9: Analysis of NMR C spectrum data of sample**

| No. | Chemical shift (ppm) | | Carbon type | Assignment | Chemical shift (ppm) | Chemical shift (ppm) |
|---|---|---|---|---|---|---|
| | Sample | Standard | | | Correlated proton | Remotely correlated proton |
| 1 | 175.39 | 175.24 | Quaternary carbon | 16 | - | 1.36,3.66 |
| 2 | 157.83 | 157.79 | Quaternary carbon | 11 | - | 4.11, 7.72, 8.13 |
| 3 | 150.03 | 149.97 | Quaternary carbon | 14 | - | 4.11, 7.09, 7.16, 7.18 |
| 4 | 136.71 | 136.63 | Quaternary carbon | 6 | - | 1.36, 3.66, 7.09 |
| 5 | 120.00 | 119.92 | Quaternary carbon | 13 | - | 4.11, 7.09 |
| 6 | 115.62 | 115.53 | Quaternary carbon | 12 | - | 4.11, 7.15 |
| 7 | 146.26 | 146.17 | Tertiary carbon | 2 | 8.13 | 7.72, 7.15, 4.11 |
| 8 | 138.89 | 138.76 | Tertiary carbon | 4 | 7.72 | 8.13,4.11 |
| 9 | 127.79 | 127.68 | Tertiary carbon | 5 | 7.18 | 4.11, 3.66 |
| 10 | 127.01 | 126.91 | Tertiary carbon | 7 | 7.16 | 7.18, 3.66 |
| 11 | 120.27 | 120.15 | Tertiary carbon | 3 | 7.15 | 4.11 |
| 12 | 116.41 | 116.31 | Tertiary carbon | 8 | 7.09 | - |
| 13 | 27.19 | 27.14 | Tertiary carbon | 15 | 3.66 | 7.18,7.16 |
| 14 | 43.94 | 43.88 | Secondary carbon | 10 | 4.11 | 7.16, 1.36 |
| 15 | 18.54 | 18.44 | Primary carbon | 17 | 1.36 | 3.66 |

| | | | | | | |
|---|---|---|---|---|---|---|
| Analysis: The molecule of pranoprofen contains 15 carbon atoms, including 1 primary carbon, 1 secondary carbon, 7 tertiary carbons, and 6 quaternary carbons; and contains 13 hydrogen atoms, and the analysis was as follows: 1. The carbon at δ175.39ppm was quaternary carbon atom, which was related to the hydrogen atoms on the 15- and 17-position carbons in the HMBC spectrum, and could be determined as the 16-position carboxyl carbon. 2. The carbon at δ157.83ppm was quaternary carbon atom, which was related to the pyridine ring hydrogen atoms on the 2-position and 4-position and the hydrogen atoms on the 10-position methylene in the HMBC spectrum, and could be determined as the 11-position oxygen-linked pyridine ring carbon. 3. The carbon at δ150.03ppm was quaternary carbon atom, which was related to the benzene ring hydrogen atoms on the 5-position, 7-position, 8-position and the hydrogen atoms on the 10-position methylene carbon in the HMBC spectrum, and could be determined as the 14-position oxygen-linked benzene ring carbon. 4. The carbon at δ136.71ppm was quaternary carbon atom, which was related to the hydrogen on the 7-position benzene ring carbon, the hydrogen on the 15-position isopropyl carbon, and the hydrogen on the 17-position methyl in the HMBC spectrum, and could be determined as the 6-position benzene ring carbon. 5. The carbon at δ120.00ppm was quaternary carbon atom, which was related to the hydrogen on the 8-position benzene ring carbon and the hydrogen on the 10-position methylene carbon in the HMBC spectrum, and could be determined as the 14-position benzene ring carbon. 6. The carbon at δ115.62ppm was quaternary carbon atom, which was related to the hydrogen on the 4-position pyridine ring carbon and the hydrogen on the 10-position methylene carbon in the HMBC spectrum, and could be determined as the 12-position pyridine ring carbon. 7. The carbon at δ146.26ppm was tertiary carbon atom, which was related to the hydrogen on the 2-position pyridine ring carbon in the HSQC spectrum, and related to the hydrogen atoms on the 3-position and 4-position pyridine ring carbon atoms in the HMBC spectrum, and could be determined as the 2-position pyridine ring carbon. 8. The carbon at δ138.89ppm was tertiary carbon atom, which was related to the hydrogen on the 4-position pyridine ring carbon in the HSQC spectrum, and related to the hydrogen on the 2-position pyridine ring carbon and the hydrogen on the 10-position methylene carbon in the HMBC spectrum, and could be determined as the 4-position pyridine ring carbon. 9. The carbon at δ127.79ppm was tertiary carbon atom, which was related to the hydrogen on the 5-position benzene ring carbon in the HSQC spectrum, and related to the 10-position methylene hydrogen and the 15-position isopropyl hydrogen in the HMBC spectrum, and could be determined as the 5-position benzene ring carbon. 10. The carbon at δ127.01ppm was tertiary carbon atom, which was related to the hydrogen on the 7-position benzene ring carbon in the HSQC spectrum, and related to the hydrogen on the 5-position benzene ring carbon and the 15-position isopropyl hydrogen in the HMBC spectrum, and could be determined as the 7-position benzene ring carbon. 11. The carbon at δ120.27ppm was tertiary carbon atom, which was related to the hydrogen on the 3-position pyridine ring carbon in the HSQC spectrum, and related to the 10-position methylene hydrogen in the HMBC spectrum, and could be determined as the 3-position pyridine ring carbon. 12. The carbon at δ116.41ppm was tertiary carbon atom, which was related to the hydrogen on the 8-position benzene ring carbon in the HSQC spectrum, and related to no hydrogen in the HMBC spectrum, and could be determined as the 8-position benzene ring carbon. 13. The carbon at δ27.19ppm was tertiary carbon atom, which was related to the hydrogen on the 15-position isopropyl carbon in the HSQC spectrum, and related to the hydrogen atoms on the 5-position and 7-position benzene ring carbons in the HMBC spectrum, and could be determined as the 15-position isopropyl carbon. 14. The carbon at δ43.94ppm was secondary carbon atom, which was related to the 10-position methylene hydrogen in the HSQC spectrum, and could be determined as the 10-position methylene carbon. 15. The carbon at δ18.54ppm was primary carbon atom, which was related to the hydrogen on the 17-position methyl carbon in the HSQC spectrum, and related to the 15-position isopropyl hydrogen in the HMBC spectrum, and could be determined as the 17-position methyl carbon. | | | | | | |

Conclusion: The ¹³C-NMR spectrum data of the sample was consistent with the structure of pranoprofen, and the spectrum of the sample was consistent with that of the reference substance.

### Summary:

(1) The measurement results of IR, UV, NMR, and MS of this sample were consistent with those of the reference substance, and thus they were compounds of the same structure and the same crystal form, that was, this sample was pranoprofen.
(2) The sample m/z 295.201619 measured by high-resolution mass spectrometry was the [M-H]⁻ peak, and the corresponding element composition was C₁₅H₁₂NO₃, so the element composition of the sample was C₁₅H₁₃NO₃, which was consistent with the structure of the organic base moiety in the molecule.
(3) The UV spectrum showed strong absorption peaks at 260 to 270nm, the IR spectrum showed absorption peaks at 1591.9cm⁻¹, 1521.1cm⁻¹, 804.0cm⁻¹ and 767.8cm⁻¹, the ¹H-NMR spectrum showed absorption peaks at δ7.30ppm, δ7.25ppm, δ6.87ppm, and the ¹³C-NMR spectrum showed absorption peaks at δ118.14ppm, δ113.35ppm and δ109.91ppm, indicating that there was a three-substituted benzene ring structural fragment.
(4) The absorption peak at 1706.7cm⁻¹ in the IR spectrum and the absorption peak at δ175.39ppm in the ¹³C-NMR spectrum indicated the presence of carbonyl structural fragment.

In summary, the molecular structure of the sample was consistent with that of pranoprofen.

### Experimental Example 2: Study on stability

### 1. Experimental samples

According to the process of the present invention, three scale-up batches in kilogram-scale were prepared under optimal experimental conditions, and named as 20190101, 20190201, and 20190202, respectively. The three batches were products prepared in independent batches, the synthesis process was carried out according to the preparation conditions of 1a, 2c, 3c, 4b, and 5a, and they were obtained by refining and purifying with methanol (i.e., methanol recrystallization). The products of the three batches had purity of 99.91%, 99.90% and 99.95%, respectively.

### 2. Experimental method

In accordance with the relevant regulations in the "Technical Guidelines for Stability Research of Chemical Drugs", the samples of the above three batches were taken and tested for 6 months under the conditions of 40°C ± 2°C and RH 75% ± 5%. And at the end of the 0^{th}, 1^{st}, 2^{nd}, 3^{rd}, and 6^{th} months of the experiment, the samples were taken for the detection of indicators.

### Chromatographic conditions:

The experiment was carried out according to the high performance liquid chromatography (the General Rule 0512 of the Chinese Pharmacopoeia, 2015 edition), in which octadecylsilane bonded silica gel was used as filler (5µm, 250mm×4.6mm); 0.05mol/L sodium perchlorate aqueous solution-methanol (90:10, adjusted to pH 3.2 by adding perchloric acid) was used as mobile phase A, methanol was used as mobile phase B, and gradient elution was carried out according to the table below, flow rate was 1.0ml/min, column temperature was 40°C, and detection wavelength was 275nm. The elution procedure was shown in Table 10 below.

**Table 10: Elution procedure**

| Time | Mobile phase A | Mobile phase B |
|---|---|---|
| 0 | 80 | 20 |
| 40 | 55 | 45 |
| 60 | 45 | 55 |

10 µl of the test solution was precisely taken and injected into the liquid chromatograph. If there were impurity peaks in the chromatogram of the test solution, a single impurity must not exceed 0.1%, and the sum of impurities must not exceed 0.5%.

The impurity objects to be detected were total impurity, impurity A, impurity C, impurity D, impurity L and maximum single impurity; and the detection conditions were the same as above.

Preparation method of impurity A: Purified water (140ml) and sodium hydroxide (14.8g) were added to a 1000ml three-necked flask, stirred for dissolution, then added with Compound **III-A** (35g) and anhydrous methanol (20ml), heated to 45°C and reacted for 2 hours, then cooled to 20°C to 30°C, and added with 70ml of purified water for dilution, the reaction solution was extracted with 105ml of ethyl acetate, and the water phase was collected. The aqueous phase was adjusted to pH 5-6 with 20% phosphoric acid aqueous solution to precipitate a white solid, stirred for 30 minutes, and filtered with suction, and the filter cake was washed with 105ml of water, and blow dried at 50°C to obtain the target product.

Preparation method of impurity C: 2-Chloronicotinic acid (30g, 0.19mol), 4-ethylphenol (69.6g, 0.57mol) and anhydrous potassium carbonate (52.5g, 0.38mol), cuprous iodide (1.8g, 0.01mol) and N,N-dimethylformyl (30ml) were added to a 500ml three-necked flask, heated to 100°C to 110°C and reacted for 6 hours, cooled to 30°C to 35°C, then added with 120ml of water for dilution, transferred into a 2L plastic beaker, adjusted to pH 3-4 with 30% phosphoric acid, and filtered with suction, and the filter cake was rinsed with petroleum ether. The filter cake was added to 100ml of water, adjusted to pH 7-8 with saturated aqueous sodium bicarbonate solution, and filtered with suction, and the filtrate was collected, adjusted to pH 3-4 with 30% phosphoric acid, filtered with suction, and the filter cake was dried.

37g of solid was taken and added to a 1000ml three-necked flask, added with 125g of polyphosphoric acid, heated to 120°C to 130°C and reacted for 4 hours, then cooled to 20°C to 30°C, added with 600ml of water, and the filter cake after suction filtration was washed with 200ml of water, and the obtained filter cake was blow dried at 60°C to obtain the target product.

Preparation method of impurity D: Pranoprofen (15g), N,N-dimethylformamide (4.5ml) and anhydrous methanol (90ml) were added into a 250ml three-necked flask, and the system was suspended. A solution of thionyl chloride (8.4 g) in dichloromethane (20 ml) was added dropwise, and reacted at 20°C to 30°C for 4 hours after the dropwise reaction. After the reaction was completed, the system was transferred into a 500ml single-necked bottle, concentrated under reduced pressure in a 55°C water bath until no obvious distillate was distilled off, the resulting concentrate was diluted with 150ml of dichloromethane, adjusted to pH 7-8 with saturated aqueous sodium bicarbonate solution, stirred for 10 minutes, and allowed to stand for liquid separation. The organic phase was washed with 100 ml of saturated aqueous sodium bicarbonate solution and 100 ml of saturated brine, respectively. The organic phase was concentrated under reduced pressure in a water bath at 45°C until no obvious distillate was distilled off to obtain a white solid, which was added with 100ml of n-hexane and stirred for 30 minutes, filtered with suction, and the filter cake was vacuum-dried at 40°C to obtain the target product.

Preparation method of impurity L: THF (150ml) and Compound **IV-A** (25g) were added to a 500ml single-necked bottle, dissolved to clear, and then added with acetone (30.7g), and stirred to obtain a colorless solution. Aluminum trichloride (23.5g) was added in batches, and after the addition was completed, a light yellow solution was obtained, which was subjected to reaction for 4 hours, and concentrated under pressure at 45°C until no obvious distillate was distilled off, then the concentrate was poured into 100ml of water, and adjusted to pH 2-3 with saturated aqueous sodium bicarbonate solution, extracted with 100ml of DCM twice, the organic phases were combined and washed with 50ml of water, the organic phase was taken and concentrated under reduced pressure at 45°C until no obvious distillate was distilled off, and subjected to column chromatography to obtain a product. The obtained product (25g), methanol (125ml), water (12.5ml) and sodium hydroxide (9.2g) were stirred and reacted for 1 hour. The reaction solution was transferred to a 500ml beaker, added with purified water (300g), then the water phase was extracted 4 times with ethyl acetate (90g), respectively, after the water phase was separated out, it was adjusted to pH about 5.8 with 20% acetic acid aqueous solution, and gradually precipitated a white powdery solid. The filter cake after filtration was rinsed with purified water and cold methanol in turn, and dried in vacuum at 40°C to obtain the target product.

Preparation of test solution: 50mg of this product was taken, dissolved in a solvent (acetonitrile-water=1:1) and diluted to 50ml to obtain the test solution.

Control stock solution of mixed impurities: 10.0 mg of each of impurities A, C, D, and L were weighed, respectively, and added to 10.0ml volumetric flask, respectively, then added with methanol for dissolution and dilution to the mark, 1.0 ml of each of the above solutions was taken and added to 100.0 ml volumetric flask, then added with solvent for dilution to the mark to obtain the control stock solution of impurities.

Control solution of mixed impurities: 1.0ml of the test solution was accurately taken, and added with solvent for dilution to 100.0ml; 1.0ml of the above solution was accurately taken, added with 1.0ml of the control stock solution of mixed impurities, and added with solvent to 10.0ml to obtain the control solution of mixed impurities.

Preparation of system suitability solution: 1.0ml of the control stock solution of mixed impurities was accurately taken, and added with the test solution for dilution to 10ml to obtain the system suitability solution.

Preparation of control solution: 1.0ml of the test solution was taken, and added with solvent for dilution to 10.0ml; 1.0ml of the above solution was taken, and added with solvent for dilution to 100.0ml to obtain the control solution.

### 3. Experimental results

The experimental results were shown in Table 11 below.

**Table 11**

| | 20190101 | 20190201 | 20190202 |
|---|---|---|---|
| 0^{th} month | Impurity A: 0.05% | Impurity A: 0.07% | Impurity A: 0.02% |
| | Impurity L: 0.02% | Impurity L: 0.01% | Impurity L: 0.01% |
| | Impurity D: 0.02% | Impurity D: 0.02% | Impurity D: 0.02% |
| | Total impurities: 0.09% | Total impurities: 0.10% | Total impurities: 0.05% |
| 1^{st} month after acceleration | Impurity A: 0.03% | Impurity A: 0.07% | Impurity A: 0.05% |
| | Impurity L: 0.01% | Impurity L: 0.01% | Impurity L: 0.02% |
| | Impurity D: 0.02% | Impurity D: 0.03% | Impurity D: 0.01% |
| | Total impurities: 0.06% | Total impurities: 0.11% | Total impurities: 0.06% |
| 2^{nd} month after acceleration | Impurity A: 0.04% | Impurity A: 0.07% | Impurity A: 0.04% |
| | Impurity L: 0.01% | Impurity L: 0.01% | Impurity L: 0.01% |
| | Impurity D: 0.02% | Impurity D: 0.02% | Impurity D: 0.02% |
| | Total impurities: 0.07% | Total impurities: 0.10% | Total impurities: 0.07% |
| 3^{rd} month after acceleration | Impurity A: 0.06% | Impurity A: 0.07% | Impurity A: 0.04% |
| | Impurity L: 0.02% | Impurity L: 0.02% | Impurity L: 0.01% |
| | Impurity D: 0.01% | Impurity D: 0.02% | Impurity D: 0.02% |
| | Total impurities: 0.08% | Total impurities: 0.11% | Total impurities: 0.07% |
| 6^{th} month after acceleration | Impurity A: 0.06% | Impurity A: 0.10% | Impurity A: 0.09% |
| | Impurity L: 0.02% | Impurity L: 0.02% | Impurity L: 0.02% |
| | Impurity D: 0.02% | Impurity D: 0.01% | Impurity D: 0.01% |
| | Impurity C 0.03% | Impurity C 0.03% | Impurity C 0.01% |
| | Maximum single impurity: 0.02% | Maximum single impurity: 0.08% | Maximum single impurity: 0.02% |
| | Total impurities: 0.17% | Total impurities: 0.24% | Total impurities: 0.15% |

| | | | |
|---|---|---|---|
| Notes: The maximum single impurity usually referred to the maximum unknown single impurity, and the known impurities were directly expressed by the impurity codes. | | | |

The results showed that the quality of the products of the three batches prepared by the present invention was stable and reliable.

Although specific embodiments of the present invention have been described in detail, those skilled in the art will understand that based on all the teachings that have been disclosed, various modifications and substitutions can be made to those details, and these changes are all within the scope of the present invention. The full scope of the present invention is given by the appended claims and any equivalents thereof.

## Claims

1. A method for preparing pranoprofen or a pharmaceutically acceptable salt or ester of pranoprofen, comprising a step of preparing:
a compound represented by Formula I or pharmaceutically acceptable salt or ester thereof,
a compound represented by Formula II or pharmaceutically acceptable salt or ester thereof,
a compound represented by Formula III or pharmaceutically acceptable salt or ester thereof, and/or
a compound represented by Formula IV or pharmaceutically acceptable salt or ester thereof, 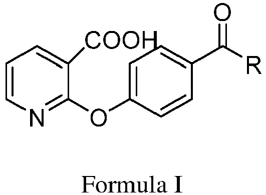
wherein, R is C₂-C₁₀ straight chain or branched chain alkyl, preferably, R is C₂-C₆ straight chain or branched chain alkyl, more preferably, R is C₂-C₄ straight chain or branched chain alkyl, such as ethyl, propyl, isopropyl or n-butyl; 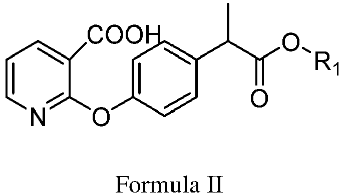
wherein, R₁ is C₁-C₁₀ straight chain or branched chain alkyl, preferably, R₁ is C₁-C₅ straight chain or branched chain alkyl, more preferably, R₁ is C₁-C₃ straight chain or branched chain alkyl, such as methyl, ethyl, propyl or isopropyl; 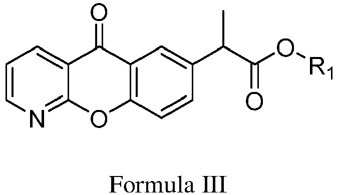
wherein, R₁ is C₁-C₁₀ straight chain or branched chain alkyl, preferably, R₁ is C₁-C₅ straight chain or branched chain alkyl, more preferably, R₁ is C₁-C₃ straight chain or branched chain alkyl, such as methyl, ethyl, propyl or isopropyl; 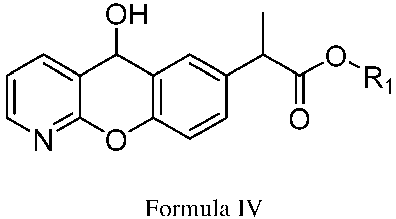
wherein, R₁ is C₁-C₁₀ straight chain or branched chain alkyl, preferably, R₁ is C₁-C₅ straight chain or branched chain alkyl, more preferably, R₁ is C₁-C₃ straight chain or branched chain alkyl, such as methyl, ethyl, propyl or isopropyl.

2. The preparation method according to claim 1, comprises the following steps:
① using a compound represented by Formula A and a compound represented by Formula B as starting materials, and performing Ullmann condensation reaction in the presence of cuprous iodide to prepare the compound represented by Formula I;
② under the action of acid and iodobenzene diacetate, performing a rearrangement reaction in trimethyl orthoformate to obtain the compound represented by Formula II;
③ undergoing halogenation to form an acyl chloride, then performing intramolecular ring closure under the action of a Lewis acid to obtain the compound represented by Formula III;
④ undergoing borohydride reduction to obtain the compound represented by Formula IV;
⑤ finally, undergoing isopropanol hydrogen chloride reduction, alkali hydrolysis, and post-treatment acidification through the "one-pot method" to obtain pranoprofen;
wherein, the structural formulas of the formula A compound and the formula B compound are as follows: 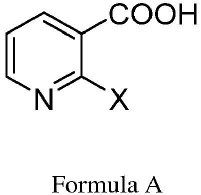 wherein,
X is halogen, such as fluorine, chlorine, bromine or iodine, preferably chlorine or bromine; 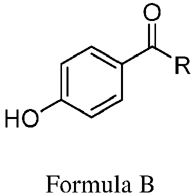
wherein, R is C₂-C₁₀ straight chain or branched chain alkyl, preferably, R is C₂-C₆ straight chain or branched chain alkyl, more preferably, R is C₂-C₄ straight chain or branched chain alkyl, such as ethyl, propyl, isopropyl or n-butyl.

3. A method for preparing pranoprofen or a pharmaceutically acceptable salt or ester of pranoprofen, comprising a step of preparing pranoprofen from a compound represented by Formula IV: 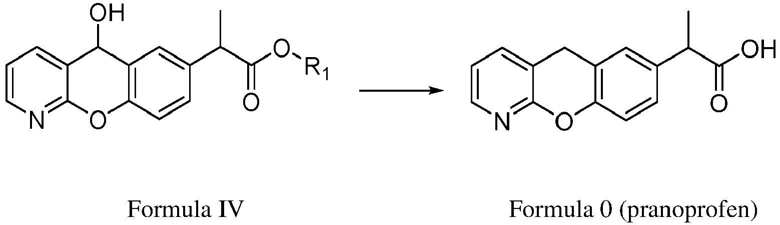 wherein, Ri is C₁-C₁₀ straight chain or branched chain alkyl, preferably, R₁ is C₁-C₅ straight chain or branched chain alkyl, more preferably, R₁ is C₁-C₃ straight chain or branched chain alkyl, such as methyl, ethyl, propyl or isopropyl.

4. The preparation method according to any one of claims 1 to 3, comprising the following steps:
(1) preparing a compound represented by Formula V from the compound represented by Formula IV;
(2) preparing pranoprofen from the compound represented by Formula V; 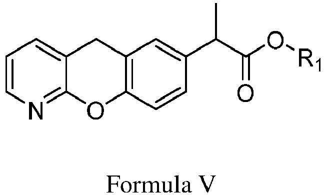
wherein, R₁ is C₁-C₁₀ straight chain or branched chain alkyl, preferably, R₁ is C₁-C₅ straight chain or branched chain alkyl, more preferably, R₁ is C₁-C₃ straight chain or branched chain alkyl, such as methyl, ethyl, propyl or isopropyl.

5. The preparation method according to any one of claims 1 to 4, wherein the compound represented by Formula IV is methyl 2-(10-hydroxyl-9-oxa-1-azaanthracen-6-yl)propionate, ethyl 2-(10-hydroxy-9-oxa-1-azaanthracen-6-yl)propionate or propyl 2-(10-hydroxy-9-oxa-1-azaanthracen-6-yl)propionate.

6. The preparation method according to any one of claims 1 to 5, wherein, in step (1), the compound represented by Formula IV is reacted with a reducing agent to obtain the compound represented by Formula V;
preferably, the reducing agent is one or more selected from the group consisting of isopropanol hydrogen chloride and isopropanol hydrochloride;
preferably, the molar ratio of the compound represented by Formula IV to the reducing agent is 1:(1.5-3.5), preferably 1:(2.5-3.5), such as 1:2.5, 1:3 or 1:3.5.

7. The preparation method according to any one of claims 1 to 6, wherein, in step (1), the reaction is carried out in an organic solvent as a reaction solvent, preferably, the organic solvent is isopropanol.

8. The preparation method according to any one of claims 1 to 7, wherein, in step (1), the reaction temperature is 50°C to 90°C, preferably 60°C to 80°C, 60°C to 70°C, 70°C to 80°C or 65°C to 75°C.

9. The preparation method according to any one of claims 1 to 8, wherein, in step (1), the reaction time is at least 0.5 hours, at least 1 hour, at least 1.5 hours, at least 2 hours, 1 to 10 hours, 2 to 8 hours, 2 to 6 hours, 2 to 4 hours, 2 to 3 hours.

10. The preparation method according to any one of claims 1 to 9, wherein, in step (2), the compound represented by Formula V is reacted with a base to prepare pranoprofen;
preferably, the base is one or more selected from the group consisting of sodium hydroxide, potassium hydroxide, lithium hydroxide, sodium carbonate and potassium carbonate.

11. The preparation method according to any one of claims 1 to 10, wherein, in step (2), the reaction temperature is 40°C to 75°C, preferably 40°C to 55°C, 40°C to 50°C, 45°C to 55°C, 40°C to 45°C, 45°C to 50°C, or 50°C to 55°C.

12. The preparation method according to any one of claims 1 to 11, wherein, in step (2), the reaction time is at least 0.5 hours, at least 1 hour, at least 1.5 hours, 0.5 to 10 hours, 1 to 8 hours, 1.5 to 6 hours, 1.5 to 4 hours, 1.5 to 3 hours or 1.5 to 2.5 hours.

13. The preparation method according to any one of claims 1 to 12, which further comprises the following steps:
(3) separating pranoprofen (compound represented by Formula 0) from the reaction system;
preferably, step (3) comprises:
purified water is added to the reaction system, and then the aqueous phase is extracted with ethyl acetate;
the aqueous phase is collected and adjusted to pH 2-7 with an acid (preferably, adjusted with 20% dilute acid solution), and gradually precipitates a solid;
after filtration, the filter cake is washed with purified water and methanol in turn, and dried in vacuum to obtain crude pranoprofen; and
refined pranoprofen is obtained by recrystallization from methanol (preferably, 8 to 12 times volume, such as 10 times volume, of methanol is used to perform the recrystallization under refluxing).

14. The preparation method according to any one of claims 1-13, wherein, in step (3), the pH is preferably 5 to 7, more preferably 5.5 to 6.5, particularly preferably 5.8 to 6.2.

15. The preparation method according to any one of claims 1 to 14, wherein, in step (3), the acid is one or more selected from the group consisting of acetic acid, phosphoric acid, hydrochloric acid and sulfuric acid, preferably acetic acid and/or hydrochloric acid.

16. The preparation method according to any one of claims 1 to 15, which does not comprise a step of isolating the compound represented by Formula V.

17. The preparation method according to any one of claims 1 to 16, comprising the following steps:
the compound represented by Formula IV, the reducing agent and isopropanol are added into a reaction vessel, stirred and heated to 50°C to 90°C, reacted by keeping the temperature for 2 to 4h, concentrated under reduced pressure at 55°C to dryness, cooled to room temperature and added with methanol (methanol is used as a solvent), then added with an alkaline water (or added with an alkaline water, then cooled to room temperature and added with methanol), and reacted at 40°C to 75°C for 2h;
the reaction solution is added with purified water, then the aqueous phase is extracted with ethyl acetate, the aqueous phase is collected and adjusted to pH 2-7 with 20% diluted acid solution, and gradually precipitate a solid;
after filtration, the filter cake is washed with purified water and methanol in turn;
vacuum drying is performed to obtain a crude product, which is refined by methanol recrystallization to obtain pranoprofen.

18. A composition, comprising pranoprofen and an impurity, wherein, according to mass percentage, the impurity in the composition has a content of less than or equal to 0.5%, less than or equal to 0.4%, less than or equal to 0.3%, less than or equal to 0.25%, less than or equal to 0.24%, less than or equal to 0.23%, less than or equal to 0.22%, less than or equal to 0.21%, less than or equal to 0.20%, less than or equal to 0.19%, less than or equal to 0.18%, less than or equal to 0.17%, less than or equal to 0.16%, less than or equal to 0.15%, less than or equal to 0.14%, less than or equal to 0.13%, less than or equal to 0.12%, less than or equal to 0.11%, less than or equal to 0.10%, less than or equal to 0.09 %, less than or equal to 0.08%, less than or equal to 0.07%, less than or equal to 0.06%, less than or equal to 0.05%, less than or equal to 0.04%, less than or equal to 0.03%, less than or equal to 0.02%, less than or equal to 0.01%, or less than or equal to 0.005%.

19. The composition according to claim 18, wherein, according to mass percentage, the pranoprofen in the composition has a content of greater than or equal to 99%, greater than or equal to 99.1%, greater than or equal to 99.2%, greater than or equal to 99.3%, greater than or equal to 99.4%, greater than or equal to 99.5%, greater than or equal to 99.6%, greater than or equal to 99.7%, greater than or equal to 99.8%, greater than or equal to 99.9%, greater than or equal to 99.91%, greater than or equal to 99.92 %, greater than or equal to 99.93%, greater than or equal to 99.94%, greater than or equal to 99.95%, greater than or equal to 99.96%, greater than or equal to 99.97%, greater than or equal to 99.98%, or greater than or equal to 99.99%.

20. The composition according to any one of claims 18 to 19, wherein the impurity has a content of greater than zero.

21. The composition according to any one of claims 18 to 20, wherein the composition consists of pranoprofen and the impurity.

22. The composition according to any one of claims 18 to 21, wherein the impurity is total impurities or maximum single impurity.

23. The composition according to any one of claims 18 to 22, wherein the impurity consists of or comprises any one, any two, any three or any four selected from the group consisting of impurity A, impurity C, impurity D and impurity L;

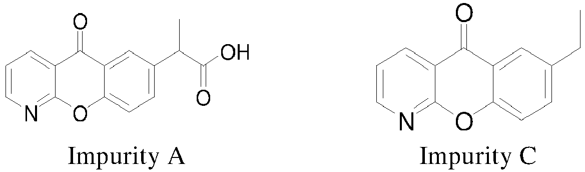

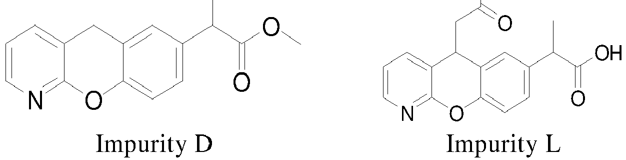

24. The composition according to any one of claims 18 to 23, wherein the total impurities has a content of less than or equal to 0.2%, less than or equal to 0.15%, less than or equal to 0.12%, less than or equal to 0.1%, less than or equal to 0.09%, less than or equal to 0.08%, less than or equal to 0.07%, less than or equal to 0.06%, less than or equal to 0.05%, less than or equal to 0.04%, less than or equal to 0.03%, less than or equal to 0.02%, or less than or equal to 0.01%.

25. The composition according to any one of claims 18 to 24, wherein the impurity A, impurity C, impurity D or impurity L has a content of less than or equal to 0.2%, less than or equal to 0.15%, less than or equal to 0.12%, less than or equal to 0.1%, less than or equal to 0.09%, less than or equal to 0.08%, less than or equal to 0.07%, less than or equal to 0.06%, less than or equal to 0.05%, less than or equal to 0.04%, less than or equal to 0.03%, less than or equal to 0.02%, less than or equal to 0.01%, or less than or equal to 0.005%.

26. The composition according to any one of claims 18 to 25, wherein, after 1 month, 2 months or 3 months of an accelerated experiment, the total impurities, maximum single impurity, impurity A, impurity C, impurity D and/or impurity L has a content of less than or equal to 0.2%, less than or equal to 0.15%, less than or equal to 0.12%, less than or equal to 0.1%, less than or equal to 0.09%, less than or equal to 0.08%, less than or equal to 0.07 %, less than or equal to 0.06%, less than or equal to 0.05%, less than or equal to 0.04%, less than or equal to 0.03%, less than or equal to 0.02%, or less than or equal to 0.01%;
preferably, the accelerated experiment is carried out under the conditions of 40°C±2°C and RH75%±5% in accordance with the regulations in the "Technical Guidelines for Stability Research of Chemical Drugs".

27. The composition according to any one of claims 18 to 26, wherein, after 6 months of an accelerated experimentation, the total impurities, maximum single impurity, impurity A, impurity C, impurity D and/or impurity L has a content of less than or equal to 0.5%, less than or equal to 0.45%, less than or equal to 0.4%, less than or equal to 0.35%, less than or equal to 0.3%, less than or equal to 0.25%, less than or equal to 0.2%, less than or equal to 0.15%, less than or equal to 0.12%, less than or equal to 0.1%, less than or equal to 0.09%, less than or equal to 0.08%, less than or equal to 0.07%, less than or equal to 0.06%, less than or equal to 0.05%, less than or equal to 0.04%, less than or equal to 0.03%, less than or equal to 0.02%, or less than or equal to 0.01%;
preferably, the accelerated experiment is carried out under the conditions of 40°C±2°C and RH75%±5% in accordance with the regulations in the "Technical Guidelines for Stability Research of Chemical Drugs".

28. A pharmaceutical preparation, comprising the composition according to any one of claims 18 to 27, and one or more pharmaceutically acceptable excipients.

29. A method for quality inspection or quality control of a pranoprofen product, comprising a step of detecting pranoprofen content and/or impurity content therein; preferably, the pranoprofen product is the composition according to any one of claims 18 to 27 or the pharmaceutical preparation according to claim 28; preferably, the method is high performance liquid chromatography.

30. Use of the following impurities or combinations of impurities in quality inspection or quality control of a pranoprofen product:
Impurity L,
Impurity L and Impurity A,
Impurity L and Impurity C,
Impurity L and Impurity D,
Impurity L, Impurity A and Impurity C,
Impurity L, Impurity A and Impurity D,
Impurity L, Impurity C and Impurity D,
or
Impurity L, Impurity A, Impurity C and Impurity D; 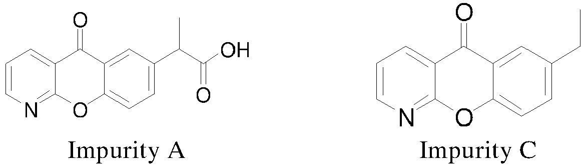 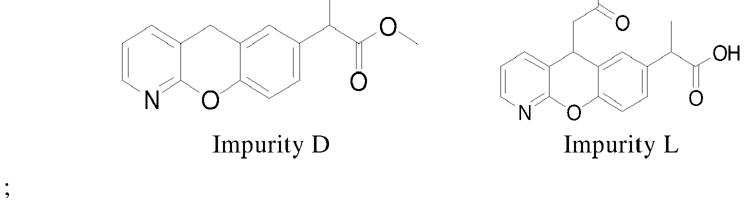
preferably, the pranoprofen product is the composition according to any one of claims 18 to 27 or the pharmaceutical preparation according to claim 28.

31. Use of the following impurities or combinations of impurities in quality inspection or quality control of a pranoprofen product:
Impurity L,
Impurity L and Impurity A,
Impurity L and Impurity C,
Impurity L and Impurity D,
Impurity L, Impurity A and Impurity C,
Impurity L, Impurity A and Impurity D,
Impurity L, Impurity C and Impurity D,
or
Impurity L, Impurity A, Impurity C and Impurity D; 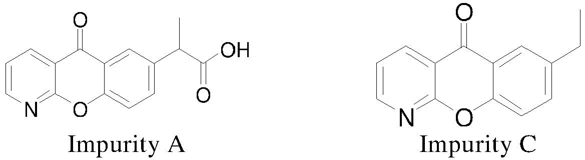 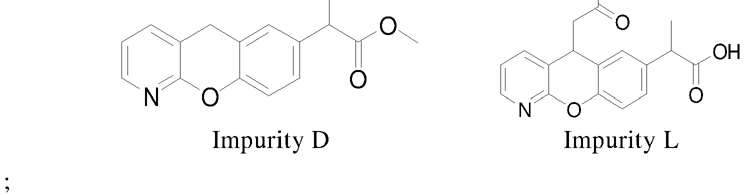
preferably, the pranoprofen product is the composition according to any one of claims 18 to 27 or the pharmaceutical preparation according to claim 28.

32. A compound represented by Formula I, or pharmaceutically acceptable salt or ester thereof, 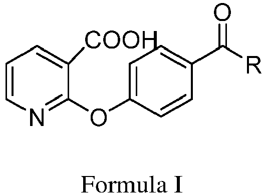 wherein, R is C₂-C₁₀ straight chain or branched chain alkyl, preferably, R is C₂-C₆ straight chain or branched chain alkyl, more preferably, R is C₂-C₄ straight chain or branched chain alkyl, such as ethyl, propyl, isopropyl or n-butyl.

33. A method for preparing the compound represented by Formula I according to claim 32, comprising a step of reacting a compound represented by Formula A with a compound represented by Formula B to generate the compound represented by Formula I: 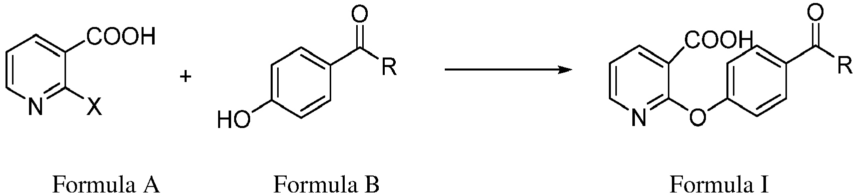 wherein,
X is halogen, such as fluorine, chlorine, bromine or iodine, preferably chlorine or bromine;
R is C₂-C₁₀ straight chain or branched chain alkyl, preferably, R is C₂-C₆ straight chain or branched chain alkyl, more preferably, R is C₂-C₄ straight chain or branched chain alkyl, such as ethyl, propyl, isopropyl or n-butyl.

34. The preparation method according to claim 33, wherein the compound represented by Formula A is selected from the group consisting of 2-chloronicotinic acid, 2-bromonicotinic acid and 2-iodonicotinic acid.

35. The preparation method according to any one of claims 33 to 34, wherein the compound represented by Formula B is selected from the group consisting of 4-hydroxypropiophenone, 4-hydroxybutyrophenone and 4-hydroxyvalerophenone.

36. The preparation method according to any one of claims 33 to 35, wherein the reaction system has a temperature of 60°C to 150°C, preferably 80°C to 120°C, more preferably 95°C to 120°C, 95°C to 110°C, 105°C to 120°C, 105°C to 115°C, 110°C to 120°C, 95°C to 105°C, or 105°C to 110°C.

37. The preparation method according to any one of claims 33 to 36, wherein the reaction time is at least 1 hour, at least 2 hours, at least 3 hours, at least 4 hours, at least 5 hours, 1 to 10 hours, 2 to 8 hours, 3 to 7 hours, or 4 to 6 hours.

38. The preparation method according to any one of claims 33 to 37, wherein the compound represented by Formula A reacts with the compound represented by Formula B in an organic solvent as a reaction solvent, preferably, the organic solvent is *N*,*N*-dimethylformamide or dimethylsulfoxide; more preferably, the organic solvent is *N,N-*dimethylformamide.

39. The preparation method according to any one of claims 33 to 38, wherein the compound represented by Formula A reacts with the compound represented by Formula B under the action of a catalyst, preferably, the catalyst is cuprous iodide.

40. The preparation method according to any one of claims 33 to 39, wherein the reaction system also contains an acid-binding agent, such as potassium carbonate, sodium carbonate, potassium bicarbonate or sodium bicarbonate.

41. The preparation method according to any one of claims 33 to 40, wherein, according to molar ratio calculation, the Formula A compound:the Formula B compound:the catalyst:the acid-binding agent is 1:(1-5):(0.05-1):(1-4), preferably 1:(1-3):(0.05-0.1):(1-2).

42. The preparation method according to any one of claims 33 to 41, which further comprises a step of isolating the compound represented by Formula I from the reaction system, preferably, comprising the following step:
the reaction system is cooled (e.g., to 50°C or lower), then dded with water, acidified to pH 3-4, the precipitated solid is collected, added with water to disperse, adjusted to pH 7-7.5, filtered to remove solid, the filtrate is acidified to pH 3-4, the precipitated solid is collected, washed with water and dried to obtain the compound represented by Formula I.

43. The preparation method according to any one of claims 33 to 42, comprising the following steps:
the compound represented by Formula A, cuprous iodide and the reaction solvent are added into a reaction vessel, controlled to have a temperature of 60°C to 150°C, added with the acid-binding agent and the compound represented by Formula B in batches, and reacted at temperature controlled at 60°C to 150°C for 2 to 8 hours;
the reaction solution is cooled, then added with water, and acidified to pH 3-4, the precipitated solid is collected, added with water to disperse, adjusted to pH 7-7.5 (preferably, adjusted with sodium bicarbonate), filtered to remove solid, the filtrate is acidified to pH 3-4, the precipitated solid is collected, washed with water, and dried to obtain the compound represented by Formula I.

44. Use of the compound represented by Formula I or pharmaceutically acceptable salt or ester thereof according to claim 32 in the manufacture of pranoprofen or a pharmaceutically acceptable salt or ester of pranoprofen; preferably, the compound represented by Formula I is prepared by the preparation method according to any one of claims 33 to 43.

45. The compound represented by Formula I or pharmaceutically acceptable salt or ester thereof according to claim 32, which is used in the manufacture of pranoprofen or a pharmaceutically acceptable salt or ester of pranoprofen; preferably, the compound represented by Formula I is prepared by the preparation method according to any one of claims 33 to 43.

46. A compound represented by Formula II, or pharmaceutically acceptable salt or ester thereof, 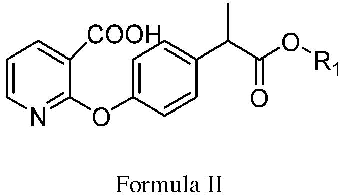 wherein, R₁ is C₁-C₁₀ straight chain or branched chain alkyl, preferably, Ri is C₁-C₅ straight chain or branched chain alkyl, more preferably, R₁ is C₁-C₃ straight chain or branched chain alkyl, such as methyl, ethyl, propyl or isopropyl.

47. A method for preparing the compound represented by Formula II according to claim 46, comprising a step of reacting a compound represented by Formula I with a compound represented by Formula C and a compound represented by Formula D to generate the compound represented by Formula II: 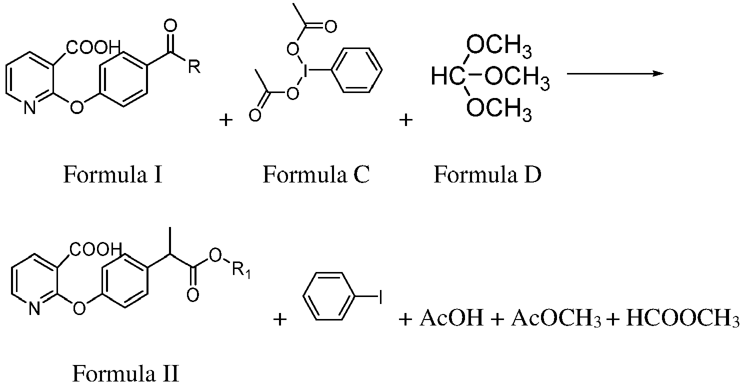 wherein,
R is C₂-C₁₀ straight chain or branched chain alkyl, preferably, R is C₂-C₆ straight chain or branched chain alkyl, more preferably, R is C₂-C₄ straight chain or branched chain alkyl, such as ethyl, propyl, isopropyl or n-butyl;
Ri is C₁-C₁₀ straight chain or branched chain alkyl, preferably, Ri is C₁-C₅ straight chain or branched chain alkyl, more preferably, R₁ is C₁-C₃ straight chain or branched chain alkyl, such as methyl, ethyl, propyl or isopropyl;
and R has one more carbon atom than R₁.

48. The preparation method according to claim 47, wherein the compound represented by Formula I is preferably 2-[4-(1-oxopropyl)phenoxy]-3-pyridinecarboxylic acid, 2-[4-(1-oxobutyl)phenoxy]-3-pyridinecarboxylic acid or 2-[4-(1-oxopentyl)phenoxy]-3-pyridinecarboxylic acid, more preferably 2-[4-(1-oxopropyl)phenoxy]-3-pyridinecarboxylic acid.

49. The preparation method according to any one of claims 47 to 48, wherein the reaction system has a temperature of -5°C to 30°C, preferably -5°C to 20°C, more preferably -5°C to 15°C, -5°C to 10°C, -5°C to 5°C, 5°C to 20°C, 5°C to 15°C, 5°C to 10°C, 10°C to 20°C, 10°C to 15°C, or 15°C to 20°C.

50. The preparation method according to any one of claims 47 to 49, wherein the reaction time is at least 0.2 hours, at least 0.4 hours, at least 0.6 hours, at least 0.8 hours, at least 0.9 hours, at least 1 hour, 0.2 to 2 hours, 0.4 to 1.6 hours, 0.6 to 1.4 hours, or 0.8 to 1.2 hours.

51. The preparation method according to any one of claims 47 to 50, wherein the compound represented by Formula I is reacted with the compound represented by Formula C in an organic solvent as a reaction solvent, preferably, the organic solvent is trimethyl orthoformate.

52. The preparation method according to any one of claims 47 to 51, wherein the compound represented by Formula I reacts with the compound represented by Formula C under the action of a catalyst, preferably, the catalyst is an acid, preferably, the acid is one or more selected from the group consisting of concentrated sulfuric acid, hydrochloric acid, phosphoric acid and acetic acid, preferably concentrated sulfuric acid.

53. The preparation method according to any one of claims 47 to 52, wherein, according to the molar ratio calculation, the Formula I compound:the iodobenzene diacetate:the acid is 1:(1-3):(1-4), preferably 1:(1-2):(1-3).

54. The preparation method according to any one of claims 47 to 53, which further comprises a step of isolating the compound represented by Formula II from the reaction system, preferably, comprising the following step:
the reaction system is added with an alkaline solution (e.g., sodium bicarbonate aqueous solution) for neutralization, extracted with an organic solvent (preferably dichloromethane), the aqueous phase was retained, and acidified to pH 3-4, then the precipitated solid is collected, washed with water, and dried to obtain the compound represented by Formula II.

55. The preparation method according to any one of claims 47 to 54, comprising the following steps:
the compound represented by Formula I and triethyl orthoformate are added into a reaction vessel, slowly added with the acid solution, and controlled to have a reaction temperature of -5°C to 30°C;
iodobenzene diacetate is slowly added, and controlled to have a reaction temperature of -5°C to 30°C;
the acid solution is slowly added, and controlled to have a reaction temperature of -5°C to 30°C;
the reaction is carried out by keeping the temperature for 0.5 to 2 hours, then sodium bicarbonate aqueous solution is added for neutralization, the aqueous phase after extraction with dichloromethane is retained, and acidified to pH 3-4, and the precipitated solid is collected, washed with water, and dried to obtain the compound represented by Formula II.

56. Use of the compound represented by Formula II or pharmaceutically acceptable salt or ester thereof according to claim 46 in the manufacture of pranoprofen or a pharmaceutically acceptable salt or ester of pranoprofen; preferably, the compound represented by Formula II is prepared by the preparation method according to any one of claims 47 to 55.

57. The compound represented by Formula II or pharmaceutically acceptable salt or ester thereof according to claim 46, which is used in the manufacture of pranoprofen or a pharmaceutically acceptable salt or ester of pranoprofen; preferably, the compound represented by Formula II is prepared by the preparation method according to any one of claims 47 to 55.

58. A method for preparing a compound represented by Formula III, comprising a step of preparing a compound represented by Formula III from a compound represented by Formula II: 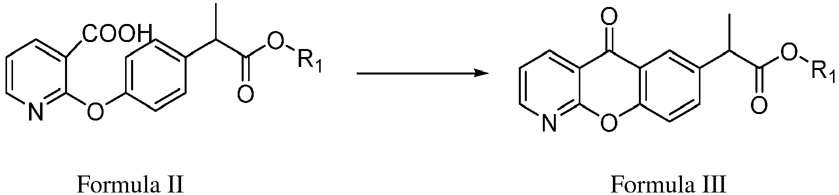 wherein, R₁ is C₁-C₁₀ straight chain or branched chain alkyl, preferably, Ri is C₁-C₅ straight chain or branched chain alkyl, more preferably, R₁ is C₁-C₃ straight chain or branched chain alkyl, such as methyl, ethyl, propyl or isopropyl.

59. The preparation method according to claim 58, comprising the following reactions, as shown below; 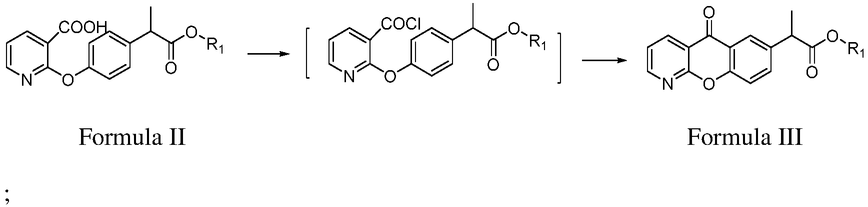 preferably, the product as shown in the above brackets is not separated; preferably, it is directly used in a subsequent reaction.

60. The preparation method according to any one of claims 58 to 59, wherein the compound represented by Formula II is preferably 2-[4-(2-methoxyl-1-methyl-2-oxoethyl)phenoxy]-3-pyridinecarboxylic acid, 2-[4-(2-ethoxy-1-methyl-2-oxoethyl)phenoxy]-3-pyridinecarboxylic acid or 2-[4-(2-propoxy-1-methyl-2-oxoethyl)phenoxy]-3-pyridinecarboxylic acid; more preferably 2-[4-(2-methoxy-1-methyl-2-oxoethyl)phenoxy]-3-pyridinecarboxylic acid.

61. The preparation method according to any one of claims 58 to 60, comprising the following steps:
(1) reacting the compound represented by Formula II with an acylating agent to obtain a reaction product,
(2) generating the compound represented by Formula III from the reaction product of step (1) under the action of a Lewis acid;
preferably, further comprising the following step:
(3) isolate the compound represented by Formula III; preferably, cooling the reaction product obtained in step (2) to below 20°C (e.g., by adding cold water to quench), adjusting pH, and removing the solvent under reduced pressure to obtain the compound represented by Formula III.

62. The preparation method according to any one of claims 58 to 61, wherein, in step (1), the reaction temperature is 10°C to 30°C, preferably 15°C to 25°C, 15°C to 20°C, or 20°C to 25°C.

63. The preparation method according to any one of claims 58 to 62, wherein, in step (1), the reaction time is at least 0.2 hours, at least 0.4 hours, at least 0.6 hours, at least 0.8 hours, at least 0.9 hours, at least 1 hour, 0.2 to 2 hours, 0.4 to 1.6 hours, 0.6 to 1.4 hours, or 0.8 to 1.2 hours.

64. The preparation method according to any one of claims 58 to 63, wherein step (1) further comprises the following step:
cooling the resulting reaction product to -10°C to 0°C.

65. The preparation method according to any one of claims 58 to 64, wherein, in step (2), the reaction temperature is 10°C to 30°C, preferably 15°C to 25°C, 15°C to 20°C, or 20°C to 25°C.

66. The preparation method according to any one of claims 58 to 65, wherein, in step (2), the reaction time is at least 0.2 hours, at least 0.4 hours, at least 0.6 hours, at least 0.8 hours, at least 0.9 hours, at least 1 hour, 0.2 to 2 hours, 0.4 to 1.6 hours, 0.6 to 1.4 hours, 0.8 to 1.2 hours, or 1 to 1.5 hours.

67. The preparation method according to any one of claims 58 to 66, wherein, in step (1), the acylating agent is one or more selected from the group consisting of oxalyl chloride and thionyl chloride.

68. The preparation method according to any one of claims 58 to 67, wherein, in step (2), the Lewis acid is a catalyst; preferably, the Lewis acid is one or more selected from the group consisting of anhydrous aluminum trichloride and titanium tetrachloride.

69. The preparation method according to any one of claims 58 to 68, wherein the reaction of step (1) and/or step (2) is carried out in an organic solvent as a reaction solvent, preferably, the organic solvent is dichloromethane and/or N,N-dimethylformamide.

70. The preparation method according to any one of claims 58 to 69, wherein the molar ratio of the compound represented by Formula II to the acylating agent is (1:1) to (1:5), preferably 1:(1.5-3), particularly preferably 1:2.5.

71. The preparation method according to any one of claims 58 to 70, wherein the molar ratio of the compound represented by Formula II to oxalyl chloride is (1:1) to (1:5), preferably 1:(1.5-3), particularly preferably 1:2.5.

72. The preparation method according to any one of claims 58 to 71, wherein step (3) comprises the following operations:
the reaction product of step (2) is added with cold water to quench the reaction, the organic phase is separated, adjusted to pH 7-8 (e.g., with sodium carbonate solution), and then the organic phase is separated; the organic phase is concentrated under reduced pressure until the distillate is no longer distilled off, then methanol or a mixed solvent of methanol and dichloromethane is added for beating for 3 to 8 hours (preferably, 5 to 6 hours), and the filter residue after filtration is rinsed with methanol, and dried to obtain the compound represented by Formula III; in some embodiments of the present invention, the volume ratio of methanol to dichloromethane is 1:(0-0.4), preferably 1:(0.1-0.2).

73. The preparation method according to any one of claims 58 to 72, comprising the following steps:
the compound represented by Formula II, dichloromethane and *N*,*N*-dimethylformamide are added into a first reaction vessel, mixed and stirred, added with the acylating reagent at a temperature of 15°C to 25°C, reacted by keeping the temperature for 0.5 to 2 hours, and then cooled to -10°C to 0°C for later use;
the Lewis acid and dichloromethane are added into a second reaction vessel, stirred evenly, added with the solution of the first reaction vessel under temperature controlled at 15°C to 25°C, reacted by keeping the temperature for 1 to 1.5h, and added slowly with cold water under temperature controlled under 20°C to quench;
the organic phase is retained, adjusted to pH 7-8 with sodium carbonate solution, and subjected to liquid separation, and the organic phase is concentrated under reduced pressure;
when the distillate is no longer distilled off, a mixed solvent of methanol and dichloromethane is added for beating for 5 to 6 hours, the filter residue after filtration is rinsed with methanol, and dried to obtain the compound represented by Formula III.

74. The preparation method according to any one of claims 58 to 73, wherein in the obtained compound represented by Formula III, the content of each of the following three compounds is less than or equal to 0.1%, preferably less than or equal to 0.05%: 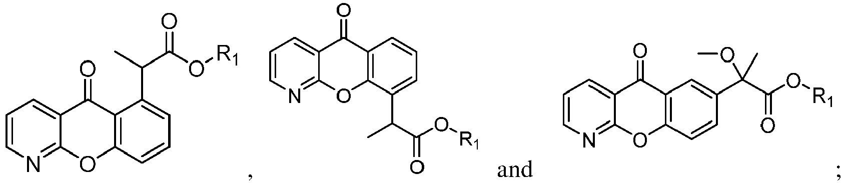 wherein, Ri is independently C₁-C₁₀ straight chain or branched chain alkyl, preferably, Ri is C₁-C₅ straight chain or branched chain alkyl, more preferably, Ri is C₁-C₃ straight chain or branched chain alkyl, such as methyl, ethyl, propyl or isopropyl.

75. Use of a compound represented by Formula III or pharmaceutically acceptable salt or ester thereof in the manufacture of pranoprofen or a pharmaceutically acceptable salt or ester of pranoprofen; 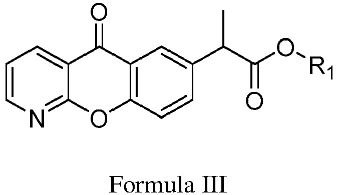
wherein, Ri is C₁-C₁₀ straight chain or branched chain alkyl, preferably, Ri is C₁-C₅ straight chain or branched chain alkyl, more preferably, R₁ is C₁-C₃ straight chain or branched chain alkyl, such as methyl, ethyl, propyl or isopropyl;
preferably, the compound represented by Formula III is prepared by the preparation method according to any one of claims 58 to 74.

76. A compound represented by Formula III or pharmaceutically acceptable salt or ester thereof, which is used in the manufacture of pranoprofen or a pharmaceutically acceptable salt or ester of pranoprofen; 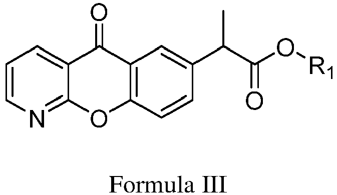
wherein, Ri is C₁-C₁₀ straight chain or branched chain alkyl, preferably, Ri is C₁-C₅ straight chain or branched chain alkyl, more preferably, R₁ is C₁-C₃ straight chain or branched chain alkyl, such as methyl, ethyl, propyl or isopropyl;
preferably, the compound represented by Formula III is prepared by the preparation method according to any one of claims 58 to 74.

77. A compound represented by Formula IV or pharmaceutically acceptable salt or ester thereof, 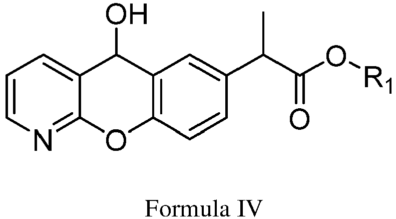 wherein, Ri is C₁-C₁₀ straight chain or branched chain alkyl, preferably, Ri is C₁-C₅ straight chain or branched chain alkyl, more preferably, R₁ is C₁-C₃ straight chain or branched chain alkyl, such as methyl, ethyl, propyl or isopropyl.

78. A method for preparing the compound represented by Formula IV according to claim 77, comprising a step of preparing the compound represented by Formula IV from the compound represented by Formula III: 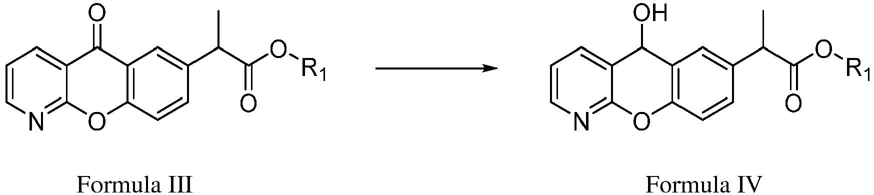 wherein, Ri is C₁-C₁₀ straight chain or branched chain alkyl, preferably, Ri is C₁-C₅ straight chain or branched chain alkyl, more preferably, R₁ is C₁-C₃ straight chain or branched chain alkyl, such as methyl, ethyl, propyl or isopropyl.

79. The preparation method according to claim 78, wherein the compound represented by Formula III is methyl 2-(10-oxo-9-oxa-1-azaanthracen-6-yl)propionate, ethyl 2-(10-oxo-9-oxa-1-azaanthracen-6-yl)propionate or propyl 2-(10-oxo-9-oxa-1-azaanthracen-6-yl)propionate.

80. The preparation method according to any one of claims 78 to 79, wherein the compound represented by Formula III is reacted with a reducing agent to prepare the compound represented by Formula IV; preferably, the reducing agent is a borohydride, more preferably sodium borohydride.

81. The preparation method according to any one of claims 78-80, wherein the reaction temperature is 20°C to 40°C, preferably 30°C to 40°C, 30°C to 35°C, or 35°C to 40°C.

82. The preparation method according to any one of claims 78 to 81, wherein the reaction time is at least 1 hour, at least 2 hours, at least 3 hours, at least 4 hours, at least 5 hours, at least 6 hours, 1 to 100 hours, 2 to 50 hours, 3 to 40 hours, 4 to 30 hours, 5 to 25 hours, or 6 to 20 hours.

83. The preparation method according to any one of claims 78 to 82, wherein the reaction is carried out in an organic solvent as a reaction solvent, preferably, the organic solvent is a mixed solvent of dichloromethane and methanol; preferably, the molar ratio of dichloromethane to methanol is 1:(2.11-3.16), preferably 1:2.11;
preferably, the mass (g) of the compound represented by Formula III:the volume (mL) of the mixed solvent is 1:(5-15), preferably 1:(8-10).

84. The preparation method according to any one of claims 78 to 83, wherein the molar ratio of the compound represented by Formula III to the reducing agent is (1:0.8) to (1:1.2), preferably 1:0.9-1.1.

85. The preparation method according to any one of claims 78 to 84, further comprising a step of separating the compound represented by Formula IV from the reaction system; preferably, comprising the following steps:
the reaction product is cooled to -10°C to 10°C, and adjusted to pH 5.5-6.5; preferably, a diluted acid solution (e.g., 30% acetic acid aqueous solution) is slowly added dropwise to pH 5.5-6.5;
stirring is carried out at -10°C to 10°C for 5 to 30 minutes (preferably 15 minutes), then purified water is added, and the organic phase is collected, and washed with saturated sodium bicarbonate and purified water in turn;
the washed organic phase is concentrated to dryness under reduced pressure at 40°C to obtain a yellow-green oil;
a crystallization solvent is added and stirred at 20°C to 40°C for 5 to 30 minutes (preferably 15 minutes); and
purified water is slowly added dropwise, the solid is collected, washed with water, and dried in vacuum to obtain the compound represented by Formula IV.

86. The preparation method according to any one of claims 78 to 85, wherein the crystallization solvent is selected from the group consisting of a methanol aqueous solution (preferably, methanol:water is 1:2), an acetone aqueous solution (preferably, acetone:water is 1:2), and a mixture of methanol, acetone and water (preferably, methanol:acetone:water is 1:1.5:5);
preferably, the mass (g) of the compound represented by Formula III:the volume (mL) of the crystallization solvent is 1:5.

87. The preparation method according to any one of claims 78 to 86, comprising the following steps:
the compound represented by Formula III, dichloromethane and methanol are added into a reaction vessel, and stirred for dissolution at a temperature of 15°C to 30°C;
the borohydride is slowly added, reacted at a temperature controlled at 20°C to 40°C for 6 to 20 hours, then cooled to -10°C to 10°C, slowly added dropwise with 30% acetic acid aqueous solution to pH 5.5-6.5, stirred at a temperature controlled at -10°C to 10°C for 5 to 30 minutes (preferably, 15 minutes), and added with purified water, the organic phase is collected, washed with saturated sodium bicarbonate, and washed again with purified water;
the organic phase is concentrated to dryness under reduced pressure at 40°C to obtain a yellow-green oil;
a mixed solution of methanol/acetone is added, and stirred to disperse at 20°C to 40°C for 15 minutes;
purified water is slowly added dropwise for crystallization for 1 to 5 hours (preferably, 2 hours), the solid is collected, washed with water, and dried in vacuum to obtain the compound represented by Formula IV.

88. Use of a compound represented by Formula IV or pharmaceutically acceptable salt or ester thereof in the manufacture of pranoprofen or a pharmaceutically acceptable salt or ester of pranoprofen; preferably, the compound represented by Formula IV is prepared by the preparation method according to any one of claims 78 to 87.

89. A compound represented by Formula IV or pharmaceutically acceptable salt or ester thereof, which is used in the manufacture of pranoprofen or a pharmaceutically acceptable salt or ester of pranoprofen; preferably, the compound represented by Formula IV is prepared by the preparation method according to any one of claims 78 to 87.
